# NEW EUROPEAN PATENT SPECIFICATION

(11) **EP 3 722 434 B2**
(45) Date of publication and mention of the opposition decision: **27.05.2026**
(45) Mention of the grant of the patent: 27.07.2022
(21) Application number: 19169121.1
(22) Date of filing: 12.04.2019
(51) Int. Cl.: C12N 15/86

(54) **PLASMID SYSTEM**
PLASMIDSYSTEM
SYSTÈME PLASMIDIQUE

(43) Date of publication of application: 14.10.2020
(73) Proprietor: Ascend Gene and Cell Therapies Ltd, Cheshire WA14 2DT (GB)
(72) Inventor: HÖRER, Markus, 82152 Planegg-Steinkirchen (DE); SONNTAG, Florian, 82152 Planegg-Steinkirchen (DE); KOBER, Renée, 82152 Planegg-Steinkirchen (DE)
(74) Representative: J A Kemp LLP

(56) References cited:
- CN-A- 108 048 483
- US-A1- 2003 103 939
- US-A1- 2005 080 027
- EMMERLING VERENA V., PEGEL ANTJE, MILIAN ERNEST G., VENEREO‐SANCHEZ ALINA, KUNZ MARION, WEGELE JESSICA, KAMEN AMINE A., KOCHANEK S: "Supporting Information - Rational plasmid design and bioprocess optimization to enhance recombinant adeno‐associated virus (AAV) productivity in mammalian cells", BIOTECHNOLOGY JOURNAL, WILEY-VCH VERLAG, WEINHEIM, DE, 1 January 2015 (2015-01-01), DE , XP093151807, ISSN: 1860-6768, DOI: 10.1002/biot.201500176

### SEQUENCE LISTING

<110> FREELINE THERAPEUTICS LIMITED
<120> PLASMID SYSTEM
<130> N415537EP
<160> 5
<170> PatentIn version 3.5
<210> 1
   <211> 4679
   <212> DNA
   <213> Adeno associated virus type 2
<400> 1
   ttggccactc cctctctgcg cgctcgctcg ctcactgagg ccgggcgacc aaaggtcgcc      60
   cgacgcccgg gctttgcccg ggcggcctca gtgagcgagc gagcgcgcag agagggagtg     120
   gccaactcca tcactagggg ttcctggagg ggtggagtcg tgacgtgaat tacgtcatag     180
   ggttagggag gtcctgtatt agaggtcacg tgagtgtttt gcgacatttt gcgacaccat     240
   gtggtcacgc tgggtattta agcccgagtg agcacgcagg gtctccattt tgaagcggga     300
   ggtttgaacg cgcagccgcc atgccggggt tttacgagat tgtgattaag gtccccagcg     360
   accttgacga gcatctgccc ggcatttctg acagctttgt gaactgggtg gccgagaagg     420
   aatgggagtt gccgccagat tctgacatgg atctgaatct gattgagcag gcacccctga     480
   ccgtggccga gaagctgcag cgcgactttc tgacggaatg gcgccgtgtg agtaaggccc     540
   cggaggccct tttctttgtg caatttgaga agggagagag ctacttccac atgcacgtgc     600
   tcgtggaaac caccggggtg aaatccatgg ttttgggacg tttcctgagt cagattcgcg     660
   aaaaactgat tcagagaatt taccgcggga tcgagccgac tttgccaaac tggttcgcgg     720
   tcacaaagac cagaaatggc gccggaggcg ggaacaaggt ggtggatgag tgctacatcc     780
   ccaattactt gctccccaaa acccagcctg agctccagtg ggcgtggact aatatggaac     840
   agtatttaag cgcctgtttg aatctcacgg agcgtaaacg gttggtggcg cagcatctga     900
   cgcacgtgtc gcagacgcag gagcagaaca aagagaatca gaatcccaat tctgatgcgc     960
   cggtgatcag atcaaaaact tcagccaggt acatggagct ggtcgggtgg ctcgtggaca    1020
   aggggattac ctcggagaag cagtggatcc aggaggacca ggcctcatac atctccttca    1080
   atgcggcctc caactcgcgg tcccaaatca aggctgcctt ggacaatgcg ggaaagatta    1140
   tgagcctgac taaaaccgcc cccgactacc tggtgggcca gcagcccgtg gaggacattt    1200
   ccagcaatcg gatttataaa attttggaac taaacgggta cgatccccaa tatgcggctt    1260
   ccgtctttct gggatgggcc acgaaaaagt tcggcaagag gaacaccatc tggctgtttg    1320
   ggcctgcaac taccgggaag accaacatcg cggaggccat agcccacact gtgcccttct    1380
   acgggtgcgt aaactggacc aatgagaact ttcccttcaa cgactgtgtc gacaagatgg 1440
   tgatctggtg ggaggagggg aagatgaccg ccaaggtcgt ggagtcggcc aaagccattc 1500
   tcggaggaag caaggtgcgc gtggaccaga aatgcaagtc ctcggcccag atagacccga 1560
   ctcccgtgat cgtcacctcc aacaccaaca tgtgcgccgt gattgacggg aactcaacga 1620
   ccttcgaaca ccagcagccg ttgcaagacc ggatgttcaa atttgaactc acccgccgtc 1680
   tggatcatga ctttgggaag gtcaccaagc aggaagtcaa agactttttc cggtgggcaa 1740
   aggatcacgt ggttgaggtg gagcatgaat tctacgtcaa aaagggtgga gccaagaaaa 1800
   gacccgcccc cagtgacgca gatataagtg agcccaaacg ggtgcgcgag tcagttgcgc 1860
   agccatcgac gtcagacgcg gaagcttcga tcaactacgc agacaggtac caaaacaaat 1920
   gttctcgtca cgtgggcatg aatctgatgc tgtttccctg cagacaatgc gagagaatga 1980
   atcagaattc aaatatctgc ttcactcacg gacagaaaga ctgtttagag tgctttcccg 2040
   tgtcagaatc tcaacccgtt tctgtcgtca aaaaggcgta tcagaaactg tgctacattc 2100
   atcatatcat gggaaaggtg ccagacgctt gcactgcctg cgatctggtc aatgtggatt 2160
   tggatgactg catctttgaa caataaatga tttaaatcag gtatggctgc cgatggttat 2220
   cttccagatt ggctcgagga cactctctct gaaggaataa gacagtggtg gaagctcaaa 2280
   cctggcccac caccaccaaa gcccgcagag cggcataagg acgacagcag gggtcttgtg 2340
   cttcctgggt acaagtacct cggacccttc aacggactcg acaagggaga gccggtcaac 2400
   gaggcagacg ccgcggccct cgagcacgac aaagcctacg accggcagct cgacagcgga 2460
   gacaacccgt acctcaagta caaccacgcc gacgcggagt ttcaggagcg ccttaaagaa 2520
   gatacgtctt ttgggggcaa cctcggacga gcagtcttcc aggcgaaaaa gagggttctt 2580
   gaacctctgg gcctggttga ggaacctgtt aagacggctc cgggaaaaaa gaggccggta 2640
   gagcactctc ctgtggagcc agactcctcc tcgggaaccg gaaaggcggg ccagcagcct 2700
   gcaagaaaaa gattgaattt tggtcagact ggagacgcag actcagtacc tgacccccag 2760
   cctctcggac agccaccagc agccccctct ggtctgggaa ctaatacgat ggctacaggc 2820
   agtggcgcac caatggcaga caataacgag ggcgccgacg gagtgggtaa ttcctcggga 2880
   aattggcatt gcgattccac atggatgggc gacagagtca tcaccaccag cacccgaacc 2940
   tgggccctgc ccacctacaa caaccacctc tacaaacaaa tttccagcca atcaggagcc 3000
   tcgaacgaca atcactactt tggctacagc accccttggg ggtattttga cttcaacaga 3060
   ttccactgcc acttttcacc acgtgactgg caaagactca tcaacaacaa ctggggattc 3120
   cgacccaaga gactcaactt caagctcttt aacattcaag tcaaagaggt cacgcagaat 3180
   gacggtacga cgacgattgc caataacctt accagcacgg ttcaggtgtt tactgactcg 3240
   gagtaccagc tcccgtacgt cctcggctcg gcgcatcaag gatgcctccc gccgttccca 3300
   gcagacgtct tcatggtgcc acagtatgga tacctcaccc tgaacaacgg gagtcaggca 3360
   gtaggacgct cttcatttta ctgcctggag tactttcctt ctcagatgct gcgtaccgga 3420
   aacaacttta ccttcagcta cacttttgag gacgttcctt tccacagcag ctacgctcac 3480
   agccagagtc tggaccgtct catgaatcct ctcatcgacc agtacctgta ttacttgagc 3540
   agaacaaaca ctccaagtgg aaccaccacg cagtcaaggc ttcagttttc tcaggccgga 3600
   gcgagtgaca ttcgggacca gtctaggaac tggcttcctg gaccctgtta ccgccagcag 3660
   cgagtatcaa agacatctgc ggataacaac aacagtgaat actcgtggac tggagctacc 3720
   aagtaccacc tcaatggcag agactctctg gtgaatccgg gcccggccat ggcaagccac 3780
   aaggacgatg aagaaaagtt ttttcctcag agcggggttc tcatctttgg gaagcaaggc 3840
   tcagagaaaa caaatgtgga cattgaaaag gtcatgatta cagacgaaga ggaaatcagg 3900
   acaaccaatc ccgtggctac ggagcagtat ggttctgtat ctaccaacct ccagagaggc 3960
   aacagacaag cagctaccgc agatgtcaac acacaaggcg ttcttccagg catggtctgg 4020
   caggacagag atgtgtacct tcaggggccc atctgggcaa agattccaca cacggacgga 4080
   cattttcacc cctctcccct catgggtgga ttcggactta aacaccctcc tccacagatt 4140
   ctcatcaaga acaccccggt acctgcgaat ccttcgacca ccttcagtgc ggcaaagttt 4200
   gcttccttca tcacacagta ctccacggga caggtcagcg tggagatcga gtgggagctg 4260
   cagaaggaaa acagcaaacg ctggaatccc gaaattcagt acacttccaa ctacaacaag 4320
   tctgttaatg tggactttac tgtggacact aatggcgtgt attcagagcc tcgccccatt 4380
   ggcaccagat acctgactcg taatctgtaa ttgcttgtta atcaataaac cgtttaattc 4440
   gtttcagttg aactttggtc tctgcgtatt tctttcttat ctagtttcca tggctacgta 4500
   gataagtagc atggcgggtt aatcattaac tacaaggaac ccctagtgat ggagttggcc 4560
   actccctctc tgcgcgctcg ctcgctcact gaggccgggc gaccaaaggt cgcccgacgc 4620
   ccgggctttg cccgggcggc ctcagtgagc gagcgagcgc gcagagaggg agtggccaa 4679
<210> 2
   <211> 35938
   <212> DNA
   <213> adenovirus type 5
<400> 2
   catcatcaat aatatacctt attttggatt gaagccaata tgataatgag ggggtggagt       60
   ttgtgacgtg gcgcggggcg tgggaacggg gcgggtgacg tagtagtgtg gcggaagtgt      120
   gatgttgcaa gtgtggcgga acacatgtaa gcgacggatg tggcaaaagt gacgtttttg      180
   gtgtgcgccg gtgtacacag gaagtgacaa ttttcgcgcg gttttaggcg gatgttgtag      240
   taaatttggg cgtaaccgag taagatttgg ccattttcgc gggaaaactg aataagagga 300
   agtgaaatct gaataatttt gtgttactca tagcgcgtaa tatttgtcta gggccgcggg 360
   gactttgacc gtttacgtgg agactcgccc aggtgttttt ctcaggtgtt ttccgcgttc 420
   cgggtcaaag ttggcgtttt attattatag tcagctgacg tgtagtgtat ttatacccgg 480
   tgagttcctc aagaggccac tcttgagtgc cagcgagtag agttttctcc tccgagccgc 540
   tccgacaccg ggactgaaaa tgagacatat tatctgccac ggaggtgtta ttaccgaaga 600
   aatggccgcc agtcttttgg accagctgat cgaagaggta ctggctgata atcttccacc 660
   tcctagccat tttgaaccac ctacccttca cgaactgtat gatttagacg tgacggcccc 720
   cgaagatccc aacgaggagg cggtttcgca gatttttccc gactctgtaa tgttggcggt 780
   gcaggaaggg attgacttac tcacttttcc gccggcgccc ggttctccgg agccgcctca 840
   cctttcccgg cagcccgagc agccggagca gagagccttg ggtccggttt ctatgccaaa 900
   ccttgtaccg gaggtgatcg atcttacctg ccacgaggct ggctttccac ccagtgacga 960
   cgaggatgaa gagggtgagg agtttgtgtt agattatgtg gagcaccccg ggcacggttg 1020
   caggtcttgt cattatcacc ggaggaatac gggggaccca gatattatgt gttcgctttg 1080
   ctatatgagg acctgtggca tgtttgtcta cagtaagtga aaattatggg cagtgggtga 1140
   tagagtggtg ggtttggtgt ggtaattttt tttttaattt ttacagtttt gtggtttaaa 1200
   gaattttgta ttgtgatttt tttaaaaggt cctgtgtctg aacctgagcc tgagcccgag 1260
   ccagaaccgg agcctgcaag acctacccgc cgtcctaaaa tggcgcctgc tatcctgaga 1320
   cgcccgacat cacctgtgtc tagagaatgc aatagtagta cggatagctg tgactccggt 1380
   ccttctaaca cacctcctga gatacacccg gtggtcccgc tgtgccccat taaaccagtt 1440
   gccgtgagag ttggtgggcg tcgccaggct gtggaatgta tcgaggactt gcttaacgag 1500
   cctgggcaac ctttggactt gagctgtaaa cgccccaggc cataaggtgt aaacctgtga 1560
   ttgcgtgtgt ggttaacgcc tttgtttgct gaatgagttg atgtaagttt aataaagggt 1620
   gagataatgt ttaacttgca tggcgtgtta aatggggcgg ggcttaaagg gtatataatg 1680
   cgccgtgggc taatcttggt tacatctgac ctcatggagg cttgggagtg tttggaagat 1740
   ttttctgctg tgcgtaactt gctggaacag agctctaaca gtacctcttg gttttggagg 1800
   tttctgtggg gctcatccca ggcaaagtta gtctgcagaa ttaaggagga ttacaagtgg 1860
   gaatttgaag agcttttgaa atcctgtggt gagctgtttg attctttgaa tctgggtcac 1920
   caggcgcttt tccaagagaa ggtcatcaag actttggatt tttccacacc ggggcgcgct 1980
   gcggctgctg ttgctttttt gagttttata aaggataaat ggagcgaaga aacccatctg 2040
   agcggggggt acctgctgga ttttctggcc atgcatctgt ggagagcggt tgtgagacac 2100
   aagaatcgcc tgctactgtt gtcttccgtc cgcccggcga taataccgac ggaggagcag 2160
   cagcagcagc aggaggaagc caggcggcgg cggcaggagc agagcccatg gaacccgaga 2220
   gccggcctgg accctcggga atgaatgttg tacaggtggc tgaactgtat ccagaactga 2280
   gacgcatttt gacaattaca gaggatgggc aggggctaaa gggggtaaag agggagcggg 2340
   gggcttgtga ggctacagag gaggctagga atctagcttt tagcttaatg accagacacc 2400
   gtcctgagtg tattactttt caacagatca aggataattg cgctaatgag cttgatctgc 2460
   tggcgcagaa gtattccata gagcagctga ccacttactg gctgcagcca ggggatgatt 2520
   ttgaggaggc tattagggta tatgcaaagg tggcacttag gccagattgc aagtacaaga 2580
   tcagcaaact tgtaaatatc aggaattgtt gctacatttc tgggaacggg gccgaggtgg 2640
   agatagatac ggaggatagg gtggccttta gatgtagcat gataaatatg tggccggggg 2700
   tgcttggcat ggacggggtg gttattatga atgtaaggtt tactggcccc aattttagcg 2760
   gtacggtttt cctggccaat accaacctta tcctacacgg tgtaagcttc tatgggttta 2820
   acaatacctg tgtggaagcc tggaccgatg taagggttcg gggctgtgcc ttttactgct 2880
   gctggaaggg ggtggtgtgt cgccccaaaa gcagggcttc aattaagaaa tgcctctttg 2940
   aaaggtgtac cttgggtatc ctgtctgagg gtaactccag ggtgcgccac aatgtggcct 3000
   ccgactgtgg ttgcttcatg ctagtgaaaa gcgtggctgt gattaagcat aacatggtat 3060
   gtggcaactg cgaggacagg gcctctcaga tgctgacctg ctcggacggc aactgtcacc 3120
   tgctgaagac cattcacgta gccagccact ctcgcaaggc ctggccagtg tttgagcata 3180
   acatactgac ccgctgttcc ttgcatttgg gtaacaggag gggggtgttc ctaccttacc 3240
   aatgcaattt gagtcacact aagatattgc ttgagcccga gagcatgtcc aaggtgaacc 3300
   tgaacggggt gtttgacatg accatgaaga tctggaaggt gctgaggtac gatgagaccc 3360
   gcaccaggtg cagaccctgc gagtgtggcg gtaaacatat taggaaccag cctgtgatgc 3420
   tggatgtgac cgaggagctg aggcccgatc acttggtgct ggcctgcacc cgcgctgagt 3480
   ttggctctag cgatgaagat acagattgag gtactgaaat gtgtgggcgt ggcttaaggg 3540
   tgggaaagaa tatataaggt gggggtctta tgtagttttg tatctgtttt gcagcagccg 3600
   ccgccgccat gagcaccaac tcgtttgatg gaagcattgt gagctcatat ttgacaacgc 3660
   gcatgccccc atgggccggg gtgcgtcaga atgtgatggg ctccagcatt gatggtcgcc 3720
   ccgtcctgcc cgcaaactct actaccttga cctacgagac cgtgtctgga acgccgttgg 3780
   agactgcagc ctccgccgcc gcttcagccg ctgcagccac cgcccgcggg attgtgactg 3840
   actttgcttt cctgagcccg cttgcaagca gtgcagcttc ccgttcatcc gcccgcgatg 3900
   acaagttgac ggctcttttg gcacaattgg attctttgac ccgggaactt aatgtcgttt 3960
   ctcagcagct gttggatctg cgccagcagg tttctgccct gaaggcttcc tcccctccca 4020
   atgcggttta aaacataaat aaaaaaccag actctgtttg gatttggatc aagcaagtgt 4080
   cttgctgtct ttatttaggg gttttgcgcg cgcggtaggc ccgggaccag cggtctcggt 4140
   cgttgagggt cctgtgtatt ttttccagga cgtggtaaag gtgactctgg atgttcagat 4200
   acatgggcat aagcccgtct ctggggtgga ggtagcacca ctgcagagct tcatgctgcg 4260
   gggtggtgtt gtagatgatc cagtcgtagc aggagcgctg ggcgtggtgc ctaaaaatgt 4320
   ctttcagtag caagctgatt gccaggggca ggcccttggt gtaagtgttt acaaagcggt 4380
   taagctggga tgggtgcata cgtggggata tgagatgcat cttggactgt atttttaggt 4440
   tggctatgtt cccagccata tccctccggg gattcatgtt gtgcagaacc accagcacag 4500
   tgtatccggt gcacttggga aatttgtcat gtagcttaga aggaaatgcg tggaagaact 4560
   tggagacgcc cttgtgacct ccaagatttt ccatgcattc gtccataatg atggcaatgg 4620
   gcccacgggc ggcggcctgg gcgaagatat ttctgggatc actaacgtca tagttgtgtt 4680
   ccaggatgag atcgtcatag gccattttta caaagcgcgg gcggagggtg ccagactgcg 4740
   gtataatggt tccatccggc ccaggggcgt agttaccctc acagatttgc atttcccacg 4800
   ctttgagttc agatgggggg atcatgtcta cctgcggggc gatgaagaaa acggtttccg 4860
   gggtagggga gatcagctgg gaagaaagca ggttcctgag cagctgcgac ttaccgcagc 4920
   cggtgggccc gtaaatcaca cctattaccg ggtgcaactg gtagttaaga gagctgcagc 4980
   tgccgtcatc cctgagcagg ggggccactt cgttaagcat gtccctgact cgcatgtttt 5040
   ccctgaccaa atccgccaga aggcgctcgc cgcccagcga tagcagttct tgcaaggaag 5100
   caaagttttt caacggtttg agaccgtccg ccgtaggcat gcttttgagc gtttgaccaa 5160
   gcagttccag gcggtcccac agctcggtca cctgctctac ggcatctcga tccagcatat 5220
   ctcctcgttt cgcgggttgg ggcggctttc gctgtacggc agtagtcggt gctcgtccag 5280
   acgggccagg gtcatgtctt tccacgggcg cagggtcctc gtcagcgtag tctgggtcac 5340
   ggtgaagggg tgcgctccgg gctgcgcgct ggccagggtg cgcttgaggc tggtcctgct 5400
   ggtgctgaag cgctgccggt cttcgccctg cgcgtcggcc aggtagcatt tgaccatggt 5460
   gtcatagtcc agcccctccg cggcgtggcc cttggcgcgc agcttgccct tggaggaggc 5520
   gccgcacgag gggcagtgca gacttttgag ggcgtagagc ttgggcgcga gaaataccga 5580
   ttccggggag taggcatccg cgccgcaggc cccgcagacg gtctcgcatt ccacgagcca 5640
   ggtgagctct ggccgttcgg ggtcaaaaac caggtttccc ccatgctttt tgatgcgttt 5700
   cttacctctg gtttccatga gccggtgtcc acgctcggtg acgaaaaggc tgtccgtgtc 5760
   cccgtataca gacttgagag gcctgtcctc gagcggtgtt ccgcggtcct cctcgtatag 5820
   aaactcggac cactctgaga caaaggctcg cgtccaggcc agcacgaagg aggctaagtg 5880
   ggaggggtag cggtcgttgt ccactagggg gtccactcgc tccagggtgt gaagacacat 5940
   gtcgccctct tcggcatcaa ggaaggtgat tggtttgtag gtgtaggcca cgtgaccggg 6000
   tgttcctgaa ggggggctat aaaagggggt gggggcgcgt tcgtcctcac tctcttccgc 6060
   atcgctgtct gcgagggcca gctgttgggg tgagtactcc ctctgaaaag cgggcatgac 6120
   ttctgcgcta agattgtcag tttccaaaaa cgaggaggat ttgatattca cctggcccgc 6180
   ggtgatgcct ttgagggtgg ccgcatccat ctggtcagaa aagacaatct ttttgttgtc 6240
   aagcttggtg gcaaacgacc cgtagagggc gttggacagc aacttggcga tggagcgcag 6300
   ggtttggttt ttgtcgcgat cggcgcgctc cttggccgcg atgtttagct gcacgtattc 6360
   gcgcgcaacg caccgccatt cgggaaagac ggtggtgcgc tcgtcgggca ccaggtgcac 6420
   gcgccaaccg cggttgtgca gggtgacaag gtcaacgctg gtggctacct ctccgcgtag 6480
   gcgctcgttg gtccagcaga ggcggccgcc cttgcgcgag cagaatggcg gtagggggtc 6540
   tagctgcgtc tcgtccgggg ggtctgcgtc cacggtaaag accccgggca gcaggcgcgc 6600
   gtcgaagtag tctatcttgc atccttgcaa gtctagcgcc tgctgccatg cgcgggcggc 6660
   aagcgcgcgc tcgtatgggt tgagtggggg accccatggc atggggtggg tgagcgcgga 6720
   ggcgtacatg ccgcaaatgt cgtaaacgta gaggggctct ctgagtattc caagatatgt 6780
   agggtagcat cttccaccgc ggatgctggc gcgcacgtaa tcgtatagtt cgtgcgaggg 6840
   agcgaggagg tcgggaccga ggttgctacg ggcgggctgc tctgctcgga agactatctg 6900
   cctgaagatg gcatgtgagt tggatgatat ggttggacgc tggaagacgt tgaagctggc 6960
   gtctgtgaga cctaccgcgt cacgcacgaa ggaggcgtag gagtcgcgca gcttgttgac 7020
   cagctcggcg gtgacctgca cgtctagggc gcagtagtcc agggtttcct tgatgatgtc 7080
   atacttatcc tgtccctttt ttttccacag ctcgcggttg aggacaaact cttcgcggtc 7140
   tttccagtac tcttggatcg gaaacccgtc ggcctccgaa cggtaagagc ctagcatgta 7200
   gaactggttg acggcctggt aggcgcagca tcccttttct acgggtagcg cgtatgcctg 7260
   cgcggccttc cggagcgagg tgtgggtgag cgcaaaggtg tccctgacca tgactttgag 7320
   gtactggtat ttgaagtcag tgtcgtcgca tccgccctgc tcccagagca aaaagtccgt 7380
   gcgctttttg gaacgcggat ttggcagggc gaaggtgaca tcgttgaaga gtatctttcc 7440
   cgcgcgaggc ataaagttgc gtgtgatgcg gaagggtccc ggcacctcgg aacggttgtt 7500
   aattacctgg gcggcgagca cgatctcgtc aaagccgttg atgttgtggc ccacaatgta 7560
   aagttccaag aagcgcggga tgcccttgat ggaaggcaat tttttaagtt cctcgtaggt 7620
   gagctcttca ggggagctga gcccgtgctc tgaaagggcc cagtctgcaa gatgagggtt 7680
   ggaagcgacg aatgagctcc acaggtcacg ggccattagc atttgcaggt ggtcgcgaaa 7740
   ggtcctaaac tggcgaccta tggccatttt ttctggggtg atgcagtaga aggtaagcgg 7800
   gtcttgttcc cagcggtccc atccaaggtt cgcggctagg tctcgcgcgg cagtcactag 7860
   aggctcatct ccgccgaact tcatgaccag catgaagggc acgagctgct tcccaaaggc 7920
   ccccatccaa gtataggtct ctacatcgta ggtgacaaag agacgctcgg tgcgaggatg 7980
   cgagccgatc gggaagaact ggatctcccg ccaccaattg gaggagtggc tattgatgtg 8040
   gtgaaagtag aagtccctgc gacgggccga acactcgtgc tggcttttgt aaaaacgtgc 8100
   gcagtactgg cagcggtgca cgggctgtac atcctgcacg aggttgacct gacgaccgcg 8160
   cacaaggaag cagagtggga atttgagccc ctcgcctggc gggtttggct ggtggtcttc 8220
   tacttcggct gcttgtcctt gaccgtctgg ctgctcgagg ggagttacgg tggatcggac 8280
   caccacgccg cgcgagccca aagtccagat gtccgcgcgc ggcggtcgga gcttgatgac 8340
   aacatcgcgc agatgggagc tgtccatggt ctggagctcc cgcggcgtca ggtcaggcgg 8400
   gagctcctgc aggtttacct cgcatagacg ggtcagggcg cgggctagat ccaggtgata 8460
   cctaatttcc aggggctggt tggtggcggc gtcgatggct tgcaagaggc cgcatccccg 8520
   cggcgcgact acggtaccgc gcggcgggcg gtgggccgcg ggggtgtcct tggatgatgc 8580
   atctaaaagc ggtgacgcgg gcgagccccc ggaggtaggg ggggctccgg acccgccggg 8640
   agagggggca ggggcacgtc ggcgccgcgc gcgggcagga gctggtgctg cgcgcgtagg 8700
   ttgctggcga acgcgacgac gcggcggttg atctcctgaa tctggcgcct ctgcgtgaag 8760
   acgacgggcc cggtgagctt gagcctgaaa gagagttcga cagaatcaat ttcggtgtcg 8820
   ttgacggcgg cctggcgcaa aatctcctgc acgtctcctg agttgtcttg ataggcgatc 8880
   tcggccatga actgctcgat ctcttcctcc tggagatctc cgcgtccggc tcgctccacg 8940
   gtggcggcga ggtcgttgga aatgcgggcc atgagctgcg agaaggcgtt gaggcctccc 9000
   tcgttccaga cgcggctgta gaccacgccc ccttcggcat cgcgggcgcg catgaccacc 9060
   tgcgcgagat tgagctccac gtgccgggcg aagacggcgt agtttcgcag gcgctgaaag 9120
   aggtagttga gggtggtggc ggtgtgttct gccacgaaga agtacataac ccagcgtcgc 9180
   aacgtggatt cgttgatatc ccccaaggcc tcaaggcgct ccatggcctc gtagaagtcc 9240
   acggcgaagt tgaaaaactg ggagttgcgc gccgacacgg ttaactcctc ctccagaaga 9300
   cggatgagct cggcgacagt gtcgcgcacc tcgcgctcaa aggctacagg ggcctcttct 9360
   tcttcttcaa tctcctcttc cataaggccc tccccttctt cttcttctgg cggcggtggg     9420
   ggagggggga cacggcggcg acgacggcgc accgggaggc ggtcgacaaa gcgctcgatc     9480
   atctccccgc ggcgacggcg catggtctcg gtgacggcgc ggccgttctc gcgggggcgc 9540
   agttggaaga cgccgcccgt catgtcccgg ttatgggttg gcggggggct gccatgcggc 9600
   agggatacgg cgctaacgat gcatctcaac aattgttgtg taggtactcc gccgccgagg 9660
   gacctgagcg agtccgcatc gaccggatcg gaaaacctct cgagaaaggc gtctaaccag 9720
   tcacagtcgc aaggtaggct gagcaccgtg gcgggcggca gcgggcggcg gtcggggttg 9780
   tttctggcgg aggtgctgct gatgatgtaa ttaaagtagg cggtcttgag acggcggatg 9840
   gtcgacagaa gcaccatgtc cttgggtccg gcctgctgaa tgcgcaggcg gtcggccatg 9900
   ccccaggctt cgttttgaca tcggcgcagg tctttgtagt agtcttgcat gagcctttct 9960
   accggcactt cttcttctcc ttcctcttgt cctgcatctc ttgcatctat cgctgcggcg 10020
   gcggcggagt ttggccgtag gtggcgccct cttcctccca tgcgtgtgac cccgaagccc 10080
   ctcatcggct gaagcagggc taggtcggcg acaacgcgct cggctaatat ggcctgctgc 10140
   acctgcgtga gggtagactg gaagtcatcc atgtccacaa agcggtggta tgcgcccgtg 10200
   ttgatggtgt aagtgcagtt ggccataacg gaccagttaa cggtctggtg acccggctgc 10260
   gagagctcgg tgtacctgag acgcgagtaa gccctcgagt caaatacgta gtcgttgcaa 10320
   gtccgcacca ggtactggta tcccaccaaa aagtgcggcg gcggctggcg gtagaggggc 10380
   cagcgtaggg tggccggggc tccgggggcg agatcttcca acataaggcg atgatatccg 10440
   tagatgtacc tggacatcca ggtgatgccg gcggcggtgg tggaggcgcg cggaaagtcg 10500
   cggacgcggt tccagatgtt gcgcagcggc aaaaagtgct ccatggtcgg gacgctctgg 10560
   ccggtcaggc gcgcgcaatc gttgacgctc tagaccgtgc aaaaggagag cctgtaagcg 10620
   ggcactcttc cgtggtctgg tggataaatt cgcaagggta tcatggcgga cgaccggggt 10680
   tcgagccccg tatccggccg tccgccgtga tccatgcggt taccgcccgc gtgtcgaacc 10740
   caggtgtgcg acgtcagaca acgggggagt gctccttttg gcttccttcc aggcgcggcg 10800
   gctgctgcgc tagctttttt ggccactggc cgcgcgcagc gtaagcggtt aggctggaaa 10860
   gcgaaagcat taagtggctc gctccctgta gccggagggt tattttccaa gggttgagtc 10920
   gcgggacccc cggttcgagt ctcggaccgg ccggactgcg gcgaacgggg gtttgcctcc 10980
   ccgtcatgca agaccccgct tgcaaattcc tccggaaaca gggacgagcc ccttttttgc 11040
   ttttcccaga tgcatccggt gctgcggcag atgcgccccc ctcctcagca gcggcaagag 11100
   caagagcagc ggcagacatg cagggcaccc tcccctcctc ctaccgcgtc aggaggggcg 11160
   acatccgcgg ttgacgcggc agcagatggt gattacgaac ccccgcggcg ccgggcccgg 11220
   cactacctgg acttggagga gggcgagggc ctggcgcggc taggagcgcc ctctcctgag 11280
   cggtacccaa gggtgcagct gaagcgtgat acgcgtgagg cgtacgtgcc gcggcagaac 11340
   ctgtttcgcg accgcgaggg agaggagccc gaggagatgc gggatcgaaa gttccacgca 11400
   gggcgcgagc tgcggcatgg cctgaatcgc gagcggttgc tgcgcgagga ggactttgag 11460
   cccgacgcgc gaaccgggat tagtcccgcg cgcgcacacg tggcggccgc cgacctggta 11520
   accgcatacg agcagacggt gaaccaggag attaactttc aaaaaagctt taacaaccac 11580
   gtgcgtacgc ttgtggcgcg cgaggaggtg gctataggac tgatgcatct gtgggacttt 11640
   gtaagcgcgc tggagcaaaa cccaaatagc aagccgctca tggcgcagct gttccttata 11700
   gtgcagcaca gcagggacaa cgaggcattc agggatgcgc tgctaaacat agtagagccc 11760
   gagggccgct ggctgctcga tttgataaac atcctgcaga gcatagtggt gcaggagcgc 11820
   agcttgagcc tggctgacaa ggtggccgcc atcaactatt ccatgcttag cctgggcaag 11880
   ttttacgccc gcaagatata ccatacccct tacgttccca tagacaagga ggtaaagatc 11940
   gaggggttct acatgcgcat ggcgctgaag gtgcttacct tgagcgacga cctgggcgtt 12000
   tatcgcaacg agcgcatcca caaggccgtg agcgtgagcc ggcggcgcga gctcagcgac 12060
   cgcgagctga tgcacagcct gcaaagggcc ctggctggca cgggcagcgg cgatagagag 12120
   gccgagtcct actttgacgc gggcgctgac ctgcgctggg ccccaagccg acgcgccctg 12180
   gaggcagctg gggccggacc tgggctggcg gtggcacccg cgcgcgctgg caacgtcggc 12240
   ggcgtggagg aatatgacga ggacgatgag tacgagccag aggacggcga gtactaagcg 12300
   gtgatgtttc tgatcagatg atgcaagacg caacggaccc ggcggtgcgg gcggcgctgc 12360
   agagccagcc gtccggcctt aactccacgg acgactggcg ccaggtcatg gaccgcatca 12420
   tgtcgctgac tgcgcgcaat cctgacgcgt tccggcagca gccgcaggcc aaccggctct 12480
   ccgcaattct ggaagcggtg gtcccggcgc gcgcaaaccc cacgcacgag aaggtgctgg 12540
   cgatcgtaaa cgcgctggcc gaaaacaggg ccatccggcc cgacgaggcc ggcctggtct 12600
   acgacgcgct gcttcagcgc gtggctcgtt acaacagcgg caacgtgcag accaacctgg 12660
   accggctggt gggggatgtg cgcgaggccg tggcgcagcg tgagcgcgcg cagcagcagg 12720
   gcaacctggg ctccatggtt gcactaaacg ccttcctgag tacacagccc gccaacgtgc 12780
   cgcggggaca ggaggactac accaactttg tgagcgcact gcggctaatg gtgactgaga 12840
   caccgcaaag tgaggtgtac cagtctgggc cagactattt tttccagacc agtagacaag 12900
   gcctgcagac cgtaaacctg agccaggctt tcaaaaactt gcaggggctg tggggggtgc 12960
   gggctcccac aggcgaccgc gcgaccgtgt ctagcttgct gacgcccaac tcgcgcctgt 13020
   tgctgctgct aatagcgccc ttcacggaca gtggcagcgt gtcccgggac acatacctag 13080
   gtcacttgct gacactgtac cgcgaggcca taggtcaggc gcatgtggac gagcatactt 13140
   tccaggagat tacaagtgtc agccgcgcgc tggggcagga ggacacgggc agcctggagg 13200
   caaccctaaa ctacctgctg accaaccggc ggcagaagat cccctcgttg cacagtttaa 13260
   acagcgagga ggagcgcatt ttgcgctacg tgcagcagag cgtgagcctt aacctgatgc 13320
   gcgacggggt aacgcccagc gtggcgctgg acatgaccgc gcgcaacatg gaaccgggca 13380
   tgtatgcctc aaaccggccg tttatcaacc gcctaatgga ctacttgcat cgcgcggccg 13440
   ccgtgaaccc cgagtatttc accaatgcca tcttgaaccc gcactggcta ccgccccctg 13500
   gtttctacac cgggggattc gaggtgcccg agggtaacga tggattcctc tgggacgaca 13560
   tagacgacag cgtgttttcc ccgcaaccgc agaccctgct agagttgcaa cagcgcgagc 13620
   aggcagaggc ggcgctgcga aaggaaagct tccgcaggcc aagcagcttg tccgatctag 13680
   gcgctgcggc cccgcggtca gatgctagta gcccatttcc aagcttgata gggtctctta 13740
   ccagcactcg caccacccgc ccgcgcctgc tgggcgagga ggagtaccta aacaactcgc 13800
   tgctgcagcc gcagcgcgaa aaaaacctgc ctccggcatt tcccaacaac gggatagaga 13860
   gcctagtgga caagatgagt agatggaaga cgtacgcgca ggagcacagg gacgtgccag 13920
   gcccgcgccc gcccacccgt cgtcaaaggc acgaccgtca gcggggtctg gtgtgggagg 13980
   acgatgactc ggcagacgac agcagcgtcc tggatttggg agggagtggc aacccgtttg 14040
   cgcaccttcg ccccaggctg gggagaatgt tttaaaaaaa aaaaagcatg atgcaaaata 14100
   aaaaactcac caaggccatg gcaccgagcg ttggttttct tgtattcccc ttagtatgcg 14160
   gcgcgcggcg atgtatgagg aaggtcctcc tccctcctac gagagtgtgg tgagcgcggc 14220
   gccagtggcg gcggcgctgg gttctccctt cgatgctccc ctggacccgc cgtttgtgcc 14280
   tccgcggtac ctgcggccta ccggggggag aaacagcatc cgttactctg agttggcacc 14340
   cctattcgac accacccgtg tgtacctggt ggacaacaag tcaacggatg tggcatccct 14400
   gaactaccag aacgaccaca gcaactttct gaccacggtc attcaaaaca atgactacag 14460
   cccgggggag gcaagcacac agaccatcaa tcttgacgac cggtcgcact ggggcggcga 14520
   cctgaaaacc atcctgcata ccaacatgcc aaatgtgaac gagttcatgt ttaccaataa 14580
   gtttaaggcg cgggtgatgg tgtcgcgctt gcctactaag gacaatcagg tggagctgaa 14640
   atacgagtgg gtggagttca cgctgcccga gggcaactac tccgagacca tgaccataga 14700
   ccttatgaac aacgcgatcg tggagcacta cttgaaagtg ggcagacaga acggggttct 14760
   ggaaagcgac atcggggtaa agtttgacac ccgcaacttc agactggggt ttgaccccgt 14820
   cactggtctt gtcatgcctg gggtatatac aaacgaagcc ttccatccag acatcatttt 14880
   gctgccagga tgcggggtgg acttcaccca cagccgcctg agcaacttgt tgggcatccg 14940
   caagcggcaa cccttccagg agggctttag gatcacctac gatgatctgg agggtggtaa 15000
   cattcccgca ctgttggatg tggacgccta ccaggcgagc ttgaaagatg acaccgaaca 15060
   gggcgggggt ggcgcaggcg gcagcaacag cagtggcagc ggcgcggaag agaactccaa 15120
   cgcggcagcc gcggcaatgc agccggtgga ggacatgaac gatcatgcca ttcgcggcga 15180
   cacctttgcc acacgggctg aggagaagcg cgctgaggcc gaagcagcgg ccgaagctgc 15240
   cgcccccgct gcgcaacccg aggtcgagaa gcctcagaag aaaccggtga tcaaacccct 15300
   gacagaggac agcaagaaac gcagttacaa cctaataagc aatgacagca ccttcaccca 15360
   gtaccgcagc tggtaccttg catacaacta cggcgaccct cagaccggaa tccgctcatg 15420
   gaccctgctt tgcactcctg acgtaacctg cggctcggag caggtctact ggtcgttgcc 15480
   agacatgatg caagaccccg tgaccttccg ctccacgcgc cagatcagca actttccggt 15540
   ggtgggcgcc gagctgttgc ccgtgcactc caagagcttc tacaacgacc aggccgtcta 15600
   ctcccaactc atccgccagt ttacctctct gacccacgtg ttcaatcgct ttcccgagaa 15660
   ccagattttg gcgcgcccgc cagcccccac catcaccacc gtcagtgaaa acgttcctgc 15720
   tctcacagat cacgggacgc taccgctgcg caacagcatc ggaggagtcc agcgagtgac 15780
   cattactgac gccagacgcc gcacctgccc ctacgtttac aaggccctgg gcatagtctc 15840
   gccgcgcgtc ctatcgagcc gcactttttg agcaagcatg tccatcctta tatcgcccag 15900
   caataacaca ggctggggcc tgcgcttccc aagcaagatg tttggcgggg ccaagaagcg 15960
   ctccgaccaa cacccagtgc gcgtgcgcgg gcactaccgc gcgccctggg gcgcgcacaa 16020
   acgcggccgc actgggcgca ccaccgtcga tgacgccatc gacgcggtgg tggaggaggc 16080
   gcgcaactac acgcccacgc cgccaccagt gtccacagtg gacgcggcca ttcagaccgt 16140
   ggtgcgcgga gcccggcgct atgctaaaat gaagagacgg cggaggcgcg tagcacgtcg 16200
   ccaccgccgc cgacccggca ctgccgccca acgcgcggcg gcggccctgc ttaaccgcgc 16260
   acgtcgcacc ggccgacggg cggccatgcg ggccgctcga aggctggccg cgggtattgt 16320
   cactgtgccc cccaggtcca ggcgacgagc ggccgccgca gcagccgcgg ccattagtgc 16380
   tatgactcag ggtcgcaggg gcaacgtgta ttgggtgcgc gactcggtta gcggcctgcg 16440
   cgtgcccgtg cgcacccgcc ccccgcgcaa ctagattgca agaaaaaact acttagactc 16500
   gtactgttgt atgtatccag cggcggcggc gcgcaacgaa gctatgtcca agcgcaaaat 16560
   caaagaagag atgctccagg tcatcgcgcc ggagatctat ggccccccga agaaggaaga 16620
   gcaggattac aagccccgaa agctaaagcg ggtcaaaaag aaaaagaaag atgatgatga 16680
   tgaacttgac gacgaggtgg aactgctgca cgctaccgcg cccaggcgac gggtacagtg 16740
   gaaaggtcga cgcgtaaaac gtgttttgcg acccggcacc accgtagtct ttacgcccgg 16800
   tgagcgctcc acccgcacct acaagcgcgt gtatgatgag gtgtacggcg acgaggacct 16860
   gcttgagcag gccaacgagc gcctcgggga gtttgcctac ggaaagcggc ataaggacat 16920
   gctggcgttg ccgctggacg agggcaaccc aacacctagc ctaaagcccg taacactgca 16980
   gcaggtgctg cccgcgcttg caccgtccga agaaaagcgc ggcctaaagc gcgagtctgg 17040
   tgacttggca cccaccgtgc agctgatggt acccaagcgc cagcgactgg aagatgtctt 17100
   ggaaaaaatg accgtggaac ctgggctgga gcccgaggtc cgcgtgcggc caatcaagca 17160
   ggtggcgccg ggactgggcg tgcagaccgt ggacgttcag atacccacta ccagtagcac 17220
   cagtattgcc accgccacag agggcatgga gacacaaacg tccccggttg cctcagcggt 17280
   ggcggatgcc gcggtgcagg cggtcgctgc ggccgcgtcc aagacctcta cggaggtgca 17340
   aacggacccg tggatgtttc gcgtttcagc cccccggcgc ccgcgcggtt cgaggaagta 17400
   cggcgccgcc agcgcgctac tgcccgaata tgccctacat ccttccattg cgcctacccc 17460
   cggctatcgt ggctacacct accgccccag aagacgagca actacccgac gccgaaccac 17520
   cactggaacc cgccgccgcc gtcgccgtcg ccagcccgtg ctggccccga tttccgtgcg 17580
   cagggtggct cgcgaaggag gcaggaccct ggtgctgcca acagcgcgct accaccccag 17640
   catcgtttaa aagccggtct ttgtggttct tgcagatatg gccctcacct gccgcctccg 17700
   tttcccggtg ccgggattcc gaggaagaat gcaccgtagg aggggcatgg ccggccacgg 17760
   cctgacgggc ggcatgcgtc gtgcgcacca ccggcggcgg cgcgcgtcgc accgtcgcat 17820
   gcgcggcggt atcctgcccc tccttattcc actgatcgcc gcggcgattg gcgccgtgcc 17880
   cggaattgca tccgtggcct tgcaggcgca gagacactga ttaaaaacaa gttgcatgtg 17940
   gaaaaatcaa aataaaaagt ctggactctc acgctcgctt ggtcctgtaa ctattttgta 18000
   gaatggaaga catcaacttt gcgtctctgg ccccgcgaca cggctcgcgc ccgttcatgg 18060
   gaaactggca agatatcggc accagcaata tgagcggtgg cgccttcagc tggggctcgc 18120
   tgtggagcgg cattaaaaat ttcggttcca ccgttaagaa ctatggcagc aaggcctgga 18180
   acagcagcac aggccagatg ctgagggata agttgaaaga gcaaaatttc caacaaaagg 18240
   tggtagatgg cctggcctct ggcattagcg gggtggtgga cctggccaac caggcagtgc 18300
   aaaataagat taacagtaag cttgatcccc gccctcccgt agaggagcct ccaccggccg 18360
   tggagacagt gtctccagag ggcgtggcgg aaaagcgtcc gcgccccgac agggaagaaa 18420
   ctctggtgac gcaaatagac gagcctccct cgtacgagga ggcactaaag caaggcctgc 18480
   ccaccacccg tcccatcgcg cccatggcta ccggagtgct gggccagcac acacccgtaa 18540
   cgctggacct gcctcccccc gccgacaccc agcagaaacc tgtgctgcca ggcccgaccg 18600
   ccgttgttgt aacccgtcct agccgcgcgt ccctgcgccg cgccgccagc ggtccgcgat 18660
   cgttgcggcc cgtagccagt ggcaactggc aaagcacact gaacagcatc gtgggtctgg 18720
   gggtgcaatc cctgaagcgc cgacgatgct tctgaatagc taacgtgtcg tatgtgtgtc 18780
   atgtatgcgt ccatgtcgcc gccagaggag ctgctgagcc gccgcgcgcc cgctttccaa 18840
   gatggctacc ccttcgatga tgccgcagtg gtcttacatg cacatctcgg gccaggacgc 18900
   ctcggagtac ctgagccccg ggctggtgca gtttgcccgc gccaccgaga cgtacttcag 18960
   cctgaataac aagtttagaa accccacggt ggcgcctacg cacgacgtga ccacagaccg 19020
   gtcccagcgt ttgacgctgc ggttcatccc tgtggaccgt gaggatactg cgtactcgta 19080
   caaggcgcgg ttcaccctag ctgtgggtga taaccgtgtg ctggacatgg cttccacgta 19140
   ctttgacatc cgcggcgtgc tggacagggg ccctactttt aagccctact ctggcactgc 19200
   ctacaacgcc ctggctccca agggtgcccc aaatccttgc gaatgggatg aagctgctac 19260
   tgctcttgaa ataaacctag aagaagagga cgatgacaac gaagacgaag tagacgagca 19320
   agctgagcag caaaaaactc acgtatttgg gcaggcgcct tattctggta taaatattac 19380
   aaaggagggt attcaaatag gtgtcgaagg tcaaacacct aaatatgccg ataaaacatt 19440
   tcaacctgaa cctcaaatag gagaatctca gtggtacgaa actgaaatta atcatgcagc 19500
   tgggagagtc cttaaaaaga ctaccccaat gaaaccatgt tacggttcat atgcaaaacc 19560
   cacaaatgaa aatggagggc aaggcattct tgtaaagcaa caaaatggaa agctagaaag 19620
   tcaagtggaa atgcaatttt tctcaactac tgaggcgacc gcaggcaatg gtgataactt 19680
   gactcctaaa gtggtattgt acagtgaaga tgtagatata gaaaccccag acactcatat 19740
   ttcttacatg cccactatta aggaaggtaa ctcacgagaa ctaatgggcc aacaatctat 19800
   gcccaacagg cctaattaca ttgcttttag ggacaatttt attggtctaa tgtattacaa 19860
   cagcacgggt aatatgggtg ttctggcggg ccaagcatcg cagttgaatg ctgttgtaga 19920
   tttgcaagac agaaacacag agctttcata ccagcttttg cttgattcca ttggtgatag 19980
   aaccaggtac ttttctatgt ggaatcaggc tgttgacagc tatgatccag atgttagaat 20040
   tattgaaaat catggaactg aagatgaact tccaaattac tgctttccac tgggaggtgt 20100
   gattaataca gagactctta ccaaggtaaa acctaaaaca ggtcaggaaa atggatggga 20160
   aaaagatgct acagaatttt cagataaaaa tgaaataaga gttggaaata attttgccat 20220
   ggaaatcaat ctaaatgcca acctgtggag aaatttcctg tactccaaca tagcgctgta 20280
   tttgcccgac aagctaaagt acagtccttc caacgtaaaa atttctgata acccaaacac 20340
   ctacgactac atgaacaagc gagtggtggc tcccgggtta gtggactgct acattaacct 20400
   tggagcacgc tggtcccttg actatatgga caacgtcaac ccatttaacc accaccgcaa 20460
   tgctggcctg cgctaccgct caatgttgct gggcaatggt cgctatgtgc ccttccacat 20520
   ccaggtgcct cagaagttct ttgccattaa aaacctcctt ctcctgccgg gctcatacac 20580
   ctacgagtgg aacttcagga aggatgttaa catggttctg cagagctccc taggaaatga 20640
   cctaagggtt gacggagcca gcattaagtt tgatagcatt tgcctttacg ccaccttctt 20700
   ccccatggcc cacaacaccg cctccacgct tgaggccatg cttagaaacg acaccaacga 20760
   ccagtccttt aacgactatc tctccgccgc caacatgctc taccctatac ccgccaacgc 20820
   taccaacgtg cccatatcca tcccctcccg caactgggcg gctttccgcg gctgggcctt 20880
   cacgcgcctt aagactaagg aaaccccatc actgggctcg ggctacgacc cttattacac 20940
   ctactctggc tctataccct acctagatgg aaccttttac ctcaaccaca cctttaagaa 21000
   ggtggccatt acctttgact cttctgtcag ctggcctggc aatgaccgcc tgcttacccc 21060
   caacgagttt gaaattaagc gctcagttga cggggagggt tacaacgttg cccagtgtaa 21120
   catgaccaaa gactggttcc tggtacaaat gctagctaac tacaacattg gctaccaggg 21180
   cttctatatc ccagagagct acaaggaccg catgtactcc ttctttagaa acttccagcc 21240
   catgagccgt caggtggtgg atgatactaa atacaaggac taccaacagg tgggcatcct 21300
   acaccaacac aacaactctg gatttgttgg ctaccttgcc cccaccatgc gcgaaggaca 21360
   ggcctaccct gctaacttcc cctatccgct tataggcaag accgcagttg acagcattac 21420
   ccagaaaaag tttctttgcg atcgcaccct ttggcgcatc ccattctcca gtaactttat 21480
   gtccatgggc gcactcacag acctgggcca aaaccttctc tacgccaact ccgcccacgc 21540
   gctagacatg acttttgagg tggatcccat ggacgagccc acccttcttt atgttttgtt 21600
   tgaagtcttt gacgtggtcc gtgtgcaccg gccgcaccgc ggcgtcatcg aaaccgtgta 21660
   cctgcgcacg cccttctcgg ccggcaacgc cacaacataa agaagcaagc aacatcaaca 21720
   acagctgccg ccatgggctc cagtgagcag gaactgaaag ccattgtcaa agatcttggt 21780
   tgtgggccat atttttgggc cacctatgac aagcgctttc caggctttgt ttctccacac 21840
   aagctcgcct gcgccatagt caatacggcc ggtcgcgaga ctgggggcgt acactggatg 21900
   gcctttgcct ggaacccgca ctcaaaaaca tgctacctct ttgagccctt tggcttttct 21960
   gaccagcgac tcaagcaggt ttaccagttt gagtacgagt cactcctgcg ccgtagcgcc 22020
   attgcttctt cccccgaccg ctgtataacg ctggaaaagt ccacccaaag cgtacagggg 22080
   cccaactcgg ccgcctgtgg actattctgc tgcatgtttc tccacgcctt tgccaactgg 22140
   ccccaaactc ccatggatca caaccccacc atgaacctta ttaccggggt acccaactcc 22200
   atgctcaaca gtccccaggt acagcccacc ctgcgtcgca accaggaaca gctctacagc 22260
   ttcctggagc gccactcgcc ctacttccgc agccacagtg cgcagattag gagcgccact 22320
   tctttttgtc acttgaaaaa catgtaaaaa taatgtacta gagacacttt caataaaggc 22380
   aaatgctttt atttgtacac tctcgggtga ttatttaccc ccacccttgc cgtctgcgcc 22440
   gtttaaaaat caaaggggtt ctgccgcgca tcgctatgcg ccactggcag ggacacgttg 22500
   cgatactggt gtttagtgct ccacttaaac tcaggcacaa ccatccgcgg cagctcggtg 22560
   aagttttcac tccacaggct gcgcaccatc accaacgcgt ttagcaggtc gggcgccgat 22620
   atcttgaagt cgcagttggg gcctccgccc tgcgcgcgcg agttgcgata cacagggttg 22680
   cagcactgga acactatcag cgccgggtgg tgcacgctgg ccagcacgct cttgtcggag 22740
   atcagatccg cgtccaggtc ctccgcgttg ctcagggcga acggagtcaa ctttggtagc 22800
   tgccttccca aaaagggcgc gtgcccaggc tttgagttgc actcgcaccg tagtggcatc 22860
   aaaaggtgac cgtgcccggt ctgggcgtta ggatacagcg cctgcataaa agccttgatc 22920
   tgcttaaaag ccacctgagc ctttgcgcct tcagagaaga acatgccgca agacttgccg 22980
   gaaaactgat tggccggaca ggccgcgtcg tgcacgcagc accttgcgtc ggtgttggag 23040
   atctgcacca catttcggcc ccaccggttc ttcacgatct tggccttgct agactgctcc 23100
   ttcagcgcgc gctgcccgtt ttcgctcgtc acatccattt caatcacgtg ctccttattt 23160
   atcataatgc ttccgtgtag acacttaagc tcgccttcga tctcagcgca gcggtgcagc 23220
   cacaacgcgc agcccgtggg ctcgtgatgc ttgtaggtca cctctgcaaa cgactgcagg 23280
   tacgcctgca ggaatcgccc catcatcgtc acaaaggtct tgttgctggt gaaggtcagc 23340
   tgcaacccgc ggtgctcctc gttcagccag gtcttgcata cggccgccag agcttccact 23400
   tggtcaggca gtagtttgaa gttcgccttt agatcgttat ccacgtggta cttgtccatc 23460
   agcgcgcgcg cagcctccat gcccttctcc cacgcagaca cgatcggcac actcagcggg 23520
   ttcatcaccg taatttcact ttccgcttcg ctgggctctt cctcttcctc ttgcgtccgc 23580
   ataccacgcg ccactgggtc gtcttcattc agccgccgca ctgtgcgctt acctcctttg 23640
   ccatgcttga ttagcaccgg tgggttgctg aaacccacca tttgtagcgc cacatcttct 23700
   ctttcttcct cgctgtccac gattacctct ggtgatggcg ggcgctcggg cttgggagaa 23760
   gggcgcttct ttttcttctt gggcgcaatg gccaaatccg ccgccgaggt cgatggccgc 23820
   gggctgggtg tgcgcggcac cagcgcgtct tgtgatgagt cttcctcgtc ctcggactcg 23880
   atacgccgcc tcatccgctt ttttgggggc gcccggggag gcggcggcga cggggacggg 23940
   gacgacacgt cctccatggt tgggggacgt cgcgccgcac cgcgtccgcg ctcgggggtg 24000
   gtttcgcgct gctcctcttc ccgactggcc atttccttct cctataggca gaaaaagatc 24060
   atggagtcag tcgagaagaa ggacagccta accgccccct ctgagttcgc caccaccgcc 24120
   tccaccgatg ccgccaacgc gcctaccacc ttccccgtcg aggcaccccc gcttgaggag 24180
   gaggaagtga ttatcgagca ggacccaggt tttgtaagcg aagacgacga ggaccgctca 24240
   gtaccaacag aggataaaaa gcaagaccag gacaacgcag aggcaaacga ggaacaagtc 24300
   gggcgggggg acgaaaggca tggcgactac ctagatgtgg gagacgacgt gctgttgaag 24360
   catctgcagc gccagtgcgc cattatctgc gacgcgttgc aagagcgcag cgatgtgccc 24420
   ctcgccatag cggatgtcag ccttgcctac gaacgccacc tattctcacc gcgcgtaccc 24480
   cccaaacgcc aagaaaacgg cacatgcgag cccaacccgc gcctcaactt ctaccccgta 24540
   tttgccgtgc cagaggtgct tgccacctat cacatctttt tccaaaactg caagataccc 24600
   ctatcctgcc gtgccaaccg cagccgagcg gacaagcagc tggccttgcg gcagggcgct 24660
   gtcatacctg atatcgcctc gctcaacgaa gtgccaaaaa tctttgaggg tcttggacgc 24720
   gacgagaagc gcgcggcaaa cgctctgcaa caggaaaaca gcgaaaatga aagtcactct 24780
   ggagtgttgg tggaactcga gggtgacaac gcgcgcctag ccgtactaaa acgcagcatc 24840
   gaggtcaccc actttgccta cccggcactt aacctacccc ccaaggtcat gagcacagtc 24900
   atgagtgagc tgatcgtgcg ccgtgcgcag cccctggaga gggatgcaaa tttgcaagaa 24960
   caaacagagg agggcctacc cgcagttggc gacgagcagc tagcgcgctg gcttcaaacg 25020
   cgcgagcctg ccgacttgga ggagcgacgc aaactaatga tggccgcagt gctcgttacc 25080
   gtggagcttg agtgcatgca gcggttcttt gctgacccgg agatgcagcg caagctagag 25140
   gaaacattgc actacacctt tcgacagggc tacgtacgcc aggcctgcaa gatctccaac 25200
   gtggagctct gcaacctggt ctcctacctt ggaattttgc acgaaaaccg ccttgggcaa 25260
   aacgtgcttc attccacgct caagggcgag gcgcgccgcg actacgtccg cgactgcgtt 25320
   tacttatttc tatgctacac ctggcagacg gccatgggcg tttggcagca gtgcttggag 25380
   gagtgcaacc tcaaggagct gcagaaactg ctaaagcaaa acttgaagga cctatggacg 25440
   gccttcaacg agcgctccgt ggccgcgcac ctggcggaca tcattttccc cgaacgcctg 25500
   cttaaaaccc tgcaacaggg tctgccagac ttcaccagtc aaagcatgtt gcagaacttt 25560
   aggaacttta tcctagagcg ctcaggaatc ttgcccgcca cctgctgtgc acttcctagc 25620
   gactttgtgc ccattaagta ccgcgaatgc cctccgccgc tttggggcca ctgctacctt 25680
   ctgcagctag ccaactacct tgcctaccac tctgacataa tggaagacgt gagcggtgac 25740
   ggtctactgg agtgtcactg tcgctgcaac ctatgcaccc cgcaccgctc cctggtttgc 25800
   aattcgcagc tgcttaacga aagtcaaatt atcggtacct ttgagctgca gggtccctcg 25860
   cctgacgaaa agtccgcggc tccggggttg aaactcactc cggggctgtg gacgtcggct 25920
   taccttcgca aatttgtacc tgaggactac cacgcccacg agattaggtt ctacgaagac 25980
   caatcccgcc cgccaaatgc ggagcttacc gcctgcgtca ttacccaggg ccacattctt 26040
   ggccaattgc aagccatcaa caaagcccgc caagagtttc tgctacgaaa gggacggggg 26100
   gtttacttgg acccccagtc cggcgaggag ctcaacccaa tccccccgcc gccgcagccc 26160
   tatcagcagc agccgcgggc ccttgcttcc caggatggca cccaaaaaga agctgcagct 26220
   gccgccgcca cccacggacg aggaggaata ctgggacagt caggcagagg aggttttgga 26280
   cgaggaggag gaggacatga tggaagactg ggagagccta gacgaggaag cttccgaggt 26340
   cgaagaggtg tcagacgaaa caccgtcacc ctcggtcgca ttcccctcgc cggcgcccca 26400
   gaaatcggca accggttcca gcatggctac aacctccgct cctcaggcgc cgccggcact 26460
   gcccgttcgc cgacccaacc gtagatggga caccactgga accagggccg gtaagtccaa 26520
   gcagccgccg ccgttagccc aagagcaaca acagcgccaa ggctaccgct catggcgcgg 26580
   gcacaagaac gccatagttg cttgcttgca agactgtggg ggcaacatct ccttcgcccg 26640
   ccgctttctt ctctaccatc acggcgtggc cttcccccgt aacatcctgc attactaccg 26700
   tcatctctac agcccatact gcaccggcgg cagcggcagc ggcagcaaca gcagcggcca 26760
   cacagaagca aaggcgaccg gatagcaaga ctctgacaaa gcccaagaaa tccacagcgg 26820
   cggcagcagc aggaggagga gcgctgcgtc tggcgcccaa cgaacccgta tcgacccgcg 26880
   agcttagaaa caggattttt cccactctgt atgctatatt tcaacagagc aggggccaag 26940
   aacaagagct gaaaataaaa aacaggtctc tgcgatccct cacccgcagc tgcctgtatc 27000
   acaaaagcga agatcagctt cggcgcacgc tggaagacgc ggaggctctc ttcagtaaat 27060
   actgcgcgct gactcttaag gactagtttc gcgccctttc tcaaatttaa gcgcgaaaac 27120
   tacgtcatct ccagcggcca cacccggcgc cagcacctgt cgtcagcgcc attatgagca 27180
   aggaaattcc cacgccctac atgtggagtt accagccaca aatgggactt gcggctggag 27240
   ctgcccaaga ctactcaacc cgaataaact acatgagcgc gggaccccac atgatatccc 27300
   gggtcaacgg aatccgcgcc caccgaaacc gaattctctt ggaacaggcg gctattacca 27360
   ccacacctcg taataacctt aatccccgta gttggcccgc tgccctggtg taccaggaaa 27420
   gtcccgctcc caccactgtg gtacttccca gagacgccca ggccgaagtt cagatgacta 27480
   actcaggggc gcagcttgcg ggcggctttc gtcacagggt gcggtcgccc gggcagggta 27540
   taactcacct gacaatcaga gggcgaggta ttcagctcaa cgacgagtcg gtgagctcct 27600
   cgcttggtct ccgtccggac gggacatttc agatcggcgg cgccggccgt ccttcattca 27660
   cgcctcgtca ggcaatccta actctgcaga cctcgtcctc tgagccgcgc tctggaggca 27720
   ttggaactct gcaatttatt gaggagtttg tgccatcggt ctactttaac cccttctcgg 27780
   gacctcccgg ccactatccg gatcaattta ttcctaactt tgacgcggta aaggactcgg 27840
   cggacggcta cgactgaatg ttaagtggag aggcagagca actgcgcctg aaacacctgg 27900
   tccactgtcg ccgccacaag tgctttgccc gcgactccgg tgagttttgc tactttgaat 27960
   tgcccgagga tcatatcgag ggcccggcgc acggcgtccg gcttaccgcc cagggagagc 28020
   ttgcccgtag cctgattcgg gagtttaccc agcgccccct gctagttgag cgggacaggg 28080
   gaccctgtgt tctcactgtg atttgcaact gtcctaacct tggattacat caagatcttt 28140
   gttgccatct ctgtgctgag tataataaat acagaaatta aaatatactg gggctcctat 28200
   cgccatcctg taaacgccac cgtcttcacc cgcccaagca aaccaaggcg aaccttacct 28260
   ggtactttta acatctctcc ctctgtgatt tacaacagtt tcaacccaga cggagtgagt 28320
   ctacgagaga acctctccga gctcagctac tccatcagaa aaaacaccac cctccttacc 28380
   tgccgggaac gtacgagtgc gtcaccggcc gctgcaccac acctaccgcc tgaccgtaaa 28440
   ccagactttt tccggacaga cctcaataac tctgtttacc agaacaggag gtgagcttag 28500
   aaaaccctta gggtattagg ccaaaggcgc agctactgtg gggtttatga acaattcaag 28560
   caactctacg ggctattcta attcaggttt ctctagaatc ggggttgggg ttattctctg 28620
   tcttgtgatt ctctttattc ttatactaac gcttctctgc ctaaggctcg ccgcctgctg 28680
   tgtgcacatt tgcatttatt gtcagctttt taaacgctgg ggtcgccacc caagatgatt 28740
   aggtacataa tcctaggttt actcaccctt gcgtcagccc acggtaccac ccaaaaggtg 28800
   gattttaagg agccagcctg taatgttaca ttcgcagctg aagctaatga gtgcaccact 28860
   cttataaaat gcaccacaga acatgaaaag ctgcttattc gccacaaaaa caaaattggc 28920
   aagtatgctg tttatgctat ttggcagcca ggtgacacta cagagtataa tgttacagtt 28980
   ttccagggta aaagtcataa aacttttatg tatacttttc cattttatga aatgtgcgac 29040
   attaccatgt acatgagcaa acagtataag ttgtggcccc cacaaaattg tgtggaaaac 29100
   actggcactt tctgctgcac tgctatgcta attacagtgc tcgctttggt ctgtacccta 29160
   ctctatatta aatacaaaag cagacgcagc tttattgagg aaaagaaaat gccttaattt 29220
   actaagttac aaagctaatg tcaccactaa ctgctttact cgctgcttgc aaaacaaatt 29280
   caaaaagtta gcattataat tagaatagga tttaaacccc ccggtcattt cctgctcaat 29340
   accattcccc tgaacaattg actctatgtg ggatatgctc cagcgctaca accttgaagt 29400
   caggcttcct ggatgtcagc atctgacttt ggccagcacc tgtcccgcgg atttgttcca 29460
   gtccaactac agcgacccac cctaacagag atgaccaaca caaccaacgc ggccgccgct 29520
   accggactta catctaccac aaatacaccc caagtttctg cctttgtcaa taactgggat 29580
   aacttgggca tgtggtggtt ctccatagcg cttatgtttg tatgccttat tattatgtgg 29640
   ctcatctgct gcctaaagcg caaacgcgcc cgaccaccca tctatagtcc catcattgtg 29700
   ctacacccaa acaatgatgg aatccataga ttggacggac tgaaacacat gttcttttct 29760
   cttacagtat gattaaatga gacatgattc ctcgagtttt tatattactg acccttgttg 29820
   cgcttttttg tgcgtgctcc acattggctg cggtttctca catcgaagta gactgcattc 29880
   cagccttcac agtctatttg ctttacggat ttgtcaccct cacgctcatc tgcagcctca 29940
   tcactgtggt catcgccttt atccagtgca ttgactgggt ctgtgtgcgc tttgcatatc 30000
   tcagacacca tccccagtac agggacagga ctatagctga gcttcttaga attctttaat 30060
   tatgaaattt actgtgactt ttctgctgat tatttgcacc ctatctgcgt tttgttcccc 30120
   gacctccaag cctcaaagac atatatcatg cagattcact cgtatatgga atattccaag 30180
   ttgctacaat gaaaaaagcg atctttccga agcctggtta tatgcaatca tctctgttat 30240
   ggtgttctgc agtaccatct tagccctagc tatatatccc taccttgaca ttggctggaa 30300
   acgaatagat gccatgaacc acccaacttt ccccgcgccc gctatgcttc cactgcaaca 30360
   agttgttgcc ggcggctttg tcccagccaa tcagcctcgc cccacttctc ccacccccac 30420
   tgaaatcagc tactttaatc taacaggagg agatgactga caccctagat ctagaaatgg 30480
   acggaattat tacagagcag cgcctgctag aaagacgcag ggcagcggcc gagcaacagc 30540
   gcatgaatca agagctccaa gacatggtta acttgcacca gtgcaaaagg ggtatctttt 30600
   gtctggtaaa gcaggccaaa gtcacctacg acagtaatac caccggacac cgccttagct 30660
   acaagttgcc aaccaagcgt cagaaattgg tggtcatggt gggagaaaag cccattacca 30720
   taactcagca ctcggtagaa accgaaggct gcattcactc accttgtcaa ggacctgagg 30780
   atctctgcac ccttattaag accctgtgcg gtctcaaaga tcttattccc tttaactaat 30840
   aaaaaaaaat aataaagcat cacttactta aaatcagtta gcaaatttct gtccagttta 30900
   ttcagcagca cctccttgcc ctcctcccag ctctggtatt gcagcttcct cctggctgca 30960
   aactttctcc acaatctaaa tggaatgtca gtttcctcct gttcctgtcc atccgcaccc 31020
   actatcttca tgttgttgca gatgaagcgc gcaagaccgt ctgaagatac cttcaacccc 31080
   gtgtatccat atgacacgga aaccggtcct ccaactgtgc cttttcttac tcctcccttt 31140
   gtatccccca atgggtttca agagagtccc cctggggtac tctctttgcg cctatccgaa 31200
   cctctagtta cctccaatgg catgcttgcg ctcaaaatgg gcaacggcct ctctctggac 31260
   gaggccggca accttacctc ccaaaatgta accactgtga gcccacctct caaaaaaacc 31320
   aagtcaaaca taaacctgga aatatctgca cccctcacag ttacctcaga agccctaact 31380
   gtggctgccg ccgcacctct aatggtcgcg ggcaacacac tcaccatgca atcacaggcc 31440
   ccgctaaccg tgcacgactc caaacttagc attgccaccc aaggacccct cacagtgtca 31500
   gaaggaaagc tagccctgca aacatcaggc cccctcacca ccaccgatag cagtaccctt 31560
   actatcactg cctcaccccc tctaactact gccactggta gcttgggcat tgacttgaaa 31620
   gagcccattt atacacaaaa tggaaaacta ggactaaagt acggggctcc tttgcatgta 31680
   acagacgacc taaacacttt gaccgtagca actggtccag gtgtgactat taataatact 31740
   tccttgcaaa ctaaagttac tggagccttg ggttttgatt cacaaggcaa tatgcaactt 31800
   aatgtagcag gaggactaag gattgattct caaaacagac gccttatact tgatgttagt 31860
   tatccgtttg atgctcaaaa ccaactaaat ctaagactag gacagggccc tctttttata 31920
   aactcagccc acaacttgga tattaactac aacaaaggcc tttacttgtt tacagcttca 31980
   aacaattcca aaaagcttga ggttaaccta agcactgcca aggggttgat gtttgacgct 32040
   acagccatag ccattaatgc aggagatggg cttgaatttg gttcacctaa tgcaccaaac 32100
   acaaatcccc tcaaaacaaa aattggccat ggcctagaat ttgattcaaa caaggctatg 32160
   gttcctaaac taggaactgg ccttagtttt gacagcacag gtgccattac agtaggaaac 32220
   aaaaataatg ataagctaac tttgtggacc acaccagctc catctcctaa ctgtagacta 32280
   aatgcagaga aagatgctaa actcactttg gtcttaacaa aatgtggcag tcaaatactt 32340
   gctacagttt cagttttggc tgttaaaggc agtttggctc caatatctgg aacagttcaa 32400
   agtgctcatc ttattataag atttgacgaa aatggagtgc tactaaacaa ttccttcctg 32460
   gacccagaat attggaactt tagaaatgga gatcttactg aaggcacagc ctatacaaac 32520
   gctgttggat ttatgcctaa cctatcagct tatccaaaat ctcacggtaa aactgccaaa 32580
   agtaacattg tcagtcaagt ttacttaaac ggagacaaaa ctaaacctgt aacactaacc 32640
   attacactaa acggtacaca ggaaacagga gacacaactc caagtgcata ctctatgtca 32700
   ttttcatggg actggtctgg ccacaactac attaatgaaa tatttgccac atcctcttac 32760
   actttttcat acattgccca agaataaaga atcgtttgtg ttatgtttca acgtgtttat 32820
   ttttcaattg cagaaaattt caagtcattt ttcattcagt agtatagccc caccaccaca 32880
   tagcttatac agatcaccgt accttaatca aactcacaga accctagtat tcaacctgcc 32940
   acctccctcc caacacacag agtacacagt cctttctccc cggctggcct taaaaagcat 33000
   catatcatgg gtaacagaca tattcttagg tgttatattc cacacggttt cctgtcgagc 33060
   caaacgctca tcagtgatat taataaactc cccgggcagc tcacttaagt tcatgtcgct 33120
   gtccagctgc tgagccacag gctgctgtcc aacttgcggt tgcttaacgg gcggcgaagg 33180
   agaagtccac gcctacatgg gggtagagtc ataatcgtgc atcaggatag ggcggtggtg 33240
   ctgcagcagc gcgcgaataa actgctgccg ccgccgctcc gtcctgcagg aatacaacat 33300
   ggcagtggtc tcctcagcga tgattcgcac cgcccgcagc ataaggcgcc ttgtcctccg 33360
   ggcacagcag cgcaccctga tctcacttaa atcagcacag taactgcagc acagcaccac 33420
   aatattgttc aaaatcccac agtgcaaggc gctgtatcca aagctcatgg cggggaccac 33480
   agaacccacg tggccatcat accacaagcg caggtagatt aagtggcgac ccctcataaa 33540
   cacgctggac ataaacatta cctcttttgg catgttgtaa ttcaccacct cccggtacca 33600
   tataaacctc tgattaaaca tggcgccatc caccaccatc ctaaaccagc tggccaaaac 33660
   ctgcccgccg gctatacact gcagggaacc gggactggaa caatgacagt ggagagccca 33720
   ggactcgtaa ccatggatca tcatgctcgt catgatatca atgttggcac aacacaggca 33780
   cacgtgcata cacttcctca ggattacaag ctcctcccgc gttagaacca tatcccaggg 33840
   aacaacccat tcctgaatca gcgtaaatcc cacactgcag ggaagacctc gcacgtaact 33900
   cacgttgtgc attgtcaaag tgttacattc gggcagcagc ggatgatcct ccagtatggt 33960
   agcgcgggtt tctgtctcaa aaggaggtag acgatcccta ctgtacggag tgcgccgaga 34020
   caaccgagat cgtgttggtc gtagtgtcat gccaaatgga acgccggacg tagtcatatt 34080
   tcctgaagca aaaccaggtg cgggcgtgac aaacagatct gcgtctccgg tctcgccgct 34140
   tagatcgctc tgtgtagtag ttgtagtata tccactctct caaagcatcc aggcgccccc 34200
   tggcttcggg ttctatgtaa actccttcat gcgccgctgc cctgataaca tccaccaccg 34260
   cagaataagc cacacccagc caacctacac attcgttctg cgagtcacac acgggaggag 34320
   cgggaagagc tggaagaacc atgttttttt ttttattcca aaagattatc caaaacctca 34380
   aaatgaagat ctattaagtg aacgcgctcc cctccggtgg cgtggtcaaa ctctacagcc 34440
   aaagaacaga taatggcatt tgtaagatgt tgcacaatgg cttccaaaag gcaaacggcc 34500
   ctcacgtcca agtggacgta aaggctaaac ccttcagggt gaatctcctc tataaacatt 34560
   ccagcacctt caaccatgcc caaataattc tcatctcgcc accttctcaa tatatctcta 34620
   agcaaatccc gaatattaag tccggccatt gtaaaaatct gctccagagc gccctccacc 34680
   ttcagcctca agcagcgaat catgattgca aaaattcagg ttcctcacag acctgtataa 34740
   gattcaaaag cggaacatta acaaaaatac cgcgatcccg taggtccctt cgcagggcca 34800
   gctgaacata atcgtgcagg tctgcacgga ccagcgcggc cacttccccg ccaggaacca 34860
   tgacaaaaga acccacactg attatgacac gcatactcgg agctatgcta accagcgtag 34920
   ccccgatgta agcttgttgc atgggcggcg atataaaatg caaggtgctg ctcaaaaaat 34980
   caggcaaagc ctcgcgcaaa aaagaaagca catcgtagtc atgctcatgc agataaaggc 35040
   aggtaagctc cggaaccacc acagaaaaag acaccatttt tctctcaaac atgtctgcgg 35100
   gtttctgcat aaacacaaaa taaaataaca aaaaaacatt taaacattag aagcctgtct 35160
   tacaacagga aaaacaaccc ttataagcat aagacggact acggccatgc cggcgtgacc 35220
   gtaaaaaaac tggtcaccgt gattaaaaag caccaccgac agctcctcgg tcatgtccgg 35280
   agtcataatg taagactcgg taaacacatc aggttgattc acatcggtca gtgctaaaaa 35340
   gcgaccgaaa tagcccgggg gaatacatac ccgcaggcgt agagacaaca ttacagcccc 35400
   cataggaggt ataacaaaat taataggaga gaaaaacaca taaacacctg aaaaaccctc 35460
   ctgcctaggc aaaatagcac cctcccgctc cagaacaaca tacagcgctt ccacagcggc 35520
   agccataaca gtcagcctta ccagtaaaaa agaaaaccta ttaaaaaaac accactcgac 35580
   acggcaccag ctcaatcagt cacagtgtaa aaaagggcca agtgcagagc gagtatatat 35640
   aggactaaaa aatgacgtaa cggttaaagt ccacaaaaaa cacccagaaa accgcacgcg 35700
   aacctacgcc cagaaacgaa agccaaaaaa cccacaactt cctcaaatcg tcacttccgt 35760
   tttcccacgt tacgtaactt cccattttaa gaaaactaca attcccaaca catacaagtt 35820
   actccgccct aaaacctacg tcacccgccc cgttcccacg ccccgcgcca cgtcacaaac 35880
   tccaccccct cattatcata ttggcttcaa tccaaaataa ggtatattat tgatgatg 35938
<210> 3
   <211> 35937
   <212> DNA
   <213> adenovirus type 2
<400> 3
<210> 4
   <211> 8342
   <212> DNA
   <213> Artificial Sequence
<220>
   <223> Helper gene region
<400> 4
   ccgtgcaaaa ggagagcctg taagcgggca ctcttccgtg gtctggtgga taaattcgca      60
   agggtatcat ggcggacgac cggggttcga gccccgtatc cggccgtccg ccgtgatcca     120
   tgcggttacc gcccgcgtgt cgaacccagg tgtgcgacgt cagacaacgg gggagtgctc     180
   cttttggctt ccttccaggc gcggcggctg ctgcgctagc ttttttggcc actggccgcg     240
   cgcagcgtaa gcggttaggc tggaaagcga aagcattaag tggctcgctc cctgtagccg     300
   gagggttatt ttccaagggt tgagtcgcgg gacccccggt tcgagtctcg gaccggccgg     360
   actgcggcga acgggggttt gcctccccgt catgcaagac cccgcttgca aattcctccg     420
   gaaacaggga cgagcccctt ttttgctttt aaaggcaaat gcttttattt gtacactctc     480
   gggtgattat ttacccccac ccttgccgtc tgcgccgttt aaaaatcaaa ggggttctgc     540
   cgcgcatcgc tatgcgccac tggcagggac acgttgcgat actggtgttt agtgctccac     600
   ttaaactcag gcacaaccat ccgcggcagc tcggtgaagt tttcactcca caggctgcgc     660
   accatcacca acgcgtttag caggtcgggc gccgatatct tgaagtcgca gttggggcct     720
   ccgccctgcg cgcgcgagtt gcgatacaca gggttgcagc actggaacac tatcagcgcc     780
   gggtggtgca cgctggccag cacgctcttg tcggagatca gatccgcgtc caggtcctcc     840
   gcgttgctca gggcgaacgg agtcaacttt ggtagctgcc ttcccaaaaa gggcgcgtgc     900
   ccaggctttg agttgcactc gcaccgtagt ggcatcaaaa ggtgaccgtg cccggtctgg     960
   gcgttaggat acagcgcctg cataaaagcc ttgatctgct taaaagccac ctgagccttt    1020
   gcgccttcag agaagaacat gccgcaagac ttgccggaaa actgattggc cggacaggcc    1080
   gcgtcgtgca cgcagcacct tgcgtcggtg ttggagatct gcaccacatt tcggccccac    1140
   cggttcttca cgatcttggc cttgctagac tgctccttca gcgcgcgctg cccgttttcg    1200
   ctcgtcacat ccatttcaat cacgtgctcc ttatttatca taatgcttcc gtgtagacac    1260
   ttaagctcgc cttcgatctc agcgcagcgg tgcagccaca acgcgcagcc cgtgggctcg    1320
   tgatgcttgt aggtcacctc tgcaaacgac tgcaggtacg cctgcaggaa tcgccccatc    1380
   atcgtcacaa aggtcttgtt gctggtgaag gtcagctgca acccgcggtg ctcctcgttc    1440
   agccaggtct tgcatacggc cgccagagct tccacttggt caggcagtag tttgaagttc    1500
   gcctttagat cgttatccac gtggtacttg tccatcagcg cgcgcgcagc ctccatgccc    1560
   ttctcccacg cagacacgat cggcacactc agcgggttca tcaccgtaat ttcactttcc 1620
   gcttcgctgg gctcttcctc ttcctcttgc gtccgcatac cacgcgccac tgggtcgtct 1680
   tcattcagcc gccgcactgt gcgcttacct cctttgccat gcttgattag caccggtggg 1740
   ttgctgaaac ccaccatttg tagcgccaca tcttctcttt cttcctcgct gtccacgatt 1800
   acctctggtg atggcgggcg ctcgggcttg ggagaagggc gcttcttttt cttcttgggc 1860
   gcaatggcca aatccgccgc cgaggtcgat ggccgcgggc tgggtgtgcg cggcaccagc 1920
   gcgtcttgtg atgagtcttc ctcgtcctcg gactcgatac gccgcctcat ccgctttttt 1980
   gggggcgccc ggggaggcgg cggcgacggg gacggggacg acacgtcctc catggttggg 2040
   ggacgtcgcg ccgcaccgcg tccgcgctcg ggggtggttt cgcgctgctc ctcttcccga 2100
   ctggccattt ccttctccta taggcagaaa aagatcatgt cgacaacgtt ggagtcagtc 2160
   gagaagaagg acagcctaac cgccccctct gagttcgcca ccaccgcctc caccgatgcc 2220
   gccaacgcgc ctaccacctt ccccgtcgag gcacccccgc ttgaggagga ggaagtgatt 2280
   atcgagcagg acccaggttt tgtaagcgaa gacgacgagg accgctcagt accaacagag 2340
   gataaaaagc aagaccagga caacgcagag gcaaacgagg aacaagtcgg gcggggggac 2400
   gaaaggcatg gcgactacct agatgtggga gacgacgtgc tgttgaagca tctgcagcgc 2460
   cagtgcgcca ttatctgcga cgcgttgcaa gagcgcagcg atgtgcccct cgccatagcg 2520
   gatgtcagcc ttgcctacga acgccaccta ttctcaccgc gcgtaccccc caaacgccaa 2580
   gaaaacggca catgcgagcc caacccgcgc ctcaacttct accccgtatt tgccgtgcca 2640
   gaggtgcttg ccacctatca catctttttc caaaactgca agatacccct atcctgccgt 2700
   gccaaccgca gccgagcgga caagcagctg gccttgcggc agggcgctgt catacctgat 2760
   atcgcctcgc tcaacgaagt gccaaaaatc tttgagggtc ttggacgcga cgagaagcgc 2820
   gcggcaaacg ctctgcaaca ggaaaacagc gaaaatgaaa gtcactctgg agtgttggtg 2880
   gaactcgagg gtgacaacgc gcgcctagcc gtactaaaac gcagcatcga ggtcacccac 2940
   tttgcctacc cggcacttaa cctacccccc aaggtcatga gcacagtcat gagtgagctg 3000
   atcgtgcgcc gtgcgcagcc cctggagagg gatgcaaatt tgcaagaaca aacagaggag 3060
   ggcctacccg cagttggcga cgagcagcta gcgcgctggc ttcaaacgcg cgagcctgcc 3120
   gacttggagg agcgacgcaa actaatgatg gccgcagtgc tcgttaccgt ggagcttgag 3180
   tgcatgcagc ggttctttgc tgacccggag atgcagcgca agctagagga aacattgcac 3240
   tacacctttc gacagggcta cgtacgccag gcctgcaaga tctccaacgt ggagctctgc 3300
   aacctggtct cctaccttgg aattttgcac gaaaaccgcc ttgggcaaaa cgtgcttcat 3360
   tccacgctca agggcgaggc gcgccgcgac tacgtccgcg actgcgttta cttatttcta 3420
   tgctacacct ggcagacggc catgggcgtt tggcagcagt gcttggagga gtgcaacctc 3480
   aaggagctgc agaaactgct aaagcaaaac ttgaaggacc tatggacggc cttcaacgag 3540
   cgctccgtgg ccgcgcacct ggcggacatc attttccccg aacgcctgct taaaaccctg 3600
   caacagggtc tgccagactt caccagtcaa agcatgttgc agaactttag gaactttatc 3660
   ctagagcgct caggaatctt gcccgccacc tgctgtgcac ttcctagcga ctttgtgccc 3720
   attaagtacc gcgaatgccc tccgccgctt tggggccact gctaccttct gcagctagcc 3780
   aactaccttg cctaccactc tgacataatg gaagacgtga gcggtgacgg tctactggag 3840
   tgtcactgtc gctgcaacct atgcaccccg caccgctccc tggtttgcaa ttcgcagctg 3900
   cttaacgaaa gtcaaattat cggtaccttt gagctgcagg gtccctcgcc tgacgaaaag 3960
   tccgcggctc cggggttgaa actcactccg gggctgtgga cgtcggctta ccttcgcaaa 4020
   tttgtacctg aggactacca cgcccacgag attaggttct acgaagacca atcccgcccg 4080
   cctaatgcgg agcttaccgc ctgcgtcatt acccagggcc acattcttgg ccaattgcaa 4140
   gccatcaaca aagcccgcca agagtttctg ctacgaaagg gacggggggt ttacttggac 4200
   ccccagtccg gcgaggagct caacccaatc cccccgccgc cgcagcccta tcagcagcag 4260
   ccgcgggccc ttgcttccca ggatggcacc caaaaagaag ctgcagctgc cgccgccacc 4320
   cacggacgag gaggaatact gggacagtca ggcagaggag gttttggacg aggaggagga 4380
   ggacatgatg gaagactggg agagcctaga cgaggaagct tccgaggtcg aagaggtgtc 4440
   agacgaaaca ccgtcaccct cggtcgcatt cccctcgccg gcgccccaga aatcggcaac 4500
   cggttccagc atggctacaa cctccgctcc tcaggcgccg ccggcactgc ccgttcgccg 4560
   acccaaccgt agatgggaca ccactggaac cagggccggt aagtccaagc agccgccgcc 4620
   gttagcccaa gagcaacaac agcgccaagg ctaccgctca tggcgcgggc acaagaacgc 4680
   catagttgct tgcttgcaag actgtggggg caacatctcc ttcgcccgcc gctttcttct 4740
   ctaccatcac ggcgtggcct tcccccgtaa catcctgcat tactaccgtc atctctacag 4800
   cccatactgc accggcggca gcggcagcaa cagcagcggc cacacagaag caaaggcgac 4860
   cggatagcaa gactctgaca aagcccaaga aatccacagc ggcggcagca gcaggaggag 4920
   gagcgctgcg tctggcgccc aacgaacccg tatcgacccg cgagcttaga aacaggattt 4980
   ttcccactct gtatgctata tttcaacaga gcaggggcca agaacaagag ctgaaaataa 5040
   aaaacaggtc tctgcgatcc ctcacccgca gctgcctgta tcacaaagtc gacagcgaag 5100
   atcagcttcg gcgcacgctg gaagacgcgg aggctctctt cagtaaatac tgcgcgctga 5160
   ctcttaagga ctagtttcgc gccctttctc aaatttaagc gcgaaaacta cgtcatctcc 5220
   agcgttcgaa cttacacttt ttcatacatt gcccaagaat aaagaatcgt ttgtgttatg 5280
   tttcaacgtg tttatttttc aattgcagaa aatttcaagt catttttcat tcagtagtat 5340
   agccccacca ccacatagct tatacagatc accgtacctt aatcaaactc acagaaccct 5400
   agtattcaac ctgccacctc cctcccaaca cacagagtac acagtccttt ctccccggct 5460
   ggccttaaaa agcatcatat catgggtaac agacatattc ttaggtgtta tattccacac 5520
   ggtttcctgt cgagccaaac gctcatcagt gatattaata aactccccgg gcagctcact 5580
   taagttcatg tcgctgtcca gctgctgagc cacaggctgc tgtccaactt gcggttgctt 5640
   aacgggcggc gaaggagaag tccacgccta catgggggta gagtcataat cgtgcatcag 5700
   gatagggcgg tggtgctgca gcagcgcgcg aataaactgc tgccgccgcc gctccgtcct 5760
   gcaggaatac aacatggcag tggtctcctc agcgatgatt cgcaccgccc gcagcataag 5820
   gcgccttgtc ctccgggcac agcagcgcac cctgatctca cttaaatcag cacagtaact 5880
   gcagcacagc accacaatat tgttcaaaat cccacagtgc aaggcgctgt atccaaagct 5940
   catggcgggg accacagaac ccacgtggcc atcataccac aagcgcaggt agattaagtg 6000
   gcgacccctc ataaacacgc tggacataaa cattacctct tttggcatgt tgtaattcac 6060
   cacctcccgg taccatataa acctctgatt aaacatggcg ccatccacca ccatcctaaa 6120
   ccagctggcc aaaacctgcc cgccggctat acactgcagg gaaccgggac tggaacaatg 6180
   acagtggaga gcccaggact cgtaaccatg gatcatcatg ctcgtcatga tatcaatgtt 6240
   ggcacaacac aggcacacgt gcatacactt cctcaggatt acaagctcct cccgcgttag 6300
   aaccatatcc cagggaacaa cccattcctg aatcagcgta aatcccacac tgcagggaag 6360
   acctcgcacg taactcacgt tgtgcattgt caaagtgtta cattcgggca gcagcggatg 6420
   atcctccagt atggtagcgc gggtttctgt ctcaaaagga ggtagacgat ccctactgta 6480
   cggagtgcgc cgagacaacc gagatcgtgt tggtcgtagt gtcatgccaa atggaacgcc 6540
   ggacgtagtc atatttcctg aagcaaaacc aggtgcgggc gtggatccga caaacagatc 6600
   tgcgtctccg gtctcgccgc ttagatcgct ctgtgtagta gttgtagtat atccactctc 6660
   tcaaagcatc caggcgcccc ctggcttcgg gttctatgta aactccttca tgcgccgctg 6720
   ccctgataac atccaccacc gcagaataag ccacacccag ccaacctaca cattcgttct 6780
   gcgagtcaca cacgggagga gcgggaagag ctggaagaac catgtttttt ttttttattc 6840
   caaaagatta tccaaaacct caaaatgaag atctattaag tgaacgcgct cccctccggt 6900
   ggcgtggtca aactctacag ccaaagaaca gataatggca tttgtaagat gttgcacaat 6960
   ggcttccaaa aggcaaacgg ccctcacgtc caagtggacg taaaggctaa acccttcagg 7020
   gtgaatctcc tctataaaca ttccagcacc ttcaaccatg cccaaataat tctcatctcg 7080
   ccaccttctc aatatatctc taagcaaatc ccgaatatta agtccggcca ttgtaaaaat 7140
   ctgctccaga gcgccctcca ccttcagcct caagcagcga atcatgattg caaaaattca 7200
   ggttcctcac agacctgtat aagattcaaa agcggaacat taacaaaaat accgcgatcc 7260
   cgtaggtccc ttcgcagggc cagctgaaca taatcgtgca ggtctgcacg gaccagcgcg 7320
   gccacttccc cgccaggaac catgacaaaa gaacccacac tgattatgac acgcatactc 7380
   ggagctatgc taaccagcgt agccccgatg taagcttgtt gcatgggcgg cgatataaaa 7440
   tgcaaggtgc tgctcaaaaa atcaggcaaa gcctcgcgca aaaaagaaag cacatcgtag 7500
   tcatgctcat gcagataaag gcaggtaagc tccggaacca ccacagaaaa agacaccatt 7560
   tttctctcaa acatgtctgc gggtttctgc ataaacacaa aataaaataa caaaaaaaca 7620
   tttaaacatt agaagcctgt cttacaacag gaaaaacaac ccttataagc ataagacgga 7680
   ctacggccat gctggcgtga ccgtaaaaaa actggtcacc gtgattaaaa agcaccaccg 7740
   acagctcctc ggtcatgtcc ggagtcataa tgtaagactc ggtaaacaca tcaggttgat 7800
   tcacatcggt cagtgctaaa aagcgaccga aatagcccgg gggaatacat acccgcaggc 7860
   gtagagacaa cattacagcc cccataggag gtataacaaa attaatagga gagaaaaaca 7920
   cataaacacc tgaaaaaccc tcctgcctag gcaaaatagc accctcccgc tccagaacaa 7980
   catacagcgc ttccacagcg gcagccataa cagtcagcct taccagtaaa aaagaaaacc 8040
   tattaaaaaa acaccactcg acacggggat cccaccagct caatcagtca cagtgtaaaa 8100
   aagggccaag tgcagagcga gtatatatag gactaaaaaa tgacgtaacg gttaaagtcc 8160
   acaaaaaaca cccagaaaac cgcacgcgaa cctacgccca gaaacgaaag ccaaaaaacc 8220
   cacaacttcc tcaaatcgtc acttccgttt tcccacgtta cgtcacttcc cattttaaga 8280
   ttaattaaac tacaattccc aacacataca agttactccg ccctaaaacc tacgtcaccc 8340
   gc 8342
<210> 5
   <211> 35934
   <212> DNA
   <213> adenovirus type 5
<400> 5
   catcatcaat aatatacctt attttggatt gaagccaata tgataatgag ggggtggagt     60
   ttgtgacgtg gcgcggggcg tgggaacggg gcgggtgacg tagtagtgtg gcggaagtgt    120
   gatgttgcaa gtgtggcgga acacatgtaa gcgacggatg tggcaaaagt gacgtttttg    180
   gtgtgcgccg gtgtacacag gaagtgacaa ttttcgcgcg gttttaggcg gatgttgtag    240
   taaatttggg cgtaaccgag taagatttgg ccattttcgc gggaaaactg aataagagga    300
   agtgaaatct gaataatttt gtgttactca tagcgcgtaa tatttgtcta gggccgcggg    360
   gactttgacc gtttacgtgg agactcgccc aggtgttttt ctcaggtgtt ttccgcgttc    420
   cgggtcaaag ttggcgtttt attattatag tcagctgacg tgtagtgtat ttatacccgg    480
   tgagttcctc aagaggccac tcttgagtgc cagcgagtag agttttctcc tccgagccgc    540
   tccgacaccg ggactgaaaa tgagacatat tatctgccac ggaggtgtta ttaccgaaga 600
   aatggccgcc agtcttttgg accagctgat cgaagaggta ctggctgata atcttccacc 660
   tcctagccat tttgaaccac ctacccttca cgaactgtat gatttagacg tgacggcccc 720
   cgaagatccc aacgaggagg cggtttcgca gatttttccc gactctgtaa tgttggcggt 780
   gcaggaaggg attgacttac tcacttttcc gccggcgccc ggttctccgg agccgcctca 840
   cctttcccgg cagcccgagc agccggagca gagagccttg ggtccggttt ctatgccaaa 900
   ccttgtaccg gaggtgatcg atcttacctg ccacgaggct ggctttccac ccagtgacga 960
   cgaggatgaa gagggtgagg agtttgtgtt agattatgtg gagcaccccg ggcacggttg 1020
   caggtcttgt cattatcacc ggaggaatac gggggaccca gatattatgt gttcgctttg 1080
   ctatatgagg acctgtggca tgtttgtcta cagtaagtga aaattatggg cagtgggtga 1140
   tagagtggtg ggtttggtgt ggtaattttt tttttaattt ttacagtttt gtggtttaaa 1200
   gaattttgta ttgtgatttt tttaaaaggt cctgtgtctg aacctgagcc tgagcccgag 1260
   ccagaaccgg agcctgcaag acctacccgc cgtcctaaaa tggcgcctgc tatcctgaga 1320
   cgcccgacat cacctgtgtc tagagaatgc aatagtagta cggatagctg tgactccggt 1380
   ccttctaaca cacctcctga gatacacccg gtggtcccgc tgtgccccat taaaccagtt 1440
   gccgtgagag ttggtgggcg tcgccaggct gtggaatgta tcgaggactt gcttaacgag 1500
   cctgggcaac ctttggactt gagctgtaaa cgccccaggc cataaggtgt aaacctgtga 1560
   ttgcgtgtgt ggttaacgcc tttgtttgct gaatgagttg atgtaagttt aataaagggt 1620
   gagataatgt ttaacttgca tggcgtgtta aatggggcgg ggcttaaagg gtatataatg 1680
   cgccgtgggc taatcttggt tacatctgac ctcatggagg cttgggagtg tttggaagat 1740
   ttttctgctg tgcgtaactt gctggaacag agctctaaca gtacctcttg gttttggagg 1800
   tttctgtggg gctcatccca ggcaaagtta gtctgcagaa ttaaggagga ttacaagtgg 1860
   gaatttgaag agcttttgaa atcctgtggt gagctgtttg attctttgaa tctgggtcac 1920
   caggcgcttt tccaagagaa ggtcatcaag actttggatt tttccacacc ggggcgcgct 1980
   gcggctgctg ttgctttttt gagttttata aaggataaat ggagcgaaga aacccatctg 2040
   agcggggggt acctgctgga ttttctggcc atgcatctgt ggagagcggt tgtgagacac 2100
   aagaatcgcc tgctactgtt gtcttccgtc cgcccggcga taataccgac ggaggagcag 2160
   cagcagcagc aggaggaagc caggcggcgg cggcaggagc agagcccatg gaacccgaga 2220
   gccggcctgg accctcggga atgaatgttg tacaggtggc tgaactgtat ccagaactga 2280
   gacgcatttt gacaattaca gaggatgggc aggggctaaa gggggtaaag agggagcggg 2340
   gggcttgtga ggctacagag gaggctagga atctagcttt tagcttaatg accagacacc 2400
   gtcctgagtg tattactttt caacagatca aggataattg cgctaatgag cttgatctgc 2460
   tggcgcagaa gtattccata gagcagctga ccacttactg gctgcagcca ggggatgatt 2520
   ttgaggaggc tattagggta tatgcaaagg tggcacttag gccagattgc aagtacaaga 2580
   tcagcaaact tgtaaatatc aggaattgtt gctacatttc tgggaacggg gccgaggtgg 2640
   agatagatac ggaggatagg gtggccttta gatgtagcat gataaatatg tggccggggg 2700
   tgcttggcat ggacggggtg gttattatga atgtaaggtt tactggcccc aattttagcg 2760
   gtacggtttt cctggccaat accaacctta tcctacacgg tgtaagcttc tatgggttta 2820
   acaatacctg tgtggaagcc tggaccgatg taagggttcg gggctgtgcc ttttactgct 2880
   gctggaaggg ggtggtgtgt cgccccaaaa gcagggcttc aattaagaaa tgcctctttg 2940
   aaaggtgtac cttgggtatc ctgtctgagg gtaactccag ggtgcgccac aatgtggcct 3000
   ccgactgtgg ttgcttcatg ctagtgaaaa gcgtggctgt gattaagcat aacatggtat 3060
   gtggcaactg cgaggacagg gcctctcaga tgctgacctg ctcggacggc aactgtcacc 3120
   tgctgaagac cattcacgta gccagccact ctcgcaaggc ctggccagtg tttgagcata 3180
   acatactgac ccgctgttcc ttgcatttgg gtaacaggag gggggtgttc ctaccttacc 3240
   aatgcaattt gagtcacact aagatattgc ttgagcccga gagcatgtcc aaggtgaacc 3300
   tgaacggggt gtttgacatg accatgaaga tctggaaggt gctgaggtac gatgagaccc 3360
   gcaccaggtg cagaccctgc gagtgtggcg gtaaacatat taggaaccag cctgtgatgc 3420
   tggatgtgac cgaggagctg aggcccgatc acttggtgct ggcctgcacc cgcgctgagt 3480
   ttggctctag cgatgaagat acagattgag gtactgaaat gtgtgggcgt ggcttaaggg 3540
   tgggaaagaa tatataaggt gggggtctta tgtagttttg tatctgtttt gcagcagccg 3600
   ccgccgccat gagcaccaac tcgtttgatg gaagcattgt gagctcatat ttgacaacgc 3660
   gcatgccccc atgggccggg gtgcgtcaga atgtgatggg ctccagcatt gatggtcgcc 3720
   ccgtcctgcc cgcaaactct actaccttga cctacgagac cgtgtctgga acgccgttgg 3780
   agactgcagc ctccgccgcc gcttcagccg ctgcagccac cgcccgcggg attgtgactg 3840
   actttgcttt cctgagcccg cttgcaagca gtgcagcttc ccgttcatcc gcccgcgatg 3900
   acaagttgac ggctcttttg gcacaattgg attctttgac ccgggaactt aatgtcgttt 3960
   ctcagcagct gttggatctg cgccagcagg tttctgccct gaaggcttcc tcccctccca 4020
   atgcggttta aaacataaat aaaaaaccag actctgtttg gatttggatc aagcaagtgt 4080
   cttgctgtct ttatttaggg gttttgcgcg cgcggtaggc ccgggaccag cggtctcggt 4140
   cgttgagggt cctgtgtatt ttttccagga cgtggtaaag gtgactctgg atgttcagat 4200
   acatgggcat aagcccgtct ctggggtgga ggtagcacca ctgcagagct tcatgctgcg 4260
   gggtggtgtt gtagatgatc cagtcgtagc aggagcgctg ggcgtggtgc ctaaaaatgt 4320
   ctttcagtag caagctgatt gccaggggca ggcccttggt gtaagtgttt acaaagcggt 4380
   taagctggga tgggtgcata cgtggggata tgagatgcat cttggactgt atttttaggt 4440
   tggctatgtt cccagccata tccctccggg gattcatgtt gtgcagaacc accagcacag 4500
   tgtatccggt gcacttggga aatttgtcat gtagcttaga aggaaatgcg tggaagaact 4560
   tggagacgcc cttgtgacct ccaagatttt ccatgcattc gtccataatg atggcaatgg 4620
   gcccacgggc ggcggcctgg gcgaagatat ttctgggatc actaacgtca tagttgtgtt 4680
   ccaggatgag atcgtcatag gccattttta caaagcgcgg gcggagggtg ccagactgcg 4740
   gtataatggt tccatccggc ccaggggcgt agttaccctc acagatttgc atttcccacg 4800
   ctttgagttc agatgggggg atcatgtcta cctgcggggc gatgaagaaa acggtttccg 4860
   gggtagggga gatcagctgg gaagaaagca ggttcctgag cagctgcgac ttaccgcagc 4920
   cggtgggccc gtaaatcaca cctattaccg gctgcaactg gtagttaaga gagctgcagc 4980
   tgccgtcatc cctgagcagg ggggccactt cgttaagcat gtccctgact cgcatgtttt 5040
   ccctgaccaa atccgccaga aggcgctcgc cgcccagcga tagcagttct tgcaaggaag 5100
   caaagttttt caacggtttg agaccgtccg ccgtaggcat gcttttgagc gtttgaccaa 5160
   gcagttccag gcggtcccac agctcggtca cctgctctac ggcatctcga tccagcatat 5220
   ctcctcgttt cgcgggttgg ggcggctttc gctgtacggc agtagtcggt gctcgtccag 5280
   acgggccagg gtcatgtctt tccacgggcg cagggtcctc gtcagcgtag tctgggtcac 5340
   ggtgaagggg tgcgctccgg gctgcgcgct ggccagggtg cgcttgaggc tggtcctgct 5400
   ggtgctgaag cgctgccggt cttcgccctg cgcgtcggcc aggtagcatt tgaccatggt 5460
   gtcatagtcc agcccctccg cggcgtggcc cttggcgcgc agcttgccct tggaggaggc 5520
   gccgcacgag gggcagtgca gacttttgag ggcgtagagc ttgggcgcga gaaataccga 5580
   ttccggggag taggcatccg cgccgcaggc cccgcagacg gtctcgcatt ccacgagcca 5640
   ggtgagctct ggccgttcgg ggtcaaaaac caggtttccc ccatgctttt tgatgcgttt 5700
   cttacctctg gtttccatga gccggtgtcc acgctcggtg acgaaaaggc tgtccgtgtc 5760
   cccgtataca gacttgagag gcctgtcctc gagcggtgtt ccgcggtcct cctcgtatag 5820
   aaactcggac cactctgaga caaaggctcg cgtccaggcc agcacgaagg aggctaagtg 5880
   ggaggggtag cggtcgttgt ccactagggg gtccactcgc tccagggtgt gaagacacat 5940
   gtcgccctct tcggcatcaa ggaaggtgat tggtttgtag gtgtaggcca cgtgaccggg 6000
   tgttcctgaa ggggggctat aaaagggggt gggggcgcgt tcgtcctcac tctcttccgc 6060
   atcgctgtct gcgagggcca gctgttgggg tgagtactcc ctctgaaaag cgggcatgac 6120
   ttctgcgcta agattgtcag tttccaaaaa cgaggaggat ttgatattca cctggcccgc 6180
   ggtgatgcct ttgagggtgg ccgcatccat ctggtcagaa aagacaatct ttttgttgtc 6240
   aagcttggtg gcaaacgacc cgtagagggc gttggacagc aacttggcga tggagcgcag 6300
   ggtttggttt ttgtcgcgat cggcgcgctc cttggccgcg atgtttagct gcacgtattc 6360
   gcgcgcaacg caccgccatt cgggaaagac ggtggtgcgc tcgtcgggca ccaggtgcac 6420
   gcgccaaccg cggttgtgca gggtgacaag gtcaacgctg gtggctacct ctccgcgtag 6480
   gcgctcgttg gtccagcaga ggcggccgcc cttgcgcgag cagaatggcg gtagggggtc 6540
   tagctgcgtc tcgtccgggg ggtctgcgtc cacggtaaag accccgggca gcaggcgcgc 6600
   gtcgaagtag tctatcttgc atccttgcaa gtctagcgcc tgctgccatg cgcgggcggc 6660
   aagcgcgcgc tcgtatgggt tgagtggggg accccatggc atggggtggg tgagcgcgga 6720
   ggcgtacatg ccgcaaatgt cgtaaacgta gaggggctct ctgagtattc caagatatgt 6780
   agggtagcat cttccaccgc ggatgctggc gcgcacgtaa tcgtatagtt cgtgcgaggg 6840
   agcgaggagg tcgggaccga ggttgctacg ggcgggctgc tctgctcgga agactatctg 6900
   cctgaagatg gcatgtgagt tggatgatat ggttggacgc tggaagacgt tgaagctggc 6960
   gtctgtgaga cctaccgcgt cacgcacgaa ggaggcgtag gagtcgcgca gcttgttgac 7020
   cagctcggcg gtgacctgca cgtctagggc gcagtagtcc agggtttcct tgatgatgtc 7080
   atacttatcc tgtccctttt ttttccacag ctcgcggttg aggacaaact cttcgcggtc 7140
   tttccagtac tcttggatcg gaaacccgtc ggcctccgaa cggtaagagc ctagcatgta 7200
   gaactggttg acggcctggt aggcgcagca tcccttttct acgggtagcg cgtatgcctg 7260
   cgcggccttc cggagcgagg tgtgggtgag cgcaaaggtg tccctgacca tgactttgag 7320
   gtactggtat ttgaagtcag tgtcgtcgca tccgccctgc tcccagagca aaaagtccgt 7380
   gcgctttttg gaacgcggat ttggcagggc gaaggtgaca tcgttgaaga gtatctttcc 7440
   cgcgcgaggc ataaagttgc gtgtgatgcg gaagggtccc ggcacctcgg aacggttgtt 7500
   aattacctgg gcggcgagca cgatctcgtc aaagccgttg atgttgtggc ccacaatgta 7560
   aagttccaag aagcgcggga tgcccttgat ggaaggcaat tttttaagtt cctcgtaggt 7620
   gagctcttca ggggagctga gcccgtgctc tgaaagggcc cagtctgcaa gatgagggtt 7680
   ggaagcgacg aatgagctcc acaggtcacg ggccattagc atttgcaggt ggtcgcgaaa 7740
   ggtcctaaac tggcgaccta tggccatttt ttctggggtg atgcagtaga aggtaagcgg 7800
   gtcttgttcc cagcggtccc atccaaggtt cgcggctagg tctcgcgcgg cagtcactag 7860
   aggctcatct ccgccgaact tcatgaccag catgaagggc acgagctgct tcccaaaggc 7920
   ccccatccaa gtataggtct ctacatcgta ggtgacaaag agacgctcgg tgcgaggatg 7980
   cgagccgatc gggaagaact ggatctcccg ccaccaattg gaggagtggc tattgatgtg 8040
   gtgaaagtag aagtccctgc gacgggccga acactcgtgc tggcttttgt aaaaacgtgc 8100
   gcagtactgg cagcggtgca cgggctgtac atcctgcacg aggttgacct gacgaccgcg 8160
   cacaaggaag cagagtggga atttgagccc ctcgcctggc gggtttggct ggtggtcttc 8220
   tacttcggct gcttgtcctt gaccgtctgg ctgctcgagg ggagttacgg tggatcggac 8280
   caccacgccg cgcgagccca aagtccagat gtccgcgcgc ggcggtcgga gcttgatgac 8340
   aacatcgcgc agatgggagc tgtccatggt ctggagctcc cgcggcgtca ggtcaggcgg 8400
   gagctcctgc aggtttacct cgcatagacg ggtcagggcg cgggctagat ccaggtgata 8460
   cctaatttcc aggggctggt tggtggcggc gtcgatggct tgcaagaggc cgcatccccg 8520
   cggcgcgact acggtaccgc gcggcgggcg gtgggccgcg ggggtgtcct tggatgatgc 8580
   atctaaaagc ggtgacgcgg gcgagccccc ggaggtaggg ggggctccgg acccgccggg 8640
   agagggggca ggggcacgtc ggcgccgcgc gcgggcagga gctggtgctg cgcgcgtagg 8700
   ttgctggcga acgcgacgac gcggcggttg atctcctgaa tctggcgcct ctgcgtgaag 8760
   acgacgggcc cggtgagctt gaacctgaaa gagagttcga cagaatcaat ttcggtgtcg 8820
   ttgacggcgg cctggcgcaa aatctcctgc acgtctcctg agttgtcttg ataggcgatc 8880
   tcggccatga actgctcgat ctcttcctcc tggagatctc cgcgtccggc tcgctccacg 8940
   gtggcggcga ggtcgttgga aatgcgggcc atgagctgcg agaaggcgtt gaggcctccc 9000
   tcgttccaga cgcggctgta gaccacgccc ccttcggcat cgcgggcgcg catgaccacc 9060
   tgcgcgagat tgagctccac gtgccgggcg aagacggcgt agtttcgcag gcgctgaaag 9120
   aggtagttga gggtggtggc ggtgtgttct gccacgaaga agtacataac ccagcgtcgc 9180
   aacgtggatt cgttgatatc ccccaaggcc tcaaggcgct ccatggcctc gtagaagtcc 9240
   acggcgaagt tgaaaaactg ggagttgcgc gccgacacgg ttaactcctc ctccagaaga 9300
   cggatgagct cggcgacagt gtcgcgcacc tcgcgctcaa aggctacagg ggcctcttct 9360
   tcttcttcaa tctcctcttc cataagggcc tccccttctt cttcttctgg cggcggtggg 9420
   ggagggggga cacggcggcg acgacggcgc accgggaggc ggtcgacaaa gcgctcgatc 9480
   atctccccgc ggcgacggcg catggtctcg gtgacggcgc ggccgttctc gcgggggcgc 9540
   agttggaaga cgccgcccgt catgtcccgg ttatgggttg gcggggggct gccatgcggc 9600
   agggatacgg cgctaacgat gcatctcaac aattgttgtg taggtactcc gccgccgagg 9660
   gacctgagcg agtccgcatc gaccggatcg gaaaacctct cgagaaaggc gtctaaccag 9720
   tcacagtcgc aaggtaggct gagcaccgtg gcgggcggca gcgggcggcg gtcggggttg 9780
   tttctggcgg aggtgctgct gatgatgtaa ttaaagtagg cggtcttgag acggcggatg 9840
   gtcgacagaa gcaccatgtc cttgggtccg gcctgctgaa tgcgcaggcg gtcggccatg 9900
   ccccaggctt cgttttgaca tcggcgcagg tctttgtagt agtcttgcat gagcctttct 9960
   accggcactt cttcttctcc ttcctcttgt cctgcatctc ttgcatctat cgctgcggcg 10020
   gcggcggagt ttggccgtag gtggcgccct cttcctccca tgcgtgtgac cccgaagccc 10080
   ctcatcggct gaagcagggc taggtcggcg acaacgcgct cggctaatat ggcctgctgc 10140
   acctgcgtga gggtagactg gaagtcatcc atgtccacaa agcggtggta tgcgcccgtg 10200
   ttgatggtgt aagtgcagtt ggccataacg gaccagttaa cggtctggtg acccggctgc 10260
   gagagctcgg tgtacctgag acgcgagtaa gccctcgagt caaatacgta gtcgttgcaa 10320
   gtccgcacca ggtactggta tcccaccaaa aagtgcggcg gcggctggcg gtagaggggc 10380
   cagcgtaggg tggccggggc tccgggggcg agatcttcca acataaggcg atgatatccg 10440
   tagatgtacc tggacatcca ggtgatgccg gcggcggtgg tggaggcgcg cggaaagtcg 10500
   cggacgcggt tccagatgtt gcgcagcggc aaaaagtgct ccatggtcgg gacgctctgg 10560
   ccggtcaggc gcgcgcaatc gttgacgctc tagaccgtgc aaaaggagag cctgtaagcg 10620
   ggcactcttc cgtggtctgg tggataaatt cgcaagggta tcatggcgga cgaccggggt 10680
   tcgagccccg tatccggccg tccgccgtga tccatgcggt taccgcccgc gtgtcgaacc 10740
   caggtgtgcg acgtcagaca acgggggagt gctccttttg gcttccttcc aggcgcggcg 10800
   gctgctgcgc tagctttttt ggccactggc cgcgcgcagc gtaagcggtt aggctggaaa 10860
   gcgaaagcat taagtggctc gctccctgta gccggagggt tattttccaa gggttgagtc 10920
   gcgggacccc cggttcgagt ctcggaccgg ccggactgcg gcgaacgggg gtttgcctcc 10980
   ccgtcatgca agaccccgct tgcaaattcc tccggaaaca gggacgagcc ccttttttgc 11040
   ttttcccaga tgcatccggt gctgcggcag atgcgccccc ctcctcagca gcggcaagag 11100
   caagagcagc ggcagacatg cagggcaccc tcccctcctc ctaccgcgtc aggaggggcg 11160
   acatccgcgg ttgacgcggc agcagatggt gattacgaac ccccgcggcg ccgggcccgg 11220
   cactacctgg acttggagga gggcgagggc ctggcgcggc taggagcgcc ctctcctgag 11280
   cggcacccaa gggtgcagct gaagcgtgat acgcgtgagg cgtacgtgcc gcggcagaac 11340
   ctgtttcgcg accgcgaggg agaggagccc gaggagatgc gggatcgaaa gttccacgca 11400
   gggcgcgagc tgcggcatgg cctgaatcgc gagcggttgc tgcgcgagga ggactttgag 11460
   cccgacgcgc gaaccgggat tagtcccgcg cgcgcacacg tggcggccgc cgacctggta 11520
   accgcatacg agcagacggt gaaccaggag attaactttc aaaaaagctt taacaaccac 11580
   gtgcgtacgc ttgtggcgcg cgaggaggtg gctataggac tgatgcatct gtgggacttt 11640
   gtaagcgcgc tggagcaaaa cccaaatagc aagccgctca tggcgcagct gttccttata 11700
   gtgcagcaca gcagggacaa cgaggcattc agggatgcgc tgctaaacat agtagagccc 11760
   gagggccgct ggctgctcga tttgataaac atcctgcaga gcatagtggt gcaggagcgc 11820
   agcttgagcc tggctgacaa ggtggccgcc atcaactatt ccatgcttag cctgggcaag 11880
   ttttacgccc gcaagatata ccatacccct tacgttccca tagacaagga ggtaaagatc 11940
   gaggggttct acatgcgcat ggcgctgaag gtgcttacct tgagcgacga cctgggcgtt 12000
   tatcgcaacg agcgcatcca caaggccgtg agcgtgagcc ggcggcgcga gctcagcgac 12060
   cgcgagctga tgcacagcct gcaaagggcc ctggctggca cgggcagcgg cgatagagag 12120
   gccgagtcct actttgacgc gggcgctgac ctgcgctggg ccccaagccg acgcgccctg 12180
   gaggcagctg gggccggacc tgggctggcg gtggcacccg cgcgcgctgg caacgtcggc 12240
   ggcgtggagg aatatgacga ggacgatgag tacgagccag aggacggcga gtactaagcg 12300
   gtgatgtttc tgatcagatg atgcaagacg caacggaccc ggcggtgcgg gcggcgctgc 12360
   agagccagcc gtccggcctt aactccacgg acgactggcg ccaggtcatg gaccgcatca 12420
   tgtcgctgac tgcgcgcaat cctgacgcgt tccggcagca gccgcaggcc aaccggctct 12480
   ccgcaattct ggaagcggtg gtcccggcgc gcgcaaaccc cacgcacgag aaggtgctgg 12540
   cgatcgtaaa cgcgctggcc gaaaacaggg ccatccggcc cgacgaggcc ggcctggtct 12600
   acgacgcgct gcttcagcgc gtggctcgtt acaacagcgg caacgtgcag accaacctgg 12660
   accggctggt gggggatgtg cgcgaggccg tggcgcagcg tgagcgcgcg cagcagcagg 12720
   gcaacctggg ctccatggtt gcactaaacg ccttcctgag tacacagccc gccaacgtgc 12780
   cgcggggaca ggaggactac accaactttg tgagcgcact gcggctaatg gtgactgaga 12840
   caccgcaaag tgaggtgtac cagtctgggc cagactattt tttccagacc agtagacaag 12900
   gcctgcagac cgtaaacctg agccaggctt tcaaaaactt gcaggggctg tggggggtgc 12960
   gggctcccac aggcgaccgc gcgaccgtgt ctagcttgct gacgcccaac tcgcgcctgt 13020
   tgctgctgct aatagcgccc ttcacggaca gtggcagcgt gtcccgggac acatacctag 13080
   gtcacttgct gacactgtac cgcgaggcca taggtcaggc gcatgtggac gagcatactt 13140
   tccaggagat tacaagtgtc agccgcgcgc tggggcagga ggacacgggc agcctggagg 13200
   caaccctaaa ctacctgctg accaaccggc ggcagaagat cccctcgttg cacagtttaa 13260
   acagcgagga ggagcgcatt ttgcgctacg tgcagcagag cgtgagcctt aacctgatgc 13320
   gcgacggggt aacgcccagc gtggcgctgg acatgaccgc gcgcaacatg gaaccgggca 13380
   tgtatgcctc aaaccggccg tttatcaacc gcctaatgga ctacttgcat cgcgcggccg 13440
   ccgtgaaccc cgagtatttc accaatgcca tcttgaaccc gcactggcta ccgccccctg 13500
   gtttctacac cgggggattc gaggtgcccg agggtaacga tggattcctc tgggacgaca 13560
   tagacgacag cgtgttttcc ccgcaaccgc agaccctgct agagttgcaa cagcgcgagc 13620
   aggcagaggc ggcgctgcga aaggaaagct tccgcaggcc aagcagcttg tccgatctag 13680
   gcgctgcggc cccgcggtca gatgctagta gcccatttcc aagcttgata gggtctctta 13740
   ccagcactcg caccacccgc ccgcgcctgc tgggcgagga ggagtaccta aacaactcgc 13800
   tgctgcagcc gcagcgcgaa aaaaacctgc ctccggcatt tcccaacaac gggatagaga 13860
   gcctagtgga caagatgagt agatggaaga cgtacgcgca ggagcacagg gacgtgccag 13920
   gcccgcgccc gcccacccgt cgtcaaaggc acgaccgtca gcggggtctg gtgtgggagg 13980
   acgatgactc ggcagacgac agcagcgtcc tggatttggg agggagtggc aacccgtttg 14040
   cgcaccttcg ccccaggctg gggagaatgt tttaaaaaaa aaaaaagcat gatgcaaaat 14100
   aaaaaactca ccaaggccat ggcaccgagc gttggttttc ttgtattccc cttagtatgc 14160
   ggcgcgcggc gatgtatgag gaaggtcctc ctccctccta cgagagtgtg gtgagcgcgg 14220
   cgccagtggc ggcggcgctg ggttctccct tcgatgctcc cctggacccg ccgtttgtgc 14280
   ctccgcggta cctgcggcct accgggggga gaaacagcat ccgttactct gagttggcac 14340
   ccctattcga caccacccgt gtgtacctgg tggacaacaa gtcaacggat gtggcatccc 14400
   tgaactacca gaacgaccac agcaactttc tgaccacggt cattcaaaac aatgactaca 14460
   gcccggggga ggcaagcaca cagaccatca atcttgacga ccggtcgcac tggggcggcg 14520
   acctgaaaac catcctgcat accaacatgc caaatgtgaa cgagttcatg tttaccaata 14580
   agtttaaggc gcgggtgatg gtgtcgcgct tgcctactaa ggacaatcag gtggagctga 14640
   aatacgagtg ggtggagttc acgctgcccg agggcaacta ctccgagacc atgaccatag 14700
   accttatgaa caacgcgatc gtggagcact acttgaaagt gggcagacag aacggggttc 14760
   tggaaagcga catcggggta aagtttgaca cccgcaactt cagactgggg tttgaccccg 14820
   tcactggtct tgtcatgcct ggggtatata caaacgaagc cttccatcca gacatcattt 14880
   tgctgccagg atgcggggtg gacttcaccc acagccgcct gagcaacttg ttgggcatcc 14940
   gcaagcggca acccttccag gagggcttta ggatcaccta cgatgatctg gagggtggta 15000
   acattcccgc actgttggat gtggacgcct accaggcgag cttgaaagat gacaccgaac 15060
   agggcggggg tggcgcaggc ggcagcaaca gcagtggcag cggcgcggaa gagaactcca 15120
   acgcggcagc cgcggcaatg cagccggtgg aggacatgaa cgatcatgcc attcgcggcg 15180
   acacctttgc cacacgggct gaggagaagc gcgctgaggc cgaagcagcg gccgaagctg 15240
   ccgcccccgc tgcgcaaccc gaggtcgaga agcctcagaa gaaaccggtg atcaaacccc 15300
   tgacagagga cagcaagaaa cgcagttaca acctaataag caatgacagc accttcaccc 15360
   agtaccgcag ctggtacctt gcatacaact acggcgaccc tcagaccgga atccgctcat 15420
   ggaccctgct ttgcactcct gacgtaacct gcggctcgga gcaggtctac tggtcgttgc 15480
   cagacatgat gcaagacccc gtgaccttcc gctccacgcg ccagatcagc aactttccgg 15540
   tggtgggcgc cgagctgttg cccgtgcact ccaagagctt ctacaacgac caggccgtct 15600
   actcccaact catccgccag tttacctctc tgacccacgt gttcaatcgc tttcccgaga 15660
   accagatttt ggcgcgcccg ccagccccca ccatcaccac cgtcagtgaa aacgttcctg 15720
   ctctcacaga tcacgggacg ctaccgctgc gcaacagcat cggaggagtc cagcgagtga 15780
   ccattactga cgccagacgc cgcacctgcc cctacgttta caaggccctg ggcatagtct 15840
   cgccgcgcgt cctatcgagc cgcacttttt gagcaagcat gtccatcctt atatcgccca 15900
   gcaataacac aggctggggc ctgcgcttcc caagcaagat gtttggcggg gccaagaagc 15960
   gctccgacca acacccagtg cgcgtgcgcg ggcactaccg cgcgccctgg ggcgcgcaca 16020
   aacgcggccg cactgggcgc accaccgtcg atgacgccat cgacgcggtg gtggaggagg 16080
   cgcgcaacta cacgcccacg ccgccaccag tgtccacagt ggacgcggcc attcagaccg 16140
   tggtgcgcgg agcccggcgc tatgctaaaa tgaagagacg gcggaggcgc gtagcacgtc 16200
   gccaccgccg ccgacccggc actgccgccc aacgcgcggc ggcggccctg cttaaccgcg 16260
   cacgtcgcac cggccgacgg gcggccatgc gggccgctcg aaggctggcc gcgggtattg 16320
   tcactgtgcc ccccaggtcc aggcgacgag cggccgccgc agcagccgcg gccattagtg 16380
   ctatgactca gggtcgcagg ggcaacgtgt attgggtgcg cgactcggtt agcggcctgc 16440
   gcgtgcccgt gcgcacccgc cccccgcgca actagattgc aagaaaaaac tacttagact 16500
   cgtactgttg tatgtatcca gcggcggcgg cgcgcaacga agctatgtcc aagcgcaaaa 16560
   tcaaagaaga gatgctccag gtcatcgcgc cggagatcta tggccccccg aagaaggaag 16620
   agcaggatta caagccccga aagctaaagc gggtcaaaaa gaaaaagaaa gatgatgatg 16680
   atgaacttga cgacgaggtg gaactgctgc acgctaccgc gcccaggcga cgggtacagt 16740
   ggaaaggtcg acgcgtaaaa cgtgttttgc gacccggcac caccgtagtc tttacgcccg 16800
   gtgagcgctc cacccgcacc tacaagcgcg tgtatgatga ggtgtacggc gacgaggacc 16860
   tgcttgagca ggccaacgag cgcctcgggg agtttgccta cggaaagcgg cataaggaca 16920
   tgctggcgtt gccgctggac gagggcaacc caacacctag cctaaagccc gtaacactgc 16980
   agcaggtgct gcccgcgctt gcaccgtccg aagaaaagcg cggcctaaag cgcgagtctg 17040
   gtgacttggc acccaccgtg cagctgatgg tacccaagcg ccagcgactg gaagatgtct 17100
   tggaaaaaat gaccgtggaa cctgggctgg agcccgaggt ccgcgtgcgg ccaatcaagc 17160
   aggtggcgcc gggactgggc gtgcagaccg tggacgttca gatacccact accagtagca 17220
   ccagtattgc caccgccaca gagggcatgg agacacaaac gtccccggtt gcctcagcgg 17280
   tggcggatgc cgcggtgcag gcggtcgctg cggccgcgtc caagacctct acggaggtgc 17340
   aaacggaccc gtggatgttt cgcgtttcag ccccccggcg cccgcgccgt tcgaggaagt 17400
   acggcgccgc cagcgcgcta ctgcccgaat atgccctaca tccttccatt gcgcctaccc 17460
   ccggctatcg tggctacacc taccgcccca gaagacgagc aactacccga cgccgaacca 17520
   ccactggaac ccgccgccgc cgtcgccgtc gccagcccgt gctggccccg atttccgtgc 17580
   gcagggtggc tcgcgaagga ggcaggaccc tggtgctgcc aacagcgcgc taccacccca 17640
   gcatcgttta aaagccggtc tttgtggttc ttgcagatat ggccctcacc tgccgcctcc 17700
   gtttcccggt gccgggattc cgaggaagaa tgcaccgtag gaggggcatg gccggccacg 17760
   gcctgacggg cggcatgcgt cgtgcgcacc accggcggcg gcgcgcgtcg caccgtcgca 17820
   tgcgcggcgg tatcctgccc ctccttattc cactgatcgc cgcggcgatt ggcgccgtgc 17880
   ccggaattgc atccgtggcc ttgcaggcgc agagacactg attaaaaaca agttgcatgt 17940
   ggaaaaatca aaataaaaag tctggactct cacgctcgct tggtcctgta actattttgt 18000
   agaatggaag acatcaactt tgcgtctctg gccccgcgac acggctcgcg cccgttcatg 18060
   ggaaactggc aagatatcgg caccagcaat atgagcggtg gcgccttcag ctggggctcg 18120
   ctgtggagcg gcattaaaaa tttcggttcc accgttaaga actatggcag caaggcctgg 18180
   aacagcagca caggccagat gctgagggat aagttgaaag agcaaaattt ccaacaaaag 18240
   gtggtagatg gcctggcctc tggcattagc ggggtggtgg acctggccaa ccaggcagtg 18300
   caaaataaga ttaacagtaa gcttgatccc cgccctcccg tagaggagcc tccaccggcc 18360
   gtggagacag tgtctccaga ggggcgtggc gaaaagcgtc cgcgccccga cagggaagaa 18420
   actctggtga cgcaaataga cgagcctccc tcgtacgagg aggcactaaa gcaaggcctg 18480
   cccaccaccc gtcccatcgc gcccatggct accggagtgc tgggccagca cacacccgta 18540
   acgctggacc tgcctccccc cgccgacacc cagcagaaac ctgtgctgcc aggcccgacc 18600
   gccgttgttg taacccgtcc tagccgcgcg tccctgcgcc gcgccgccag cggtccgcga 18660
   tcgttgcggc ccgtagccag tggcaactgg caaagcacac tgaacagcat cgtgggtctg 18720
   ggggtgcaat ccctgaagcg ccgacgatgc ttctgatagc taacgtgtcg tatgtgtgtc 18780
   atgtatgcgt ccatgtcgcc gccagaggag ctgctgagcc gccgcgcgcc cgctttccaa 18840
   gatggctacc ccttcgatga tgccgcagtg gtcttacatg cacatctcgg gccaggacgc 18900
   ctcggagtac ctgagccccg ggctggtgca gtttgcccgc gccaccgaga cgtacttcag 18960
   cctgaataac aagtttagaa accccacggt ggcgcctacg cacgacgtga ccacagaccg 19020
   gtcccagcgt ttgacgctgc ggttcatccc tgtggaccgt gaggatactg cgtactcgta 19080
   caaggcgcgg ttcaccctag ctgtgggtga taaccgtgtg ctggacatgg cttccacgta 19140
   ctttgacatc cgcggcgtgc tggacagggg ccctactttt aagccctact ctggcactgc 19200
   ctacaacgcc ctggctccca agggtgcccc aaatccttgc gaatgggatg aagctgctac 19260
   tgctcttgaa ataaacctag aagaagagga cgatgacaac gaagacgaag tagacgagca 19320
   agctgagcag caaaaaactc acgtatttgg gcaggcgcct tattctggta taaatattac 19380
   aaaggagggt attcaaatag gtgtcgaagg tcaaacacct aaatatgccg ataaaacatt 19440
   tcaacctgaa cctcaaatag gagaatctca gtggtacgaa acagaaatta atcatgcagc 19500
   tgggagagtc ctaaaaaaga ctaccccaat gaaaccatgt tacggttcat atgcaaaacc 19560
   cacaaatgaa aatggagggc aaggcattct tgtaaagcaa caaaatggaa agctagaaag 19620
   tcaagtggaa atgcaatttt tctcaactac tgaggcagcc gcaggcaatg gtgataactt 19680
   gactcctaaa gtggtattgt acagtgaaga tgtagatata gaaaccccag acactcatat 19740
   ttcttacatg cccactatta aggaaggtaa ctcacgagaa ctaatgggcc aacaatctat 19800
   gcccaacagg cctaattaca ttgcttttag ggacaatttt attggtctaa tgtattacaa 19860
   cagcacgggt aatatgggtg ttctggcggg ccaagcatcg cagttgaatg ctgttgtaga 19920
   tttgcaagac agaaacacag agctttcata ccagcttttg cttgattcca ttggtgatag 19980
   aaccaggtac ttttctatgt ggaatcaggc tgttgacagc tatgatccag atgttagaat 20040
   tattgaaaat catggaactg aagatgaact tccaaattac tgctttccac tgggaggtgt 20100
   gattaataca gagactctta ccaaggtaaa acctaaaaca ggtcaggaaa atggatggga 20160
   aaaagatgct acagaatttt cagataaaaa tgaaataaga gttggaaata attttgccat 20220
   ggaaatcaat ctaaatgcca acctgtggag aaatttcctg tactccaaca tagcgctgta 20280
   tttgcccgac aagctaaagt acagtccttc caacgtaaaa atttctgata acccaaacac 20340
   ctacgactac atgaacaagc gagtggtggc tcccgggcta gtggactgct acattaacct 20400
   tggagcacgc tggtcccttg actatatgga caacgtcaac ccatttaacc accaccgcaa 20460
   tgctggcctg cgctaccgct caatgttgct gggcaatggt cgctatgtgc ccttccacat 20520
   ccaggtgcct cagaagttct ttgccattaa aaacctcctt ctcctgccgg gctcatacac 20580
   ctacgagtgg aacttcagga aggatgttaa catggttctg cagagctccc taggaaatga 20640
   cctaagggtt gacggagcca gcattaagtt tgatagcatt tgcctttacg ccaccttctt 20700
   ccccatggcc cacaacaccg cctccacgct tgaggccatg cttagaaacg acaccaacga 20760
   ccagtccttt aacgactatc tctccgccgc caacatgctc taccctatac ccgccaacgc 20820
   taccaacgtg cccatatcca tcccctcccg caactgggcg gctttccgcg gctgggcctt 20880
   cacgcgcctt aagactaagg aaaccccatc actgggctcg ggctacgacc cttattacac 20940
   ctactctggc tctataccct acctagatgg aaccttttac ctcaaccaca cctttaagaa 21000
   ggtggccatt acctttgact cttctgtcag ctggcctggc aatgaccgcc tgcttacccc 21060
   caacgagttt gaaattaagc gctcagttga cggggagggt tacaacgttg cccagtgtaa 21120
   catgaccaaa gactggttcc tggtacaaat gctagctaac tataacattg gctaccaggg 21180
   cttctatatc ccagagagct acaaggaccg catgtactcc ttctttagaa acttccagcc 21240
   catgagccgt caggtggtgg atgatactaa atacaaggac taccaacagg tgggcatcct 21300
   acaccaacac aacaactctg gatttgttgg ctaccttgcc cccaccatgc gcgaaggaca 21360
   ggcctaccct gctaacttcc cctatccgct tataggcaag accgcagttg acagcattac 21420
   ccagaaaaag tttctttgcg atcgcaccct ttggcgcatc ccattctcca gtaactttat 21480
   gtccatgggc gcactcacag acctgggcca aaaccttctc tacgccaact ccgcccacgc 21540
   gctagacatg acttttgagg tggatcccat ggacgagccc acccttcttt atgttttgtt 21600
   tgaagtcttt gacgtggtcc gtgtgcacca gccgcaccgc ggcgtcatcg aaaccgtgta 21660
   cctgcgcacg cccttctcgg ccggcaacgc cacaacataa agaagcaagc aacatcaaca 21720
   acagctgccg ccatgggctc cagtgagcag gaactgaaag ccattgtcaa agatcttggt 21780
   tgtgggccat attttttggg cacctatgac aagcgctttc caggctttgt ttctccacac 21840
   aagctcgcct gcgccatagt caatacggcc ggtcgcgaga ctgggggcgt acactggatg 21900
   gcctttgcct ggaacccgca ctcaaaaaca tgctacctct ttgagccctt tggcttttct 21960
   gaccagcgac tcaagcaggt ttaccagttt gagtacgagt cactcctgcg ccgtagcgcc 22020
   attgcttctt cccccgaccg ctgtataacg ctggaaaagt ccacccaaag cgtacagggg 22080
   cccaactcgg ccgcctgtgg actattctgc tgcatgtttc tccacgcctt tgccaactgg 22140
   ccccaaactc ccatggatca caaccccacc atgaacctta ttaccggggt acccaactcc 22200
   atgctcaaca gtccccaggt acagcccacc ctgcgtcgca accaggaaca gctctacagc 22260
   ttcctggagc gccactcgcc ctacttccgc agccacagtg cgcagattag gagcgccact 22320
   tctttttgtc acttgaaaaa catgtaaaaa taatgtacta gagacacttt caataaaggc 22380
   aaatgctttt atttgtacac tctcgggtga ttatttaccc ccacccttgc cgtctgcgcc 22440
   gtttaaaaat caaaggggtt ctgccgcgca tcgctatgcg ccactggcag ggacacgttg 22500
   cgatactggt gtttagtgct ccacttaaac tcaggcacaa ccatccgcgg cagctcggtg 22560
   aagttttcac tccacaggct gcgcaccatc accaacgcgt ttagcaggtc gggcgccgat 22620
   atcttgaagt cgcagttggg gcctccgccc tgcgcgcgcg agttgcgata cacagggttg 22680
   cagcactgga acactatcag cgccgggtgg tgcacgctgg ccagcacgct cttgtcggag 22740
   atcagatccg cgtccaggtc ctccgcgttg ctcagggcga acggagtcaa ctttggtagc 22800
   tgccttccca aaaagggcgc gtgcccaggc tttgagttgc actcgcaccg tagtggcatc 22860
   aaaaggtgac cgtgcccggt ctgggcgtta ggatacagcg cctgcataaa agccttgatc 22920
   tgcttaaaag ccacctgagc ctttgcgcct tcagagaaga acatgccgca agacttgccg 22980
   gaaaactgat tggccggaca ggccgcgtcg tgcacgcagc accttgcgtc ggtgttggag 23040
   atctgcacca catttcggcc ccaccggttc ttcacgatct tggccttgct agactgctcc 23100
   ttcagcgcgc gctgcccgtt ttcgctcgtc acatccattt caatcacgtg ctccttattt 23160
   atcataatgc ttccgtgtag acacttaagc tcgccttcga tctcagcgca gcggtgcagc 23220
   cacaacgcgc agcccgtggg ctcgtgatgc ttgtaggtca cctctgcaaa cgactgcagg 23280
   tacgcctgca ggaatcgccc catcatcgtc acaaaggtct tgttgctggt gaaggtcagc 23340
   tgcaacccgc ggtgctcctc gttcagccag gtcttgcata cggccgccag agcttccact 23400
   tggtcaggca gtagtttgaa gttcgccttt agatcgttat ccacgtggta cttgtccatc 23460
   agcgcgcgcg cagcctccat gcccttctcc cacgcagaca cgatcggcac actcagcggg 23520
   ttcatcaccg taatttcact ttccgcttcg ctgggctctt cctcttcctc ttgcgtccgc 23580
   ataccacgcg ccactgggtc gtcttcattc agccgccgca ctgtgcgctt acctcctttg 23640
   ccatgcttga ttagcaccgg tgggttgctg aaacccacca tttgtagcgc cacatcttct 23700
   ctttcttcct cgctgtccac gattacctct ggtgatggcg ggcgctcggg cttgggagaa 23760
   gggcgcttct ttttcttctt gggcgcaatg gccaaatccg ccgccgaggt cgatggccgc 23820
   gggctgggtg tgcgcggcac cagcgcgtct tgtgatgagt cttcctcgtc ctcggactcg 23880
   atacgccgcc tcatccgctt ttttgggggc gcccggggag gcggcggcga cggggacggg 23940
   gacgacacgt cctccatggt tgggggacgt cgcgccgcac cgcgtccgcg ctcgggggtg 24000
   gtttcgcgct gctcctcttc ccgactggcc atttccttct cctataggca gaaaaagatc 24060
   atggagtcag tcgagaagaa ggacagccta accgccccct ctgagttcgc caccaccgcc 24120
   tccaccgatg ccgccaacgc gcctaccacc ttccccgtcg aggcaccccc gcttgaggag 24180
   gaggaagtga ttatcgagca ggacccaggt tttgtaagcg aagacgacga ggaccgctca 24240
   gtaccaacag aggataaaaa gcaagaccag gacaacgcag aggcaaacga ggaacaagtc 24300
   gggcgggggg acgaaaggca tggcgactac ctagatgtgg gagacgacgt gctgttgaag 24360
   catctgcagc gccagtgcgc cattatctgc gacgcgttgc aagagcgcag cgatgtgccc 24420
   ctcgccatag cggatgtcag ccttgcctac gaacgccacc tattctcacc gcgcgtaccc 24480
   cccaaacgcc aagaaaacgg cacatgcgag cccaacccgc gcctcaactt ctaccccgta 24540
   tttgccgtgc cagaggtgct tgccacctat cacatctttt tccaaaactg caagataccc 24600
   ctatcctgcc gtgccaaccg cagccgagcg gacaagcagc tggccttgcg gcagggcgct 24660
   gtcatacctg atatcgcctc gctcaacgaa gtgccaaaaa tctttgaggg tcttggacgc 24720
   gacgagaagc gcgcggcaaa cgctctgcaa caggaaaaca gcgaaaatga aagtcactct 24780
   ggagtgttgg tggaactcga gggtgacaac gcgcgcctag ccgtactaaa acgcagcatc 24840
   gaggtcaccc actttgccta cccggcactt aacctacccc ccaaggtcat gagcacagtc 24900
   atgagtgagc tgatcgtgcg ccgtgcgcag cccctggaga gggatgcaaa tttgcaagaa 24960
   caaacagagg agggcctacc cgcagttggc gacgagcagc tagcgcgctg gcttcaaacg 25020
   cgcgagcctg ccgacttgga ggagcgacgc aaactaatga tggccgcagt gctcgttacc 25080
   gtggagcttg agtgcatgca gcggttcttt gctgacccgg agatgcagcg caagctagag 25140
   gaaacattgc actacacctt tcgacagggc tacgtacgcc aggcctgcaa gatctccaac 25200
   gtggagctct gcaacctggt ctcctacctt ggaattttgc acgaaaaccg ccttgggcaa 25260
   aacgtgcttc attccacgct caagggcgag gcgcgccgcg actacgtccg cgactgcgtt 25320
   tacttatttc tatgctacac ctggcagacg gccatgggcg tttggcagca gtgcttggag 25380
   gagtgcaacc tcaaggagct gcagaaactg ctaaagcaaa acttgaagga cctatggacg 25440
   gccttcaacg agcgctccgt ggccgcgcac ctggcggaca tcattttccc cgaacgcctg 25500
   cttaaaaccc tgcaacaggg tctgccagac ttcaccagtc aaagcatgtt gcagaacttt 25560
   aggaacttta tcctagagcg ctcaggaatc ttgcccgcca cctgctgtgc acttcctagc 25620
   gactttgtgc ccattaagta ccgcgaatgc cctccgccgc tttggggcca ctgctacctt 25680
   ctgcagctag ccaactacct tgcctaccac tctgacataa tggaagacgt gagcggtgac 25740
   ggtctactgg agtgtcactg tcgctgcaac ctatgcaccc cgcaccgctc cctggtttgc 25800
   aattcgcagc tgcttaacga aagtcaaatt atcggtacct ttgagctgca gggtccctcg 25860
   cctgacgaaa agtccgcggc tccggggttg aaactcactc cggggctgtg gacgtcggct 25920
   taccttcgca aatttgtacc tgaggactac cacgcccacg agattaggtt ctacgaagac 25980
   caatcccgcc cgcctaatgc ggagcttacc gcctgcgtca ttacccaggg ccacattctt 26040
   ggccaattgc aagccatcaa caaagcccgc caagagtttc tgctacgaaa gggacggggg 26100
   gtttacttgg acccccagtc cggcgaggag ctcaacccaa tccccccgcc gccgcagccc 26160
   tatcagcagc agccgcgggc ccttgcttcc caggatggca cccaaaaaga agctgcagct 26220
   gccgccgcca cccacggacg aggaggaata ctgggacagt caggcagagg aggttttgga 26280
   cgaggaggag gaggacatga tggaagactg ggagagccta gacgaggaag cttccgaggt 26340
   cgaagaggtg tcagacgaaa caccgtcacc ctcggtcgca ttcccctcgc cggcgcccca 26400
   gaaatcggca accggttcca gcatggctac aacctccgct cctcaggcgc cgccggcact 26460
   gcccgttcgc cgacccaacc gtagatggga caccactgga accagggccg gtaagtccaa 26520
   gcagccgccg ccgttagccc aagagcaaca acagcgccaa ggctaccgct catggcgcgg 26580
   gcacaagaac gccatagttg cttgcttgca agactgtggg ggcaacatct ccttcgcccg 26640
   ccgctttctt ctctaccatc acggcgtggc cttcccccgt aacatcctgc attactaccg 26700
   tcatctctac agcccatact gcaccggcgg cagcggcagc aacagcagcg gccacacaga 26760
   agcaaaggcg accggatagc aagactctga caaagcccaa gaaatccaca gcggcggcag 26820
   cagcaggagg aggagcgctg cgtctggcgc ccaacgaacc cgtatcgacc cgcgagctta 26880
   gaaacaggat ttttcccact ctgtatgcta tatttcaaca gagcaggggc caagaacaag 26940
   agctgaaaat aaaaaacagg tctctgcgat ccctcacccg cagctgcctg tatcacaaaa 27000
   gcgaagatca gcttcggcgc acgctggaag acgcggaggc tctcttcagt aaatactgcg 27060
   cgctgactct taaggactag tttcgcgccc tttctcaaat ttaagcgcga aaactacgtc 27120
   atctccagcg gccacacccg gcgccagcac ctgttgtcag cgccattatg agcaaggaaa 27180
   ttcccacgcc ctacatgtgg agttaccagc cacaaatggg acttgcggct ggagctgccc 27240
   aagactactc aacccgaata aactacatga gcgcgggacc ccacatgata tcccgggtca 27300
   acggaatacg cgcccaccga aaccgaattc tcctggaaca ggcggctatt accaccacac 27360
   ctcgtaataa ccttaatccc cgtagttggc ccgctgccct ggtgtaccag gaaagtcccg 27420
   ctcccaccac tgtggtactt cccagagacg cccaggccga agttcagatg actaactcag 27480
   gggcgcagct tgcgggcggc tttcgtcaca gggtgcggtc gcccgggcag ggtataactc 27540
   acctgacaat cagagggcga ggtattcagc tcaacgacga gtcggtgagc tcctcgcttg 27600
   gtctccgtcc ggacgggaca tttcagatcg gcggcgccgg ccgctcttca ttcacgcctc 27660
   gtcaggcaat cctaactctg cagacctcgt cctctgagcc gcgctctgga ggcattggaa 27720
   ctctgcaatt tattgaggag tttgtgccat cggtctactt taaccccttc tcgggacctc 27780
   ccggccacta tccggatcaa tttattccta actttgacgc ggtaaaggac tcggcggacg 27840
   gctacgactg aatgttaagt ggagaggcag agcaactgcg cctgaaacac ctggtccact 27900
   gtcgccgcca caagtgcttt gcccgcgact ccggtgagtt ttgctacttt gaattgcccg 27960
   aggatcatat cgagggcccg gcgcacggcg tccggcttac cgcccaggga gagcttgccc 28020
   gtagcctgat tcgggagttt acccagcgcc ccctgctagt tgagcgggac aggggaccct 28080
   gtgttctcac tgtgatttgc aactgtccta accctggatt acatcaagat ctttgttgcc 28140
   atctctgtgc tgagtataat aaatacagaa attaaaatat actggggctc ctatcgccat 28200
   cctgtaaacg ccaccgtctt cacccgccca agcaaaccaa ggcgaacctt acctggtact 28260
   tttaacatct ctccctctgt gatttacaac agtttcaacc cagacggagt gagtctacga 28320
   gagaacctct ccgagctcag ctactccatc agaaaaaaca ccaccctcct tacctgccgg 28380
   gaacgtacga gtgcgtcacc ggccgctgca ccacacctac cgcctgaccg taaaccagac 28440
   tttttccgga cagacctcaa taactctgtt taccagaaca ggaggtgagc ttagaaaacc 28500
   cttagggtat taggccaaag gcgcagctac tgtggggttt atgaacaatt caagcaactc 28560
   tacgggctat tctaattcag gtttctctag aatcggggtt ggggttattc tctgtcttgt 28620
   gattctcttt attcttatac taacgcttct ctgcctaagg ctcgccgcct gctgtgtgca 28680
   catttgcatt tattgtcagc tttttaaacg ctggggtcgc cacccaagat gattaggtac 28740
   ataatcctag gtttactcac ccttgcgtca gcccacggta ccacccaaaa ggtggatttt 28800
   aaggagccag cctgtaatgt tacattcgca gctgaagcta atgagtgcac cactcttata 28860
   aaatgcacca cagaacatga aaagctgctt attcgccaca aaaacaaaat tggcaagtat 28920
   gctgtttatg ctatttggca gccaggtgac actacagagt ataatgttac agttttccag 28980
   ggtaaaagtc ataaaacttt tatgtatact tttccatttt atgaaatgtg cgacattacc 29040
   atgtacatga gcaaacagta taagttgtgg cccccacaaa attgtgtgga aaacactggc 29100
   actttctgct gcactgctat gctaattaca gtgctcgctt tggtctgtac cctactctat 29160
   attaaataca aaagcagacg cagctttatt gaggaaaaga aaatgcctta atttactaag 29220
   ttacaaagct aatgtcacca ctaactgctt tactcgctgc ttgcaaaaca aattcaaaaa 29280
   gttagcatta taattagaat aggatttaaa ccccccggtc atttcctgct caataccatt 29340
   cccctgaaca attgactcta tgtgggatat gctccagcgc tacaaccttg aagtcaggct 29400
   tcctggatgt cagcatctga ctttggccag cacctgtccc gcggatttgt tccagtccaa 29460
   ctacagcgac ccaccctaac agagatgacc aacacaacca acgcggccgc cgctaccgga 29520
   cttacatcta ccacaaatac accccaagtt tctgcctttg tcaataactg ggataacttg 29580
   ggcatgtggt ggttctccat agcgcttatg tttgtatgcc ttattattat gtggctcatc 29640
   tgctgcctaa agcgcaaacg cgcccgacca cccatctata gtcccatcat tgtgctacac 29700
   ccaaacaatg atggaatcca tagattggac ggactgaaac acatgttctt ttctcttaca 29760
   gtatgattaa atgagacatg attcctcgag tttttatatt actgaccctt gttgcgcttt 29820
   ttttgtgcgt gctccacatt ggctgcggtt tctcacatcg aagtagactg cattccagcc 29880
   ttcacagtct atttgcttta cggatttgtc accctcacgc tcatctgcag cctcatcact 29940
   gtggtcatcg cctttatcca gtgcattgac tgggtctgtg tgcgctttgc atatctcaga 30000
   caccatcccc agtacaggga caggactata gctgagcttc ttagaattct ttaattatga 30060
   aatttactgt gacttttctg ctgattattt gcaccctatc tgcgttttgt tccccgacct 30120
   ccaagcctca aagacatata tcatgcagat tcactcgtat atggaatatt ccaagttgct 30180
   acaatgaaaa aagcgatctt tccgaagcct ggttatatgc aatcatctct gttatggtgt 30240
   tctgcagtac catcttagcc ctagctatat atccctacct tgacattggc tggaacgcaa 30300
   tagatgccat gaaccaccca actttccccg cgcccgctat gcttccactg caacaagttg 30360
   ttgccggcgg ctttgtccca gccaatcagc ctcgcccacc ttctcccacc cccactgaaa 30420
   tcagctactt taatctaaca ggaggagatg actgacaccc tagatctaga aatggacgga 30480
   attattacag agcagcgcct gctagaaaga cgcagggcag cggccgagca acagcgcatg 30540
   aatcaagagc tccaagacat ggttaacttg caccagtgca aaaggggtat cttttgtctg 30600
   gtaaagcagg ccaaagtcac ctacgacagt aataccaccg gacaccgcct tagctacaag 30660
   ttgccaacca agcgtcagaa attggtggtc atggtgggag aaaagcccat taccataact 30720
   cagcactcgg tagaaaccga aggctgcatt cactcacctt gtcaaggacc tgaggatctc 30780
   tgcaccctta ttaagaccct gtgcggtctc aaagatctta ttccctttaa ctaataaaaa 30840
   aaaataataa agcatcactt acttaaaatc agttagcaaa tttctgtcca gtttattcag 30900
   cagcacctcc ttgccctcct cccagctctg gtattgcagc ttcctcctgg ctgcaaactt 30960
   tctccacaat ctaaatggaa tgtcagtttc ctcctgttcc tgtccatccg cacccactat 31020
   cttcatgttg ttgcagatga agcgcgcaag accgtctgaa gataccttca accccgtgta 31080
   tccatatgac acggaaaccg gtcctccaac tgtgcctttt cttactcctc cctttgtatc 31140
   ccccaatggg tttcaagaga gtccccctgg ggtactctct ttgcgcctat ccgaacctct 31200
   agttacctcc aatggcatgc ttgcgctcaa aatgggcaac ggcctctctc tggacgaggc 31260
   cggcaacctt acctcccaaa atgtaaccac tgtgagccca cctctcaaaa aaaccaagtc 31320
   aaacataaac ctggaaatat ctgcacccct cacagttacc tcagaagccc taactgtggc 31380
   tgccgccgca cctctaatgg tcgcgggcaa cacactcacc atgcaatcac aggccccgct 31440
   aaccgtgcac gactccaaac ttagcattgc cacccaagga cccctcacag tgtcagaagg 31500
   aaagctagcc ctgcaaacat caggccccct caccaccacc gatagcagta cccttactat 31560
   cactgcctca ccccctctaa ctactgccac tggtagcttg ggcattgact tgaaagagcc 31620
   catttataca caaaatggaa aactaggact aaagtacggg gctcctttgc atgtaacaga 31680
   cgacctaaac actttgaccg tagcaactgg tccaggtgtg actattaata atacttcctt 31740
   gcaaactaaa gttactggag ccttgggttt tgattcacaa ggcaatatgc aacttaatgt 31800
   agcaggagga ctaaggattg attctcaaaa cagacgcctt atacttgatg ttagttatcc 31860
   gtttgatgct caaaaccaac taaatctaag actaggacag ggccctcttt ttataaactc 31920
   agcccacaac ttggatatta actacaacaa aggcctttac ttgtttacag cttcaaacaa 31980
   ttccaaaaag cttgaggtta acctaagcac tgccaagggg ttgatgtttg acgctacagc 32040
   catagccatt aatgcaggag atgggcttga atttggttca cctaatgcac caaacacaaa 32100
   tcccctcaaa acaaaaattg gccatggcct agaatttgat tcaaacaagg ctatggttcc 32160
   taaactagga actggcctta gttttgacag cacaggtgcc attacagtag gaaacaaaaa 32220
   taatgataag ctaactttgt ggaccacacc agctccatct cctaactgta gactaaatgc 32280
   agagaaagat gctaaactca ctttggtctt aacaaaatgt ggcagtcaaa tacttgctac 32340
   agtttcagtt ttggctgtta aaggcagttt ggctccaata tctggaacag ttcaaagtgc 32400
   tcatcttatt ataagatttg acgaaaatgg agtgctacta aacaattcct tcctggaccc 32460
   agaatattgg aactttagaa atggagatct tactgaaggc acagcctata caaacgctgt 32520
   tggatttatg cctaacctat cagcttatcc aaaatctcac ggtaaaactg ccaaaagtaa 32580
   cattgtcagt caagtttact taaacggaga caaaactaaa cctgtaacac taaccattac 32640
   actaaacggt acacaggaaa caggagacac aactccaagt gcatactcta tgtcattttc 32700
   atgggactgg tctggccaca actacattaa tgaaatattt gccacatcct cttacacttt 32760
   ttcatacatt gcccaagaat aaagaatcgt ttgtgttatg tttcaacgtg tttatttttc 32820
   aattgcagaa aatttcaagt catttttcat tcagtagtat agccccacca ccacatagct 32880
   tatacagatc accgtacctt aatcaaactc acagaaccct agtattcaac ctgccacctc 32940
   cctcccaaca cacagagtac acagtccttt ctccccggct ggccttaaaa agcatcatat 33000
   catgggtaac agacatattc ttaggtgtta tattccacac ggtttcctgt cgagccaaac 33060
   gctcatcagt gatattaata aactccccgg gcagctcact taagttcatg tcgctgtcca 33120
   gctgctgagc cacaggctgc tgtccaactt gcggttgctt aacgggcggc gaaggagaag 33180
   tccacgccta catgggggta gagtcataat cgtgcatcag gatagggcgg tggtgctgca 33240
   gcagcgcgcg aataaactgc tgccgccgcc gctccgtcct gcaggaatac aacatggcag 33300
   tggtctcctc agcgatgatt cgcaccgccc gcagcataag gcgccttgtc ctccgggcac 33360
   agcagcgcac cctgatctca cttaaatcag cacagtaact gcagcacagc accacaatat 33420
   tgttcaaaat cccacagtgc aaggcgctgt atccaaagct catggcgggg accacagaac 33480
   ccacgtggcc atcataccac aagcgcaggt agattaagtg gcgacccctc ataaacacgc 33540
   tggacataaa cattacctct tttggcatgt tgtaattcac cacctcccgg taccatataa 33600
   acctctgatt aaacatggcg ccatccacca ccatcctaaa ccagctggcc aaaacctgcc 33660
   cgccggctat acactgcagg gaaccgggac tggaacaatg acagtggaga gcccaggact 33720
   cgtaaccatg gatcatcatg ctcgtcatga tatcaatgtt ggcacaacac aggcacacgt 33780
   gcatacactt cctcaggatt acaagctcct cccgcgttag aaccatatcc cagggaacaa 33840
   cccattcctg aatcagcgta aatcccacac tgcagggaag acctcgcacg taactcacgt 33900
   tgtgcattgt caaagtgtta cattcgggca gcagcggatg atcctccagt atggtagcgc 33960
   gggtttctgt ctcaaaagga ggtagacgat ccctactgta cggagtgcgc cgagacaacc 34020
   gagatcgtgt tggtcgtagt gtcatgccaa atggaacgcc ggacgtagtc atatttcctg 34080
   aagcaaaacc aggtgcgggc gtgacaaaca gatctgcgtc tccggtctcg ccgcttagat 34140
   cgctctgtgt agtagttgta gtatatccac tctctcaaag catccaggcg ccccctggct 34200
   tcgggttcta tgtaaactcc ttcatgcgcc gctgccctga taacatccac caccgcagaa 34260
   taagccacac ccagccaacc tacacattcg ttctgcgagt cacacacggg aggagcggga 34320
   agagctggaa gaaccatgtt tttttttttt attccaaaag attatccaaa acctcaaaat 34380
   gaagatctat taagtgaacg cgctcccctc cggtggcgtg gtcaaactct acagccaaag 34440
   aacagataat ggcatttgta agatgttgca caatggcttc caaaaggcaa acggccctca 34500
   cgtccaagtg gacgtaaagg ctaaaccctt cagggtgaat ctcctctata aacattccag 34560
   caccttcaac catgcccaaa taattctcat ctcgccacct tctcaatata tctctaagca 34620
   aatcccgaat attaagtccg gccattgtaa aaatctgctc cagagcgccc tccaccttca 34680
   gcctcaagca gcgaatcatg attgcaaaaa ttcaggttcc tcacagacct gtataagatt 34740
   caaaagcgga acattaacaa aaataccgcg atcccgtagg tcccttcgca gggccagctg 34800
   aacataatcg tgcaggtctg cacggaccag cgcggccact tccccgccag gaaccatgac 34860
   aaaagaaccc acactgatta tgacacgcat actcggagct atgctaacca gcgtagcccc 34920
   gatgtaagct tgttgcatgg gcggcgatat aaaatgcaag gtgctgctca aaaaatcagg 34980
   caaagcctcg cgcaaaaaag aaagcacatc gtagtcatgc tcatgcagat aaaggcaggt 35040
   aagctccgga accaccacag aaaaagacac catttttctc tcaaacatgt ctgcgggttt 35100
   ctgcataaac acaaaataaa ataacaaaaa aacatttaaa cattagaagc ctgtcttaca 35160
   acaggaaaaa caacccttat aagcataaga cggactacgg ccatgccggc gtgaccgtaa 35220
   aaaaactggt caccgtgatt aaaaagcacc accgacagct cctcggtcat gtccggagtc 35280
   ataatgtaag actcggtaaa cacatcaggt tgattcacat cggtcagtgc taaaaagcga 35340
   ccgaaatagc ccgggggaat acatacccgc aggcgtagag acaacattac agcccccata 35400
   ggaggtataa caaaattaat aggagagaaa aacacataaa cacctgaaaa accctcctgc 35460
   ctaggcaaaa tagcaccctc ccgctccaga acaacataca gcgcttccac agcggcagcc 35520
   ataacagtca gccttaccag taaaaaagaa aacctattaa aaaaacacca ctcgacacgg 35580
   caccagctca atcagtcaca gtgtaaaaaa gggccaagtg cagagcgagt atatatagga 35640
   ctaaaaaatg acgtaacggt taaagtccac aaaaaacacc cagaaaaccg cacgcgaacc 35700
   tacgcccaga aacgaaagcc aaaaaaccca caacttcctc aaatcgtcac ttccgttttc 35760
   ccacgttacg tcacttccca ttttaagaaa actacaattc ccaacacata caagttactc 35820
   cgccctaaaa cctacgtcac ccgccccgtt cccacgcccc gcgccacgtc acaaactcca 35880
   ccccctcatt atcatattgg cttcaatcca aaataaggta tattattgat gatg 35934

## Description

### Field of the invention

The present invention relates to two-plasmid systems, and/or helper plasmids for producing recombinant AAV (rAAV) vectors. The invention further relates to methods using, or uses of, the two-plasmid systems, and/or helper plasmids of the invention.

### Background to the invention

Recombinant adeno-associated virus (rAAV) vectors have considerable potential for gene therapy due to their promising safety profile and their ability to transduce many tissues *in vivo.* However, production is still quite difficult and complex and scale-up of production at an industrial scale has been accomplished only to a limited degree. One reason for this is that rAAV production depends on a co-infection with a helper virus to propagate and establish a productive life-cycle. Infection of cells with a replication-competent helper virus, e.g. an adenovirus, for the production of rAAV has the disadvantage that resulting rAAV stocks are contaminated with helper virus, requiring validated virus removal steps in the down-stream purification process.

For this reason, the use of adenovirus co-infection is commonly avoided by providing the relevant adenoviral helper functions on a plasmid which is co-transfected together with several other plasmids containing the AAV Rep and Cap functions, and the rAAV "*vector genome*", *i.e.* the heterologous nucleic acid comprising the genetic *"payload"* of the rAAV, flanked by the inverted terminal repeat (ITR) sequences which ensure packaging of the heterologous nucleic acid within the produced viral particles. For example, Emmerling et al (2016) in section 3.1.2 describes four-plasmid systems wherein the Rep, Cap, and adenoviral helper functions are each provided on separate plasmids, together with the vector genome on a fourth plasmid. Plasmid synthesis is a major cost-of-goods driver and the use of four plasmids - all of which must enter the same cell in order for rAAV production to occur in that cell - is disadvantageous from an efficiency and economic perspective.

In section 3.1.3 of Emmerling et al (2016) a two-plasmid system is mentioned, in which one plasmid contains Rep, Cap and vector genome, with the adenoviral functions being provided on the second plasmid. In this regard it must be noted that one major concern of drug regulatory agencies regarding virus-based product safety, whether for oncolytic viruses or gene transfer vectors, is the generation of wild type-like revertants by recombination of the genetic information carried on the starting materials, e.g. in this case upon transfection of the production cells with plasmids collectively carrying this information. Recombination events between the plasmids can result in the generation of so-called replication competent (rc) viruses (in case of AAV, of replication competent AAV (rcAAV) particles). When Rep and Cap are present on the same plasmid, there is a risk of unacceptable levels of rcAAV being produced by intra- or inter-molecular (depending on which plasmid carries the ITR-heterologous nucleic acid-ITR) recombination.

Such a plasmid arrangement is also economically sub-optimal in the situation where it is desired to switch to a different vector genome (*i.e.* heterologous nucleic acid of interest; transgene cassette), or to switch to a different capsid serotype *(i.e.* a different Cap gene) which might be desired in the event that a different tissue tropism is sought. In either case, a new (Rep-Cap-vector genome) plasmid will need to be synthesised, the cost of each synthesis being in part a function of the plasmid length, to which the (unchanging) Rep gene contributes.

By way of further background: (i) US 2005/080027 discloses the optimized production of viral vectors derived from paroviruses in packaging and production cells by hsv infection or treatment with dna methylation inhibitors; (ii) US 2003/103939 discloses pseudotyped adeno-associated viruses and uses thereof; and (iii) CN 108048483 discloses a replication type recombinant adenovirus HAdV-5 vector system and the application thereof.

In order to satisfy the current demand of rAAV vector material for clinical trials and market supply, the following goals have yet to be achieved: (a) improved safety and quality profile of the rAAV vectors to meet the regulatory demands on vector quality; (b) improved economics of rAAV manufacture in terms of cost of starting materials for a given manufacturing campaign as well as cost of switching between campaigns; (c) high rAAV production yields; and (d) control over the proportion of produced rAAV particles containing ("full") or lacking ("empty") a complete recombinant vector genome.

### Summary of the invention

The present invention relates to a two-plasmid system for manufacturing of rAAV, wherein at least some of the adenovirus helper gene functions are combined with AAV Rep on one plasmid, and AAV Cap is combined with the rAAV vector genome on a second plasmid. The 'trans-split Rep-Cap' two-plasmid system is superior over known systems in achieving a combination of improved economics resulting from the simpler and more flexible format, with enhanced safety and quality attributes in addition to other surprising advantages as disclosed herein.

Accordingly, the invention provides the following aspects.
[1] A helper plasmid comprising at least one adeno-associated virus (AAV) rep gene encoding at least one functional AAV Rep protein and at least one helper virus gene, and which does not comprise a cap gene encoding a functional set of Cap proteins.
[2] A two-plasmid system comprising the helper plasmid of [1] and a vector plasmid.
[3] The two-plasmid system of [2], wherein:
   (i) the vector plasmid comprises:
      (a) an AAV cap gene encoding at least one functional AAV Cap protein; or
      (b) at least one AAV cap gene promoter, a cloning site operably linked to the AAV cap gene promoter, and an expression cassette flanked on at least one side by an inverted terminal repeat (ITR);
      wherein the vector plasmid does not comprise a rep gene encoding a functional Rep protein and the expression cassette comprises a transgene operably linked to at least one regulatory control element; and/or
   (ii) the ratio of helper plasmid to vector plasmid is between 1:1 and 1:4.
[4] The two-plasmid system or helper plasmid of any one of the preceding aspects, wherein the at least one AAV rep gene comprises a gene encoding a functional Rep 52 protein, at least one gene encoding a functional Rep 40 protein, and a gene encoding a functional Rep 68 protein.
[5] The two-plasmid system or helper plasmid of any one of the preceding aspects, wherein:
   (i) the at least one AAV rep gene comprises a gene encoding a functional Rep 52 protein and the gene encoding a functional Rep 52 protein comprises a nucleic acid sequence having at least 98% identity to the full length or a fragment of at least 1000 nucleotides in length of nucleotides 993-2186 of SEQ ID NO: 1, or to a corresponding stretch of nucleotides in a different serotype of AAV; and/or
   (ii)
      (a) the at least one AAV rep gene comprises a gene encoding a functional Rep 40 protein and the gene encoding a functional Rep 40 protein comprises a nucleic acid sequence having at least 98% identity to the full length or to a fragment of at least 800 nucleotides in length of a stretch of nucleotides corresponding to nucleotides 993-2252 minus nucleotides 1907-2227 of SEQ ID NO: 1, or to corresponding stretches of nucleotides in a different serotype of AAV; and/or
      (b) the at least one AAV rep gene comprises a gene encoding a functional Rep 40 protein and the gene encoding a functional Rep 40 protein comprises a nucleic acid sequence having at least 98% identity to the full length or to a fragment of at least 1100 nucleotides in length of nucleotides 993-2252 of SEQ ID NO: 1, or to a corresponding stretch of nucleotides in a different serotype of AAV; and/or
   (iii) the at least one AAV rep gene comprises a gene encoding a functional Rep 68 protein and the gene encoding a functional Rep 68 protein comprises a nucleic acid sequence having at least 98% identity to the full length or to a fragment of at least 1500 nucleotides in length of a stretch of nucleotides corresponding to nucleotides 321-2252 minus nucleotides 1907-2227 of SEQ ID NO: 1, or to corresponding stretches of nucleotides in a different serotype of AAV.
[6] The two-plasmid system or helper plasmid of any one of the preceding aspects, wherein:
   (i) the helper plasmid does not comprise a gene encoding a functional Rep 78 protein; and/or
   (ii) the helper plasmid does not comprise a contiguous sequence of at least 1800 nucleotides corresponding to a contiguous stretch of nucleotides of equivalent length comprised within nucleotides 321-2186 of SEQ ID NO: 1 or within a corresponding stretch of nucleotides in a different serotype of AAV; and/or
   (iii) the at least one AAV rep gene does not comprise a functional internal p40 promoter; and/or
   (iv) the helper plasmid does not comprise a contiguous stretch of exclusively cap gene sequence of more than 60 nucleotides.
[7] The two-plasmid system or helper plasmid of any one of the preceding aspects, wherein:
   (i) the helper plasmid comprises at least one helper virus gene and the at least one helper virus gene comprises a viral associated (VA) nucleic acid, an E2A gene and an E4 gene; and/or
   (ii) the helper plasmid comprises at least one helper virus gene and the at least one helper virus gene comprises a VA nucleic acid, an E2A gene and an E4 gene and the E4 gene is not located between the VA nucleic acid and the E2A gene; and/or
   (iii) the helper plasmid comprises at least one helper virus gene and the helper plasmid is between 12000 bp and 15000 bp in length; and/or
   (iv) the helper plasmid does not comprise a contiguous sequence of at least 3000 nucleotides of a contiguous stretch of nucleotides of equivalent length comprised within nucleotides 194-3620 of SEQ ID NO: 2, or a corresponding stretch of nucleotides in a different serotype of adenovirus; and/or
   (v) the helper plasmid does not comprise a contiguous sequence of at least 60 nucleotides of a contiguous stretch of nucleotides of equivalent length comprised within nucleotides 4032-4100 of SEQ ID NO: 2, or a corresponding stretch of nucleotides in a different serotype of adenovirus; and/or
   (vi) the helper plasmid does not comprise a contiguous sequence of at least 350 nucleotides of a contiguous stretch of nucleotides of equivalent length comprised within nucleotides 4051-4413 of SEQ ID NO: 1, or a corresponding stretch of nucleotides in a different serotype of AAV.
[8] The two-plasmid system or helper plasmid of any one of the preceding aspects, wherein:
   (i) the helper plasmid does not comprise a contiguous sequence of at least 600 nucleotides of a contiguous stretch of nucleotides of equivalent length comprised within nucleotides 2301-2947 of SEQ ID NO: 1, or a corresponding stretch of nucleotides in a different serotype of AAV; and/or
   (ii) the helper plasmid comprises at least one helper virus gene and the at least one helper virus gene comprises a VA nucleic acid, an E2A gene and an E4 gene, and the VA nucleic acid, the E2A gene and the E4 gene are comprised within a contiguous portion of fewer than 10000 nucleotides; and/or
   (iii) the helper plasmid does not comprise a contiguous sequence of at least 22000 nucleotides of a contiguous stretch of nucleotides of equivalent length comprised within nucleotides 10619-32756 of SEQ ID NO: 2, or a corresponding stretch of nucleotides in a different serotype of adenovirus; and/or
   (iv) the helper plasmid and/or the vector plasmid does not comprise an artificial Rep binding site.
[9] The two-plasmid system or helper plasmid of any one of the preceding aspects, wherein the helper plasmid comprises at least one helper virus gene and the at least one helper virus gene is comprised in a contiguous stretch of the plasmid having at least 98% identity to the full length or to a fragment of at least 8000 nucleotides in length of SEQ ID NO: 4.
[10] The two-plasmid system of any one of [2]-[9], wherein:
   (i) the vector plasmid comprises an at least one AAV cap gene promoter, which comprises an AAV p40 promoter, a p5 promoter, and a p19 promoter; and/or
   (ii) the vector plasmid does not comprise any dispensable translation initiation codons; and/or
   (iii) the vector plasmid comprises a promoter region comprising one or more promoters, and in the promoter region:
      (a) nucleotides corresponding to nucleotides 321-323 of SEQ ID NO: 1 are absent;
      (b) nucleotides corresponding to nucleotides 766-768 of SEQ ID NO: 1 are not ATG;
      (c) nucleotides corresponding to nucleotides 955-957 of SEQ ID NO: 1 are absent;
      (d) nucleotides corresponding to nucleotides 993-995 of SEQ ID NO: 1 are absent; and
      (e) nucleotides corresponding to nucleotides 1014-1016 of SEQ ID NO: 1 are absent; and/or
   (iv) the vector plasmid comprises a backbone less than 3000 nucleotides in length.
[11] The two plasmid system of [10(iii)(b)], wherein nucleotides corresponding to nucleotides 766-768 of SEQ ID NO: 1 are ATT.
[12] A method for producing a recombinant AAV preparation comprising:
   (a) obtaining the two-plasmid system, the helper plasmid or the vector plasmid as defined in any one of [1]-[11];
   (b) transfecting a host cell with the two-plasmid system, the helper plasmid or the vector plasmid as defined in any one of [1]-[11]; and
   (c) culturing the host cell under conditions suitable for recombinant AAV production.
[13] The method of [12]:
   (i) further comprising a step of harvesting the recombinant AAV to provide a recombinant AAV preparation; and/or
   (ii) wherein the recombinant AAV preparation comprises a low level of replication competent AAV (rcAAV); and/or
   (iii) wherein the level of rcAAV is measured to be less than 1 rcAAV in 10⁷ recombinant AAV, less than 1 rcAAV in 10⁹ recombinant AAV, or less than 1 rcAAV in 10¹⁰ recombinant AAV; and/or
   (iv) wherein the recombinant AAV preparation comprises a low level of rcAAV and the level of rcAAV is less than the level of rcAAV produced using an equivalent method except that the vector plasmid comprises both at least one AAV rep gene and at least one AAV cap gene; and/or
   (v) comprising a step of selecting a ratio of helper plasmid to vector plasmid wherein the ratio of helper plasmid to vector plasmid is selected or adjusted to a ratio that allows the user to obtain the desired ratio of full to total particles or the high or desired yield of recombinant AAV vector; and/or
   (vi) wherein the ratio of helper plasmid to vector plasmid is selected or adjusted to a ratio that achieves a maximum yield of recombinant AAV with the minimum yield of empty particles achievable at such maximum yield of recombinant AAV; and/or
   (vii) wherein the desired ratio of full to total particles is a ratio of full to total particles that is at least 20% or at least 30% of the ratio of full to total particles achieved using an equivalent method with a ratio of helper plasmid to vector plasmid of 1.8:1; and/or
   (viii) further comprising a step of purifying the recombinant AAV.

### Description of the figures

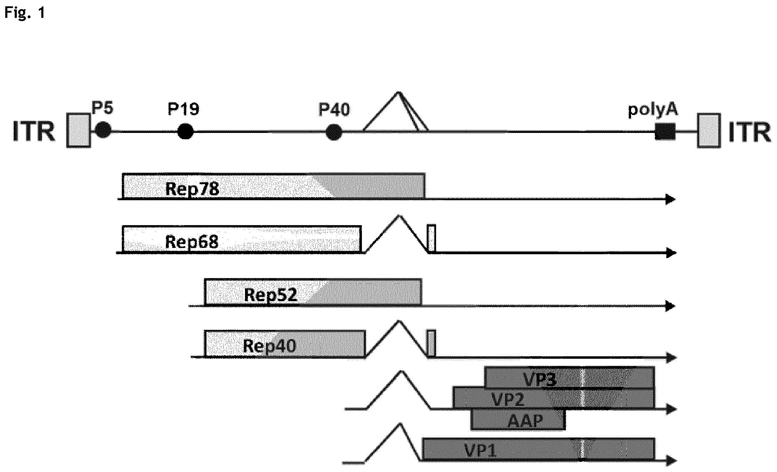

Figure 1 provides a schematic of native AAV genome showing overlapping Rep and Cap (VP1-3) transcripts and position of p5, p19 and p40 promoters. ITR = inverted terminal repeat.

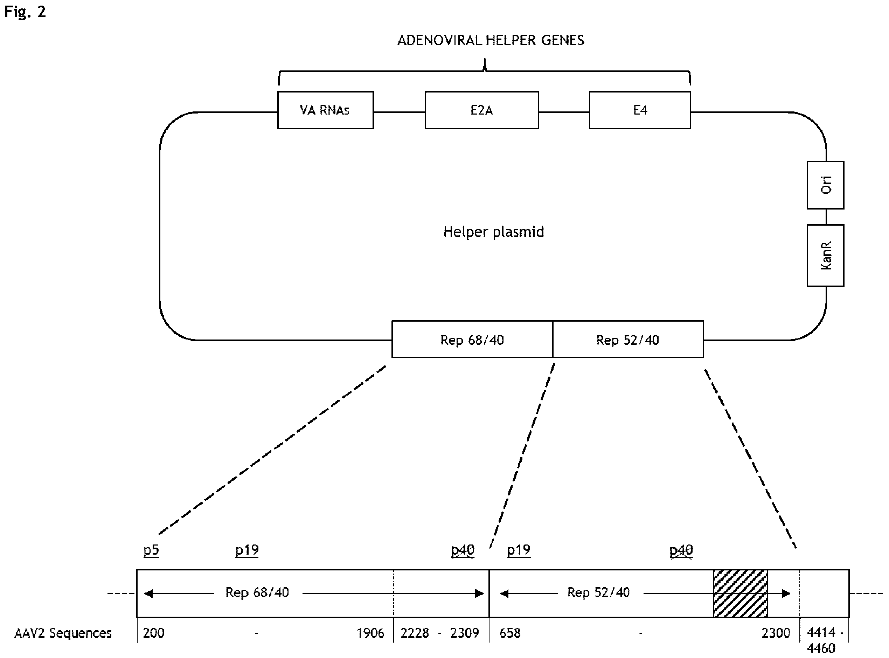

Figure 2 provides a schematic of helper plasmid constructed as described in Example 1, with inset showing rep genes including p5, p19 and p40 promoters. The crosses through "p40" indicate that these promoters have been rendered non-functional. Hatched area in the rep 52/40 gene indicates the presence of intron sequence which is spliced into rep 52 transcript but spliced out of rep 40 transcript. Ori = bacterial origin of replication. KanR = kanamycin resistance gene. Note the respective plasmid features are not shown to scale.

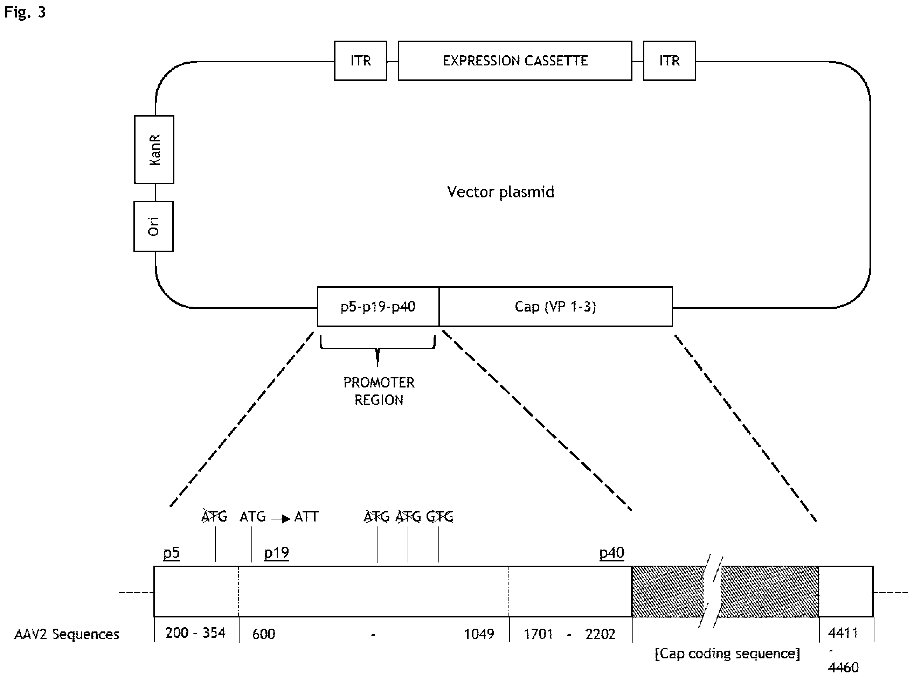

Figure 3 provides a schematic of vector plasmid constructed as described in Example 1, with inset showing cap gene and upstream promoter region including p5, p19 and p40 promoters. The crosses through "ATG" and "GTG" indicate that these potential translation initiation codons have been deleted. Ori = bacterial origin of replication. KanR = kanamycin resistance gene. ITR = inverted terminal repeat. Note the respective plasmid features are not shown to scale.

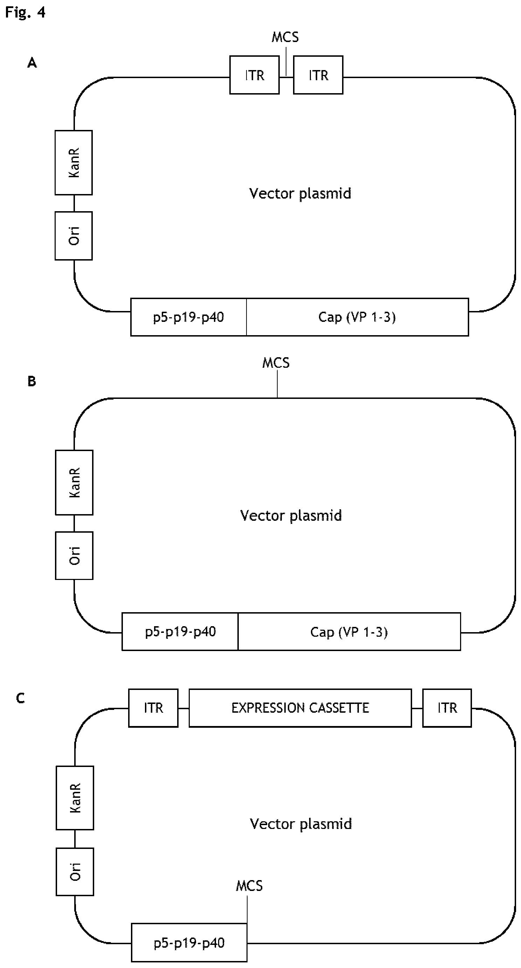

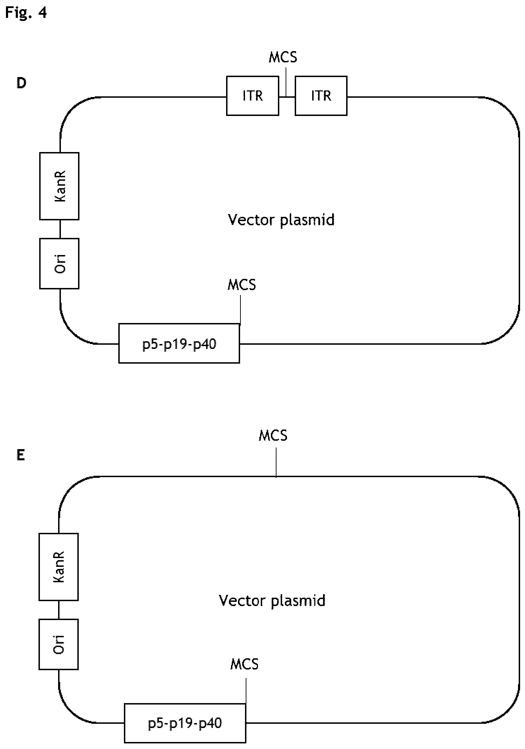

Figure 4 provides schematics showing vector plasmids: (A) containing a cap gene, and a multiple cloning site, flanked by ITRs, for cloning-in an expression cassette; (B) containing a cap gene, and a multiple cloning site for cloning-in an ITR-flanked expression cassette; (C) containing an expression cassette, flanked by ITRs, and a multiple cloning site, downstream of a promoter region containing p5, p19 and p40 promoters, for cloning-in a cap gene; (D) containing a multiple cloning site, flanked by ITRs, for cloning-in an expression cassette, and another multiple cloning site, downstream of a promoter region containing p5, p19 and p40 promoters, for cloning-in a cap gene; (E) containing a multiple cloning site for cloning-in an ITR-flanked expression cassette, and another multiple cloning site, downstream of a promoter region containing p5, p19 and p40 promoters, for cloning-in a cap gene.

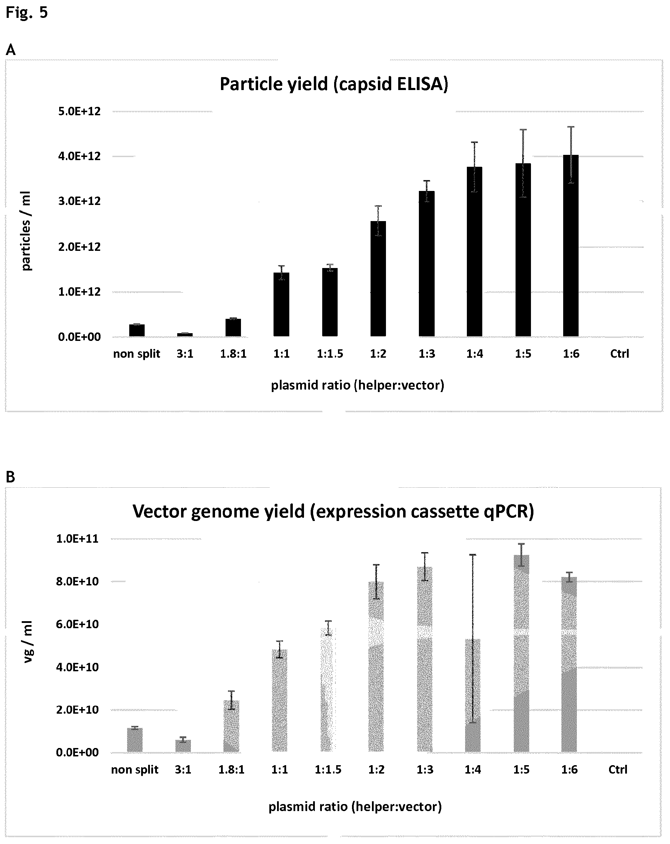

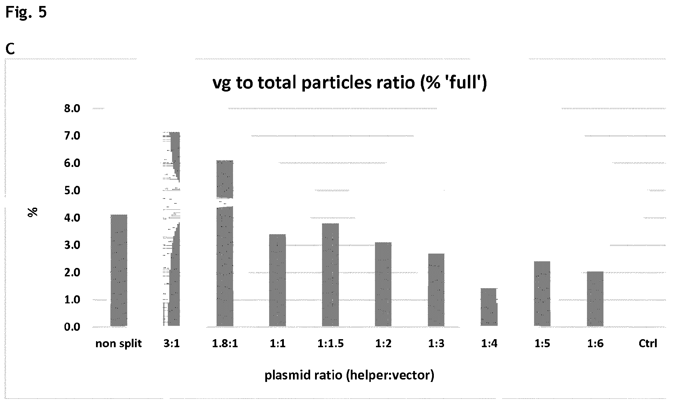

Figure 5 provides the results of analysis of rAAV produced and assayed as described in Example 2. (A) Particle titre per ml as measured by anti-capsid ELISA in rAAV produced using the two-plasmid system at varying helper:vector plasmid ratios at constant levels of total plasmid DNA. 'non-split' = two-plasmid system with Rep and Cap functions on same plasmid, used at plasmid ratio AdV helper-expression cassette:rep-cap 1.6:1. 'Ctrl' = negative control: helper plasmid transfected with pUC19 instead of vector plasmid. (B) Vector genome (vg) titre per ml as measured by qPCR amplifying a sequence within the promoter of the expression cassette, in the rAAV samples of (A). (C) Ratio of vg (from B) to total particle (from A) ratio, expressed as '% full', i.e. number of vg as a %age of number of particles. Note: 1.0E+12 = 1.0×10¹². Error bars show standard deviation from triplicate analysis of samples.

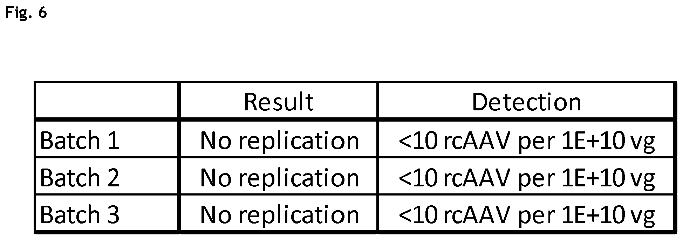

Figure 6 shows rcAAV quantification in different rAAV batches. Three different batches of rAAV containing the same (Factor IX encoding) transgene cassette were analysed for the rcAAV content.

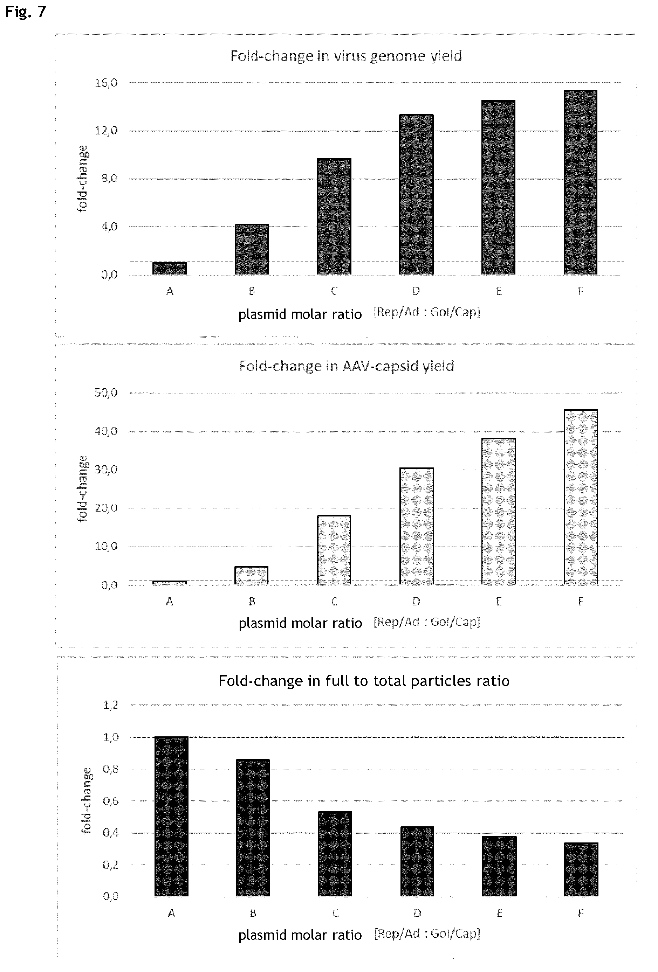

Figure 7 shows modulation of yields and full to total particles ratio by plasmid ratio modification. Six different plasmid ratios were tested for the two-plasmid system in rAAV production. The plasmid molar ratios of A to F are 3:1, 1.8:1, 1:1.5, 1:2, 1:3 and 1:5, respectively. Virus genome yields were determined by transgene cassette-specific qPCR. Capsid yields were determined by Capsid-specific ELISA. The full to total particles ratios were calculated based on the qPCR and ELISA results.

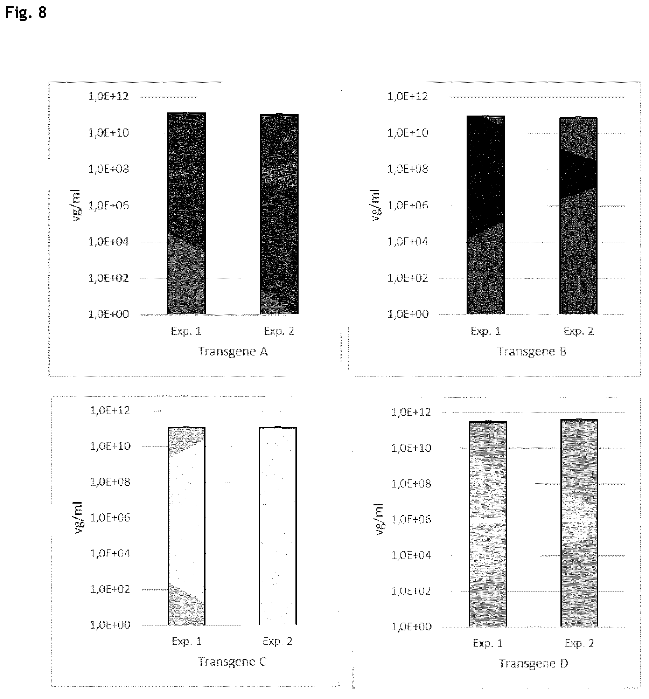

Figure 8 shows virus genome yields for four different transgenes in the two-plasmid packaging system. Four different transgenes were used for rAAV packaging. Two independent packaging experiments were performed for each transgene. Yields were quantified using transgene cassette-specific qPCRs.

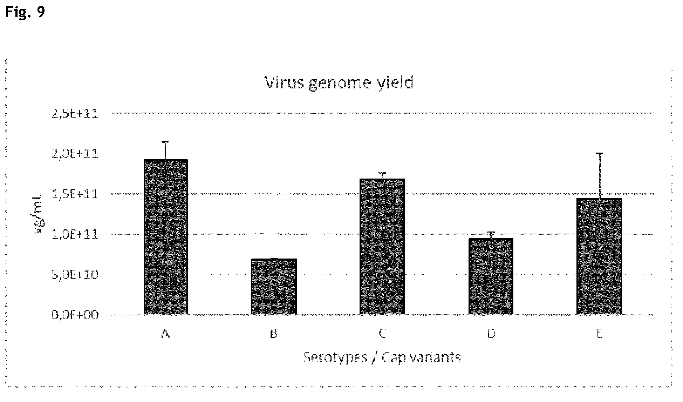

Figure 9 shows production of rAAV applying different cap serotypes or synthetic cap variants. rAAV was generated applying the identical molar plasmid ratio, five different cap serotypes / synthetic cap variants and the same transgene cassette. Virus genome yields were determined by transgene-cassette specific qPCR.

### Detailed description

### General Definitions

Unless defined otherwise, technical and scientific terms used herein have the same meaning as commonly understood by a person skilled in the art to which this invention belongs.

In general, the term *"comprising"* is intended to mean including but not limited to. For example, the phrase "*a helper plasmid comprising at least one rep gene*" should be interpreted to mean that the helper plasmid has at least one rep gene, but the helper plasmid may comprise further components such as further genes.

In some embodiments of the invention, the word *"comprising"* is replaced with the phrase *"consisting of"* or the phrase *"consisting essentially of".* The term *"consisting of"* is intended to be limiting. For example, the phrase *"a helper plasmid consisting of one rep gene"* should be understood to mean that the helper plasmid has one rep gene and no other genetic material. Similarly, the phrase *"a helper plasmid consisting essentially of one rep gene"* should be understood to mean that the helper plasmid has one rep gene and comprises no additional components that materially affect the function of the helper plasmid. For example a helper plasmid consisting essentially of a rep gene will not contain any other genes but could contain other genetic material such as spacers.

The terms *"protein"* and *"polypeptide"* are used interchangeably herein, and are intended to refer to a polymeric chain of amino acids of any length.

For the purpose of this invention, in order to determine the percent identity of two sequences (such as two polynucleotide or two polypeptide sequences), the sequences are aligned for optimal comparison purposes (*e.g.,* gaps can be introduced in a first sequence for optimal alignment with a second sequence). The nucleotides or amino acids at each position are then compared. When a position in the first sequence is occupied by the same amino acid or nucleotide as the corresponding position in the second sequence, then the amino acids or nucleotides are identical at that position. The percent identity between the two sequences is a function of the number of identical positions shared by the sequences (*i.e.,* % identity = number of identical positions /total number of positions in the reference sequence x 100).

Typically the sequence comparison is carried out over the length of the reference sequence. For example, if the user wished to determine whether a given (*"test*") sequence is 95% identical to SEQ ID NO: 1, SEQ ID NO: 1 would be the reference sequence. To assess whether a sequence is at least 80% identical to SEQ ID NO: 1 (an example of a reference sequence), the skilled person would carry out an alignment over the length of SEQ ID NO: 1, and identify how many positions in the test sequence were identical to those of SEQ ID NO: 1. If at least 80% of the positions are identical, the test sequence is at least 80% identical to SEQ ID NO: 1. If the sequence is shorter than SEQ ID NO: 1, the gaps or missing positions should be considered to be non-identical positions.

The skilled person is aware of different computer programs that are available to determine the homology or identity between two sequences. For instance, a comparison of sequences and determination of percent identity between two sequences can be accomplished using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6.

Herein, the term *"plasmid"* is intended to refer to a nucleic acid molecule that can replicate independently of a cell chromosome. The term *"plasmid"* is intended to cover circular nucleic acid molecules and linear nucleic acid molecules. Furthermore, the term *"plasmid"* is intended to cover bacterial plasmids, but also cosmids, minicircles (Nehlsen, K., Broll S., Bode, J. (2006), Gene Ther. Mol. Biol., 10: 233-244; Kay, M.A., He, C.-Y, Chen, Z.-H. (2010), Nature Biotechnology, 28: 1287-1289) and ministrings (Nafissi N, Alqawlaq S, Lee EA, Foldvari M, Spagnuolo PA, Slavcev RA. (2014), Mol Ther Nucleic Acids, 3:e165). Optionally, the plasmid is a circular nucleic acid molecule. Optionally, the plasmid is a nucleic acid molecule that is of bacterial origin.

The term *"helper"* is not intended to be limiting. Accordingly, a *"helper plasmid"* is any plasmid that:
(i) comprises at least one rep gene encoding at least one functional Rep protein and does not comprise a cap gene encoding a functional Cap protein; or
(ii) comprises at least one helper virus gene and does not comprise a cap gene encoding a functional Cap protein, and the at least one helper virus gene is comprised in a contiguous stretch of the plasmid having at least 95%, at least 98%, at least 99%, or 100% identity to the full length or to a fragment at least 6000, at least 7000, or at least 8000 nucleotides in length of SEQ ID NO: 4.

The term *"vector plasmid"* is not intended to be limiting. Accordingly, a *"vector plasmid"* is any plasmid that:
(i) is suitable for use alongside a helper plasmid in a two-plasmid system of the invention; or
(ii) comprises:
   (a) a cap gene encoding at least one functional Cap protein; or
   (b) at least one cap gene promoter, a cloning site operably linked to the cap gene promoter, and an expression cassette flanked on at least one side by an ITR;
   wherein the vector plasmid does not comprise a rep gene encoding a functional Rep protein and the expression cassette comprises a transgene operably linked to at least one regulatory control element.

The term *"nucleic acid molecule"* refers to a polymeric form of nucleotides of any length. The nucleotides may be deoxyribonucleotides, ribonucleotides or analogs thereof. Preferably, the plasmid is made up of deoxyribonucleotides or ribonucleotides. Even more preferably, the plasmid is made up of deoxyribonucleotides, *i.e.* the plasmid is a DNA molecule.

The terms *"wild type"* and *"native"* are synonymous and refer to genes present in the genome of a strain/serotype of AAV or adenovirus, or to proteins encoded by genes present in the genome of a strain/serotype of AAV or adenovirus.

### AAV production assay

In an AAV production assay, the user can determine whether a given *"test"* plasmid or two-plasmid system is effective to produce rAAV at a similar level to a *"reference"* plasmid or two-plasmid system as follows.

The user provides a *"reference* " two-plasmid system that comprises a *"reference helper plasmid"* and a *"reference vector plasmid".*

For example, the user provides a reference helper plasmid comprising wild type Adenovirus 5 helper genes encoding E2A, E4 and VA RNA I and II, *i.e.* the adenovirus helper genes comprised within SEQ ID NO: 2. Details of the nucleic acid positions in SEQ ID NO: 2 which encode these genes are set out in more detail below under the heading *"at least one virus helper gene".* The helper plasmid also comprises a wild type rep gene encoding Rep 40, Rep 52, Rep 68 and Rep 78 and the rep promoters p5, p19 and p40, *i.e.* the sequences comprised within nucleotides 200-2252 of SEQ ID NO: 1.

The user provides a reference vector plasmid comprising a wild type cap gene operably linked to a wild type cap gene promoter comprising p5, p19 and p40, *i.e.* the cap gene comprised within SEQ ID NO: 1 (nucleotides 5961-8171 of SEQ ID NO: 1). The vector plasmid further comprises a transgene flanked by two AAV2 ITRs, *i.e.* the ITRs comprised within nucleotides 1-145 and 4535-4679 of SEQ ID NO: 1.

The user then provides a *"test"* two-plasmid system comprising a *"test helper plasmid"* and a *"test vector plasmid"* that is based on the reference two-plasmid system, but has a single change relating to a characteristic that the users wishes to test. For example, if the user wishes to see whether a given rep protein was functional, the user could swap out the rep gene of the *"reference helper plasmid"* and replace it with the test rep protein to provide a *"test helper plasmid".*

The user then compares the ability of the reference two-plasmid system and the test two-plasmid system to allow for production of rAAV. To do this, the user can transfect a first set of suitable host cells (such as HEK293T cells which express the E1A/B gene so that the helper plasmid does not need to comprise an E1A/B gene) with the reference two-plasmid system, and a second set of identical host cells with the test two-plasmid system and incubate the host cells for a period of time suitable for the AAV production to occur. The yield of AAV recombinant produced from the reference two-plasmid system and the test two-plasmid system may then be harvested and measured using qPCR to quantify the number of vector genomes. For example, qPCR may be used to determine the number of instances of nucleic acid molecules comprising a component of the vector genome, such as a promoter sequence, that are produced in the host cells transfected with the test two-plasmid system compared to the host cells transfected with the reference two-plasmid system. Alternatively, the comparative yield of particles may be determined, for example by an anti-capsid ELISA.

A suitable AAV production assay is disclosed in Example 2.

### A two-plasmid system

The present invention provides a two-plasmid system comprising a helper plasmid and a vector plasmid, wherein the helper plasmid comprises at least one AAV rep gene encoding at least one functional AAV Rep protein and at least one helper virus gene, and which does not comprise a cap gene encoding a functional Cap protein.

The two-plasmid system is useful for producing rAAV. Optionally, the two-plasmid system is suitable for use in producing rAAV. Optionally, the two-plasmid system is for producing rAAV. Optionally, the two-plasmid system is for producing rAAV suitable for use in gene therapy. Optionally, the two-plasmid system is for producing rAAV for use in gene therapy.

The phrase *"two-plasmid system"* refers to a system that comprises two plasmids, and can be used without the need for additional plasmids to produce rAAV. Optionally, the two-plasmid system can be used to produce rAAV without the need for helper virus such as adenovirus. Optionally, the two-plasmid system can be used to produce rAAV without the need for genetic material originating from a host cell, optionally with the exception of a gene encoding E1A/B. However, the system may comprise additional non-plasmid components. Optionally, the two-plasmid system does not comprise a helper virus. Optionally, the two-plasmid system of the invention comprises all the necessary genetic information for the production of rAAV. For example, the two-plasmid system of the invention may comprise at least one AAV rep gene, at least one AAV cap gene and at least one helper gene. Optionally, the two-plasmid system of the invention comprises all the necessary genetic information required for the production of rAAV suitable for use in gene therapy. For example, the two-plasmid system of the invention may comprise at least one AAV rep gene, at least one AAV cap gene, at least one helper gene and an expression cassette comprising a transgene operably linked to at least one regulatory control element. However, in embodiments the two-plasmid system of the invention may lack a functional cap gene (required for the production of rAAV) and/or an expression cassette comprising a transgene operably linked to at least one regulatory control element (required for the production of rAAV suitable for use in gene therapy).

It is an advantage of the present invention that an AAV cap gene and/or a transgene in the vector plasmid may be exchanged with another in order to treat different genetic disorders. Optionally, therefore, the two-plasmid system of the invention may comprise all the necessary genetic information for the production of rAAV except a functional cap gene, and in such embodiments the two-plasmid system of the invention may comprise a site suitable for cloning in an AAV cap gene. Such a site may comprise a cloning site adjacent to an AAV cap gene promoter. The site suitable for cloning in an AAV cap gene will be present on the vector plasmid. Optionally, the two-plasmid system of the invention comprises all the necessary genetic information for the production of rAAV suitable for use in gene therapy except a functional cap gene and an expression cassette comprising a transgene and a regulatory control element, wherein said two-plasmid system comprises a site suitable for cloning in an AAV cap gene (such as an AAV cap gene promoter and a cloning site operably linked, *i.e.* adjacent, to the AAV cap gene promoter) and a site suitable for cloning in an expression cassette (such as a cloning site flanked by one or more ITRs). Optionally, the two-plasmid system of the invention comprises all the necessary genetic information for the production of rAAV suitable for use in gene therapy except an expression cassette comprising a transgene and a regulatory control element, wherein said two-plasmid system comprises a site suitable for cloning in an expression cassette (such as a cloning site flanked by one or more ITRs). In such cases the site suitable for cloning in an AAV cap gene and the site suitable for cloning in an expression cassette will be present on the vector plasmid. Optionally, the two-plasmid system of the invention comprises the following components split between the vector plasmid and the helper plasmid:
- at least one AAV rep gene encoding at least one functional AAV Rep protein;
- at least one helper virus gene;
- an AAV cap gene encoding at least one functional AAV capsid protein, or an AAV cap gene promoter and a cloning site operably linked to the AAV cap gene promoter;
- at least one ITR; and
- an expression cassette comprising a transgene operably linked to at least one regulatory control element, or a site suitable for cloning in an expression cassette flanked on at least one side by *(i.e.* adjacent to) an ITR.

The vector plasmid may comprise:
(a) an AAV cap gene encoding at least one functional AAV Cap protein; or
(b) at least one AAV cap gene promoter, a cloning site operably linked to the AAV cap gene promoter, and an expression cassette flanked on at least one side by an ITR;
wherein the vector plasmid does not comprise a rep gene encoding a functional Rep protein and the expression cassette comprises a transgene operably linked to at least one regulatory control element.

The helper plasmid comprises at least one AAV rep gene encoding at least one functional AAV Rep protein and at least one helper virus gene, and does not comprise a cap gene encoding a functional Cap protein.

Optionally, the ratio of helper plasmid to vector plasmid is between 3:1 to 1:10, between 1.5:1 and 1:9, between 1.4:1 and 1:8, between 1.3:1 and 1:7; between 1.2:1 and 1:6; between 1.1:1 and 1:5; between 1:1 and 1:4; or between 1:1.5 and 1:3. Optionally, the two-plasmid system comprises a molar excess of vector plasmid compared to helper plasmid.

As set out in more detail below, altering the ratio of helper plasmid to vector plasmid can be used to control the ratio of full to total particles (or proportion of capsids/particles that are full) produced during rAAV production, or to increase the yield of rAAV produced during rAAV production.

By the phrase *"ratio of helper plasmid to vector plasmid",* is meant a molar ratio, *i.e.* the ratio of the number of moles of helper plasmid present to the number of moles of vector plasmid present. A given ratio of helper plasmid to vector plasmid may be provided by simply mixing the helper plasmid and the vector plasmid in an appropriate molar ratio.

### A helper plasmid

The present invention provides a helper plasmid comprising at least one AAV rep gene encoding at least one functional AAV Rep protein and at least one helper virus gene, and which does not comprise a cap gene encoding a functional Cap protein.

The present invention relates to an improved two-plasmid system that is suitable for producing rAAV. A helper plasmid that comprises at least one AAV rep gene encoding at least one functional AAV Rep protein and does not comprise a cap gene encoding a functional Cap protein may be advantageous for use in such an improved two-plasmid system. Both rep genes and cap genes are required in order to produce rAAV. However, the plasmids and two-plasmid systems of the invention are arranged such that the rep and cap genes are not both present on a single plasmid ('trans-split' configuration). Ensuring that the helper plasmid does not comprise both a rep gene and a cap gene is difficult to do, as the native AAV cap and rep genes are overlapping and so separating them whilst maintaining sufficient genetic material to allow for production of the complete protein is difficult. However, splitting the rep genes and the cap genes between a helper plasmid and a vector plasmid is advantageous, as it increases the number of recombination events required to form rcAAV.

AAV can only propagate in the presence of a helper virus, which encodes proteins that aid in AAV propagation. However, growing AAV in the presence of a helper virus is not advantageous as helper viruses can be lytic to cells, including host cells used to grow AAV. Furthermore, if helper viruses are used in the production of rAAV products, such as rAAV for use in gene therapy, the helper virus may contaminate the product. As an alternative to co-infecting with helper virus such as adenovirus, the requisite genes of the helper virus can be provided on a transfected plasmid. For host cells expressing the adenoviral E1A/B genes (such as HEK293T cells) the remaining required adenoviral helper genes encode E4, E2A and VA RNA I andII. While these genes are distributed across a long stretch of the adenoviral genome, the present inventors have determined that large stretches of non-coding nucleotides separating the genomic genes can be removed without affecting expression of the genes. The inventors have, therefore, designed a minimal helper gene region, which is SEQ ID NO: 4. Using such a minimal helper gene region in a plasmid for the production of rAAV is advantageous as it allows the user to use a smaller plasmid which is less costly to produce and easier to transfect into the cell.

### At least one AAV rep gene

The helper plasmid comprises at least one AAV rep gene encoding at least one functional AAV Rep protein. AAV comprises an AAV rep gene region which encodes four AAV Rep proteins (Rep 78, Rep 68, Rep 52 and Rep 40). The gene region is under the control of the p5 and p19 promoters. When the p5 promoter is used, a gene that encodes Rep 78 and Rep 68 is transcribed. Rep 78 and Rep 68 are two alternative splice variants (Rep 78 comprises an intron that is excised in Rep 68). Similarly, when the p19 promoter is used, a gene that encodes Rep 52 and Rep 40 is transcribed. Rep 52 and Rep 40 are alternative splice variants (Rep 52 comprises an intron that is excised in Rep 40).

The four AAV Rep proteins are known to be involved in replication and packaging of the viral genome, and are, therefore, useful in rAAV production.

It is not necessary for all four AAV Rep proteins to be present. Optionally, however, the at least one AAV rep gene encodes a large Rep protein (Rep 78 or Rep 68) and a small AAV Rep protein (Rep 52 or Rep 40). Rep 78 can be toxic to cells, and Rep 78 does not need to be present in order for AAV replication to take place. In embodiments where the at least one AAV rep gene does not encode Rep 78, the at least one AAV rep gene preferably does encode Rep 68. Accordingly, the helper plasmid may comprise at least one AAV rep gene encoding:
(a) a functional Rep 52 protein;
(b) a functional Rep 40 protein; and/or
(c) a functional Rep 68 protein.

A *"functional"* AAV Rep protein is one which allows for production of AAV particles. In particular, Rep 78 or Rep 68 (the large AAV Rep proteins) are believed to be involved in replication of the AAV genome, and Rep 52 and Rep 40 (the small AAV Rep proteins) are believed to be involved in packaging of the AAV genome into a capsid. It is within the abilities of the skilled person to determine whether a given AAV Rep protein is functional. The skilled person merely needs to determine whether the AAV Rep protein supports AAV production using an AAV production assay as described above. In this case, the test two-plasmid system will comprise a helper plasmid that comprises the AAV Rep protein whose *"functionality"* is to be determined, and otherwise the test two-plasmid system used will be identical to the reference two-plasmid system.

In an embodiment, the at least one AAV rep gene of the helper plasmid encodes a *"functional"* AAV Rep protein. If the AAV Rep protein supports rAAV production at a level at least 25%, at least 40%, at least 50%, at least 70%, at least 80%, at least 90% or at least 95% of the level supported by the wild type AAV Rep protein, *i.e.* if the yield of rAAV vector genomes produced is at least 25%, at least 40%, at least 50%, at least 70%, at least 80%, at least 90% or at least 95% of the yield of rAAV vector genomes produced using the reference two-plasmid system. Preferably, the at least one AAV rep gene of the helper plasmid is functional if it supports rAAV production at a level at least 80% of the level supported by the wild type AAV Rep protein.

In general, an AAV Rep protein will only be able to support rAAV production if it is compatible with the ITR(s) surrounding the genome of the AAV to be packaged. Some AAV Rep proteins may only be able to package genomic material (such as an expression cassette) when it is flanked by ITR(s) of the same serotype as the AAV Rep protein. Other AAV Rep proteins are cross-compatible, meaning that they can package genomic material that is flanked by ITR(s) of a different serotype. For example, in the case of a two-plasmid system, it is preferred that the AAV Rep protein is able to support replication and packaging of an expression cassette comprised within the vector plasmid, and such an AAV Rep protein will be compatible with the at least one ITR flanking the expression cassette *(i.e.* able to replicate and package the expression cassette flanked on at least one side by an ITR).

Optionally, the at least one AAV rep gene comprises a gene encoding a functional Rep 52 protein, at least one gene encoding a functional Rep 40 protein, and a gene encoding a functional Rep 68 protein.

The helper plasmid may comprise two genes encoding a functional Rep 40 protein. In an embodiment, the at least one AAV rep gene comprises two genes encoding a functional Rep 40 protein. For example, the helper plasmid may comprise two AAV rep genes that are separated on the plasmid. The first of the two separate AAV rep genes could encode Rep 68 (for example using the p5 promoter or a different promoter situated near the normal position of the p5 promoter in the AAV rep gene) and Rep 40 (for example using the p19 promoter or a different promoter situated near the normal position of the p19 promoter). The second of the two separate AAV rep genes could encode Rep 52 and Rep 40. Rep 52 and Rep 40 are alternative splice variants.

If the helper plasmid comprises two genes encoding a Rep 40 protein, one of the two genes that encodes a functional Rep 40 protein may comprise an intron. In one embodiment, both genes that encode a functional Rep 40 protein comprise an intron. However, in a preferred embodiment, only one of the genes that encodes a functional Rep 40 protein comprises an intron. For example, if the user wishes to avoid the at least one AAV rep gene encoding Rep 78, the AAV rep gene may be split through partial duplication into two genes. One gene could comprise nucleotides corresponding to the full length native AAV rep gene with the sequence corresponding to the intron removed. Such a gene would encode Rep 68 and Rep 40, but would not encode either Rep 78 or Rep 52, as a portion of each of the Rep 78 and Rep 52 proteins is encoded by the sequence which acts as an intron in the context of Rep 40. The second gene could comprise nucleotides corresponding to the region of the native AAV rep gene downstream of the p19 promoter, which would encode Rep 52 (intron spliced in) and Rep 40 (intron spliced out).

SEQ ID NO: 1 provides the sequence of the genome of wild type AAV2, and nucleotides 321-2252 of SEQ ID NO: 1 encode the four AAV Rep proteins. The full length AAV rep gene (nucleotides 321-2252) encodes all four AAV Rep proteins (Rep 78 and Rep 68 from the p5 promoter and Rep 52 and Rep 40 from the p19 promoter). A shorter stretch of the AAV rep gene downstream of the p19 promoter (nucleotides 993-2252) encodes Rep 52 and Rep 40 only *(i.e.* this stretch of the AAV rep gene reaches from the end of the p19 promoter to the end of the gene). Nucleotides 1907-2227 of SEQ ID NO: 1 correspond to an intron. Rep 78 and Rep 52 comprise amino acids encoded by the intron, but Rep 68 and Rep 40 are alternative splice variants that do not comprise amino acids encoded by the intron. The relationship between the AAV rep gene and the four AAV Rep proteins is shown in Figure 1.

Optionally, the two-plasmid system or helper plasmid comprises a gene encoding a functional Rep 52 protein, and the gene encoding a functional Rep 52 protein comprises a nucleic acid sequence having at least 95%, at least 98%, at least 99%, or 100% identity to the full length or a fragment of at least 800, at least 900, at least 1000, or at least 1100 nucleotides in length of nucleotides 993-2186 of SEQ ID NO: 1, or to a corresponding stretch of nucleotides in a different serotype of AAV.

It is within the abilities of the person skilled in the art to determine whether a particular (test) stretch of nucleotides is a *"corresponding stretch of nucleotides in a different serotype of"* AAV. All that is required is that the person skilled in the art align the test stretch of nucleotides with the genome of the reference serotype *(i.e.* SEQ ID NO: 1). If the test stretch of nucleotides has greater than 90% identity with a contiguous stretch of nucleotides of the same length in SEQ ID NO: 1, the contiguous stretch is a corresponding stretch of nucleotides in a different serotype of AAV. The same applies in the case of adenovirus sequences (except here the reference serotype is SEQ ID NO: 2).

Optionally, the at least one AAV rep gene comprises a gene encoding a functional Rep 40 protein, and the gene encoding a functional Rep 40 protein comprises a nucleic acid sequence having at least 95%, at least 98%, at least 99%, or 100% identity to the full length or to a fragment of at least 600, at least 700, at least 800, or at least 900 nucleotides in length of a stretch of nucleotides corresponding to nucleotides 993-2252 minus nucleotides 1907-2227 of SEQ ID NO: 1, or to corresponding stretches of nucleotides in a different serotype of AAV. Hence, such a Rep 40-encoding gene has at least the above-specified identity to a notional stretch of nucleotides consisting of nucleotides 993-1906 of SEQ ID NO: 1 immediately juxtaposed with nucleotides 2228-2252 of SEQ ID NO: 1 (5'-[993-1906]-[2228-2252]-3') or to a notional stretch of nucleotides from a different AAV serotype.

Optionally, the at least one AAV rep gene comprises at least one gene encoding a functional Rep 40 protein, and the gene encoding a functional Rep 40 protein comprises a nucleic acid sequence having at least 95%, at least 98%, at least 99%, or 100% identity to the full length or to a fragment of at least 900, at least 1000, at least 1100, or at least 1200 nucleotides in length of nucleotides 993-2252 of SEQ ID NO: 1, or to a corresponding stretch of nucleotides in a different serotype of AAV.

Optionally, the at least one AAV rep gene comprises a gene encoding a functional Rep 68 protein, and the gene encoding a functional Rep 68 protein comprises a nucleic acid sequence having at least 95%, at least 98%, at least 99%, or 100% identity to the full length or to a fragment of at least 1000, at least 1400, at least 1500, or at least 1600 nucleotides in length of a stretch of nucleotides corresponding to nucleotides 321-2252 minus nucleotides 1907-2227 of SEQ ID NO: 1, or to corresponding stretches of nucleotides in a different serotype of AAV. Hence, such a Rep 68-encoding gene has at least the above-specified identity to a notional stretch of nucleotides consisting of nucleotides 321-1906 of SEQ ID NO: 1 immediately juxtaposed with nucleotides 2228-2252 of SEQ ID NO: 1 (5'-[321-1906]-[2228-2252]-3'), or to a notional stretch of nucleotides from a different AAV serotype.

Since Rep 68 does not comprise any amino acids encoded by the intron (nucleotides 1907-2227), the gene encoding a functional Rep 68 protein does not need to comprise nucleotides corresponding to nucleotides 1907-2227. Indeed, excluding nucleotides 1907-2227 from the at least one AAV rep gene ensures that Rep 78 is not encoded (as Rep 78 comprises amino acids encoded by the intron). Preferably the helper plasmid does not comprise a gene encoding a functional Rep 78 protein.

Optionally, the at least one AAV rep gene comprises a gene encoding functional Rep 68 and Rep 40 proteins, wherein said gene comprises a nucleic acid having at least 95%, at least 98%, at least 99%, or 100% identity to the full length or to a fragment of at least 1400, 1500, 1600 or 1700 nucleotides in length of the following stretches of native AAV2 sequence (SEQ ID NO: 1) positioned in immediate juxtaposition from 5' to 3': 200-1906; 2228-2309, or to corresponding juxtaposed stretches of nucleotides from a different serotype of AAV.

Optionally, the at least one AAV rep gene comprises a gene encoding functional Rep 52 and Rep 40 proteins, wherein said gene comprises a nucleic acid having at least 95%, at least 98%, at least 99%, or 100% identity to the full length or to a fragment of at least 1300, 1400, 1500 or 1600 nucleotides in length of the following stretch of native AAV2 sequence (SEQ ID NO: 1): 658-2300, or to a corresponding stretch of nucleotides from a different serotype of AAV.

Optionally, the at least one AAV rep gene comprises a stretch of nucleotides encoding functional Rep 68, Rep 52 and Rep 40 proteins, wherein said stretch comprises a nucleic acid having at least 95%, at least 98%, at least 99%, or 100% identity to the full length or to a fragment of at least 3000, 3200, 3300 or 3400 nucleotides in length of the following stretches of native AAV2 sequence (SEQ ID NO: 1) positioned in immediate juxtaposition from 5' to 3': 200-1906; 2228-2309; 658-2300, or to corresponding juxtaposed stretches of nucleotides from a different serotype of AAV.

Optionally, the two-plasmid system or helper plasmid does not comprise a contiguous sequence of at least 1700, at least 1800, or 1866 nucleotides corresponding to a contiguous stretch of nucleotides of equivalent length comprised within nucleotides 321-2186 of SEQ ID NO: 1, or within a corresponding stretch of nucleotides in a different serotype of AAV. The contiguous stretch of nucleotides comprised within nucleotides 321-2186 corresponds to Rep 78.

In some embodiments, the at least one rep gene does not comprise a functional internal p40 promoter. The native rep/cap gene comprises a p40 promoter (D.J. Pereira and N. Muzyczka (1997), J. Virol. 71:1747-1756). The p40 promoter drives expression of the cap gene, but is not required for expression of rep genes. A functional p40 promoter is one that is capable to drive expression of the cap gene.

Whether or not a given p40 promoter is functional may be determined by testing its ability to drive expression of a protein (using an expression assay). For example, user may prepare a *"reference"* vector comprising a native p40 promoter (such as that of nucleotides 1710-1827 of SEQ ID NO: 1) upstream of a reporter protein such as GFP. The user may then prepare a *"test"* vector that is identical to the reference vector except that the native p40 promoter is replaced by the p40 promoter whose functionality is to be tested (the *"test"* p40 promoter). The two vectors may be transfected into suitable host cells, and the host cells incubated under conditions suitable for expression of the reporter protein. The level of reporter protein expressed in the host cells could be measured by, for example, fluorescence spectroscopy. The level of expression from the reference vector (corresponding to the level of expression driven by the native p40 promoter) can then be compared to the level of expression from the test vector (corresponding to the level of expression driven by the test promoter).

A p40 promoter will be considered to be functional if its drives expression at a level at least 40% of the expression driven by the native p40 promoter. Optionally, a functional p40 promoter drives expression at a level at least 60%, at least 70%, at least 80%, at least 90%, or at least 95% of the expression driven by the native p40 promoter. Optionally the at least one AAV rep gene does not comprise a functional internal p40 promoter if it does not comprise a sequence corresponding to the p40 promoter (for example nucleotides 1710-1827 of SEQ ID NO: 1) that drives expression at a level at least 60%, at least 70%, at least 80%, at least 90%, or at least 95% of the expression driven by the native p40 promoter.

P40 promoters lacking a *"TATA box"* are non-functional. The p40 promoter comprises a TATA box starting at a position corresponding to position 1823 of SEQ ID NO: 1. Thus, the helper plasmid that does not comprise a functional internal p40 promoter may comprise one or more p40 promoters in which the TATA boxes are mutated to render the p40 promoter non-functional. Optionally, the at least one AAV rep gene does not comprise a T nucleotide at a position corresponding to position 1823 of SEQ ID NO: 1. Optionally, the at least one AAV rep gene comprises a C nucleotide at a position corresponding to position 1823 of SEQ ID NO: 1. Optionally, the at least one AAV rep gene does not comprise AAG at positions corresponding to positions 1826-1828 of SEQ ID NO: 1. Optionally, the at least one AAV rep gene comprises CTC at positions corresponding to positions 1826-1828 of SEQ ID NO: 1.

### Lack of a cap gene

A helper plasmid of the invention does not comprise a cap gene encoding a functional set of Cap proteins.

In native AAV (such as an AAV having a genome as set out in SEQ ID NO: 1), the AAV cap and the AAV rep genes are overlapping. In particular, the p40 promoter, which drives expression of the AAV cap gene is present within the AAV rep gene. Thus, any helper plasmid that encodes AAV Rep proteins will comprise nucleotides from the AAV cap gene. However, a helper plasmid of the invention does not comprise a stretch of nucleotides encoding a functional set of Cap proteins. In particular, a helper plasmid of the invention may not comprise a significant stretch of nucleotide sequence that is exclusively AAV cap gene sequence. The phrase *"exclusively cap gene sequence"* is intended to refer to gene sequence that encodes a portion of a Cap protein, but does not encode a portion of an AAV Rep protein, for example nucleotides 2253-4410 of SEQ ID NO: 1.

AAV Cap proteins (VP1, VP2 and VP3; see Figure 1) are proteins that assemble to form a capsid surrounding the viral genome. Thus, a gene encoding a functional set of AAV Cap proteins is one that encodes AAV Cap proteins capable of assembling to encapsidate a viral genome.

A *"functional"* set of AAV Cap proteins is one which is sufficient for encapsidation of AAV. It is within the abilities of the skilled person to determine whether the product of an AAV Cap gene is functional. The skilled person merely needs to determine whether the encoded AAV Cap protein(s) support AAV production using an AAV production assay as described above. In this case, the test two-plasmid system will comprise a vector plasmid that comprises the AAV cap gene which encodes the protein(s) whose *"functionality"* is to be determined, and otherwise the test two-plasmid system used will be identical to the reference two-plasmid system. The helper plasmid does not comprise a cap gene encoding a functional set of Cap proteins if it does not comprise any genetic material corresponding to a cap gene, or if any cap gene present in the helper plasmid encodes protein(s) which support AAV production at a level below 10% of the level supported by the wild type cap gene product, *i.e.* if the yield of rAAV produced is less than 10% of the yield of rAAV produced using the reference two-plasmid system.

In general, a functional cap gene is greater than 250 nucleotides in length. Accordingly, the helper plasmid, which does not comprise a cap gene encoding a functional set of Cap proteins, may not comprise a contiguous stretch of exclusively cap gene sequence of more than 250 nucleotides, more than 100 nucleotides, or more than 60 nucleotides. As discussed above, the AAV cap gene and the AAV rep gene in native AAV overlap. Optionally, the helper plasmid does not comprise a contiguous stretch of exclusively cap gene sequence of more than 60 nucleotides. Optionally the helper plasmid does not comprise a cap gene encoding a functional VP1 protein. Optionally, the helper plasmid comprises a portion of cap gene sequence, and the portion of cap gene sequence does not encode a set of functional Cap proteins. The helper plasmid can comprise a portion of AAV cap gene sequence, so long as it does not encode a functional set of AAV Cap proteins. So long as the portion of AAV cap gene sequence is not functional, multiple recombination events would be required to provide a rcAAV.

### At least one helper virus gene

The helper plasmid comprises at least one helper virus gene. AAV is only able to propagate in the presence of a helper virus. Examples of helper viruses include adenoviruses, and herpes viruses.

However, as set out above, growing AAV in the presence of a helper virus is not advantageous as helper viruses can be lytic to cells, including host cells used to grow AAV. Furthermore, if helper viruses are used in the production of rAAV products, such as gene therapy vectors, the helper virus may contaminate the product. As an alternative to co-infecting with helper virus such as adenovirus, the requisite genes of the helper virus can be provided on a transfected plasmid. For host cells expressing the adenoviral E1A/B genes (such as HEK293T cells) the remaining required helper genes are E4, E2A and VA RNA I and II (a VA nucleic acid). While these genes are distributed across a long stretch of the adenoviral genome, the present inventors have determined that large stretches of non-coding nucleotides separating the genomic genes can be removed without affecting expression of the genes. The inventors have, therefore, designed a minimal helper gene region, which is SEQ ID NO: 4. SEQ ID NO: 4 contains the VA nucleic acid, and the reverse complement (as present in a double stranded plasmid) of an E2A gene and an E4 gene. Using such a minimal helper gene region in a plasmid for the production of rAAV is advantageous as it allows the user to use a smaller plasmid which is less costly to produce and easier to transfect into the cell.

The helper plasmids of the invention comprise at least one helper virus gene. Preferably, the helper plasmids of the invention comprise sufficient helper genes to allow for AAV replication and packaging. Whether or not a helper plasmid comprises sufficient helper genes to facilitate AAV production can be assessed using an AAV production assay as described above. In this case, the test two-plasmid system will comprise a helper plasmid that comprises the helper genes whose ability to facilitate AAV production is to be tested, and otherwise the test two-plasmid system used will be identical to the reference two-plasmid system. In one embodiment, the helper gene products will be considered to facilitate AAV production if they support rAAV production at a level at least 25%, at least 40%, at least 50%, at least 60%, at least 70%, at least 80%, at least 90% or at least 95% of the level supported by adenoviral helper genes encoding E4, E2A and VA RNA I and II, *i.e.* if the yield of rAAV produced is at least 25%, at least 40%, at least 50%, at least 60%, at least 70%, at least 80%, at least 90% or at least 95% of the yield of rAAV produced using the reference two-plasmid system. Preferably, the helper gene products will be considered to facilitate AAV production if they support rAAV production at a level at least 70%, at least 80%, at least 90% or at least 95% of the level supported by adenoviral helper genes encoding E4, E2A and VA RNA I and II.

Since the helper plasmids encode the genes required to allow for efficient AAV production, the addition of a helper virus is not required.

Optionally, the at least one helper virus gene is an adenovirus gene. Adenovirus is a virus which is known to aid propagation of AAV (Xiao et al (1998), J. Virol, 72:2224-2232). Optionally, the at least one helper virus gene is an Adenovirus 5 gene or an Adenovirus 2 gene. The genome of Adenovirus 5 is set out in SEQ ID NO: 2, and the genome of Adenovirus 2 is set out in SEQ ID NO: 3. Accordingly, the helper genes may comprise a stretch of nucleotides present in SEQ ID NO: 2 or SEQ ID NO: 3, or a corresponding stretch of nucleotides in another serotype of adenovirus.

The helper genes of adenoviruses encode E1A, E1B, E4, E2A and VA RNA I and II.

E1A is encoded by nucleotides 560-1545 of the Adenovirus 5 genome (for example the genome of SEQ ID NO: 2). Nucleotides 560-1545 contain an intron, from nucleotide 1113 to nucleotide 1228. This intron is not essential, and so an E1A gene comprising nucleotides 560-1112 and 1229-1545 of SEQ ID NO: 2 would encode a functional E1A protein.

E1B is actually two proteins E1B 19K and E1B 55K, which work together to block apoptosis in adenovirus-infected cells. E1B is encoded by nucleotides 1714-2244 (E1B 19K), and by nucleotides 2019-3509 (E1B 55 K) of the Adenovirus 5 genome (for example the genome of SEQ ID NO: 2).

E4 is encoded by a number of different open reading frames (ORFs) of the Adenovirus 5 genome (for example the genome of SEQ ID NO: 2). E4 ORF 6/7 is encoded by nucleotides 32914-34077, which comprises an intron between nucleotides 33193 and 33903. This intron is not essential, and so an E4 ORF 6/7 comprising nucleotides 32914-33192 and 33904-34077 of SEQ ID NO: 2 is sufficient. E4 34K is encoded by nucleotides 33193-34077 of the Adenovirus 5 genome (for example the genome of SEQ ID NO: 2). E4 ORF 4 is encoded by nucleotides 33998-34342 of the Adenovirus 5 genome (for example the genome of SEQ ID NO: 2). E4 ORF 3 is encoded by nucleotides 34353-34703 of the Adenovirus 5 genome (for example the genome of SEQ ID NO: 2). E4 ORF B is encoded by nucleotides 34700 to 35092 of the Adenovirus 5 genome (for example the genome of SEQ ID NO: 2). E4 ORF 1 is encoded by nucleotides 35140-35526 of the Adenovirus 5 genome (for example the genome of SEQ ID NO: 2).

A functional E4 protein may only comprise amino acids encoded by ORFs 6 and 7, as only the amino acids encoded by ORFs 6 and 7 are required for activity. Optionally, therefore, the functional E4 protein comprises a polypeptide sequence encoded by all or a significant portion of ORFs 6 and 7. Optionally, the functional E4 protein does not comprise polypeptide sequence encoded by all or a portion of ORFs 1-4 an 34K. However, the amino acids encoded by ORFs 1-3 and 34K do improve the activity of the E4 protein, and so in some embodiments the functional E4 protein comprises amino acids encoded by ORFs 1-7.

The E2 (E2A) gene is encoded by nucleotides 22443-24032 of the Adenovirus 5 genome (for example the genome of SEQ ID NO: 2).

The VA RNA I and II is encoded by nucleotides 10589-11044 of the Adenovirus 5 genome (for example the genome of SEQ ID NO: 2).

E1B and E4 are believed to enhance AAV mRNA accumulation, and E2A and VA RNA I and II are believed to enhance AAV mRNA splicing and translation. E1B, E4 and E2A are proteins encoded by genes present in the adenovirus genome, whereas the VA nucleic acid encodes two RNA transcripts known as VA RNA I and VA RNA II. The transcripts themselves are functional in the cell, and are never translated into amino acid sequences. It will be appreciated, therefore, that the VA nucleic acid does not encode a protein, but does *"encode"* or *"correspond to"* an RNA, *i.e.* whilst the term *"encode"* is used the VA nucleic acid is a non-translated nucleic acid sequence.

Of the five adenovirus genes, it is possible not to include E1A or E1B in the helper plasmid, as some host cell lines (such as HEK293 cells) express one or more of E1A or E1B constitutively. Optionally, therefore, the helper plasmid does not comprise a gene encoding a functional adenoviral E1A/B protein.

In one embodiment, the at least one helper virus gene comprises:
(a) a VA (viral associated) nucleic acid encoding functional VA RNA I and II;
(b) an E2A gene encoding a functional E2A protein; and/or
(c) an E4 gene encoding a functional E4 protein.

Optionally, the at least one helper virus gene comprises a VA nucleic acid, an E2A gene and an E4 gene.

A *"functional"* VA RNA I and II, E2A protein or E4 protein is able to facilitate production of AAV. It is within the abilities of the skilled person to determine whether a given VA RNA I and II, E2A protein or E4 protein is functional. The skilled person merely needs to determine whether the VA RNA I and II, E2A protein or E4 protein supports AAV production using an AAV production assay as described above. In this case, the test two-plasmid system will comprise a helper plasmid that comprises the VA RNA I and II, E2A protein or E4 protein whose *"functionality"* is to be determined, and otherwise the test two-plasmid system used will be identical to the reference two-plasmid system. In an embodiment, the VA RNA I and II, E2A protein or E4 protein will be considered to be *"functional"* if it supports rAAV production at a level at least 25%, at least 40%, at least 50%, at least 70%, at least 80%, at least 90% or at least 95% of the level supported by the wild type (for example as found in native Adenovirus 5; SEQ ID NO: 2) VA RNA I and II, E2A protein or E4 protein, *i.e.* if the yield of rAAV produced is at least 25%, at least 40%, at least 50%, at least 70%, at least 80%, at least 90% or at least 95% of the yield of rAAV produced using the reference two-plasmid system. Preferably, the E4 protein will be considered to be *"functional"* if it supports rAAV production at a level at least 70%, at least 80%, at least 90% or at least 95% of the level supported by the wild type E4 protein.

Optionally, the E4 gene is not located between the VA nucleic acid and the E2A gene, *i.e.* the sequence of the plasmid is such that E4 gene sequence does not appear in the plasmid between the VA nucleic acid sequence and the E2A gene sequence. Optionally, the E2A gene is located between the VA nucleic acid and the E4 gene.

The helper plasmid is double stranded, and any of the genes comprised within the helper plasmid may be present in a *"forward"* or *"reverse"* orientation. For example, the E4 gene may comprise nucleotides 22443-24032 of the Adenovirus 5 genome (for example the genome of SEQ ID NO: 2) or may comprise the reverse complement of nucleotides 22443-24032. Thus, in all instances of the application where reference is made to a plasmid *"comprising"* a certain nucleic acid sequence, the references should be interpreted as encompassing embodiments where the plasmid comprises the reverse complement of the nucleic acid sequence.

The present inventors have demonstrated that removing all or part of the stretch of nucleotides present at positions 10595-10619 in the adenovirus genome (for example the genome of SEQ ID NO: 2) significantly reduces (by about 50%) the activity of the VA RNA I and II encoded by the VA nucleic acid. Avoiding this reduction in activity is advantageous.

Accordingly, in one embodiment, the VA nucleic acid has an activity level which has at least 75%, at least 80%, at least 90%, at least 95%, or between 95% and 100% of the activity of a wild type VA nucleic acid from Adenovirus 5. Optionally, the activity level of the VA nucleic acid is determined by measuring rAAV yield, for example using the AAV production assay described above. In an embodiment, the VA nucleic acid comprises a contiguous sequence at least 95%, at least 98%, or 100% identical to a stretch of at least 15 nucleotides, at least 20 nucleotides, or 26 nucleotides of nucleotides 10595-10619 of SEQ ID NO: 2, or a corresponding stretch of nucleotides in a different serotype of adenovirus.

Optionally, the E2A gene is operably linked to a promoter which comprises a contiguous sequence at least 96%, at least 98%, or 100% identical to a stretch of at least 60, at least 70, at least 80, or 100 nucleotides of nucleotides 27037-27136 of SEQ ID NO: 2, or a corresponding stretch of nucleotides in a different serotype of adenovirus.

Expression of the E4 gene is driven by a promoter (designated *"E4 promoter"* herein), corresponding to nucleotides 35585-35848 of SEQ ID NO: 2. Nucleotides corresponding to 35793-35848 of the Adenovirus 5 genome (such as a genome of SEQ ID NO: 2), are required for the promoter to be fully active. Optionally, therefore, the E4 gene is operably linked to an E4 promoter that has at least 50%, at least 70%, or at least 90% of the activity of a wild type promoter from Adenovirus 5. The activity of the E4 promoter may be determined by testing its ability to drive expression of a protein.

Whether or not a given E4 promoter is able to drive E4 gene expression may be determined using an AAV production assay as described above. Specifically, if the E4 promoter can drive E4 gene expression it will facilitate AAV production. In this case, the test two-plasmid system will comprise a vector plasmid that comprises the E4 gene and an E4 gene promoter whose ability to facilitate AAV production is to be tested, and otherwise the test two-plasmid system used will be identical to the reference two-plasmid system. In one embodiment, the E4 promoter will be considered to have at least 25%, at least 40%, at least 50%, at least 70%, or at least 90% of the activity of a wild type E4 promoter from Adenovirus 5 if it supports rAAV production at a level at least 25%, at least 40%, at least 50%, at least 60%, at least 70%, or at least 90% of the level supported by a wild type E4 gene promoter, *i.e.* if the yield of rAAV produced is at least 50%, at least 70%, or at least 90% of the yield of rAAV produced using the reference two-plasmid system. Preferably, the E4 promoter will be considered to facilitate AAV production if it supports rAAV production at a level at least 70%, at least 80%, at least 90% or at least 95% of the yield of rAAV produced using the reference two-plasmid system.

### Reducing the size of the helper plasmid

The present inventors have determined that there is a significant amount of non-helper gene nucleic acid sequence between the different helper gene sequences in native Adenovirus 5, and that a substantial portion of this nucleic acid sequence can be removed without significantly impacting the expression level and function of the proteins encoded by the helper gene sequences. Accordingly, the inventors have succeeded in defining a helper gene region with a reduced size, and this can be included in the helper plasmid to reduce its size. Having a reduced size helper gene region is advantageous as it allows the overall size of the helper plasmid to be reduced. Smaller plasmids are cheaper to produce and easier to introduce into host cells.

Accordingly, the helper plasmid may be less than 25000 bp, less than 20000 bp, less than 15000 bp, less than 14500 bp, between 10000 bp and 25000 bp, between 10000 bp and 20000 bp, between 12000 bp and 15000 bp, or around 14021 bp in length.

Similarly, the at least one helper virus gene comprises a VA nucleic acid, an E2A gene and an E4 gene, and the VA nucleic acid, the E2A gene and the E4 gene are comprised within a contiguous stretch of nucleotides on the helper plasmid of fewer than 15000, fewer than 12000, fewer than 10000, fewer than 9000, or fewer than 8500 nucleotides.

An example of a helper gene region with a reduced size is set out in SEQ ID NO: 4. The helper gene region is a contiguous region of the plasmid starting at the first nucleotide encoding a helper gene and ending at the last nucleotide encoding a helper gene. Optionally, the helper gene region comprises a sequence having at least 95%, at least 98%, at least 99%, or 100% identity to the full length or to a fragment at least 6000 nucleotides, at least 7000 nucleotides, or at least 8000 nucleotides in length of SEQ ID NO: 4, *i.e.* the at least one helper virus gene is comprised on a contiguous stretch of the plasmid having at least 95%, at least 98%, at least 99%, or 100% identity to the full length or to a fragment at least 6000 nucleotides, at least 7000 nucleotides, or at least 8000 nucleotides in length, of SEQ ID NO: 4. Optionally, the helper gene region consists of a sequence having at least 95%, at least 98%, at least 99%, or 100% identity to the full length or to a fragment at least 6000 nucleotides, at least 7000 nucleotides, or at least 8000 nucleotides in length of SEQ ID NO: 4, *i.e.* the at least one helper virus gene is comprised on a contiguous stretch of the plasmid consisting of a sequence at least 95%, at least 98%, at least 99%, or 100% identity to the full length or to a fragment at least 6000 nucleotides, at least 7000 nucleotides, or at least 8000 nucleotides in length of SEQ ID NO: 4.

The present inventors have identified specific regions of the Adenovirus 5 genome that can be excluded from the helper plasmid.

In one embodiment, the helper plasmid does not comprise a contiguous sequence of at least 2000, at least 2500, at least 3000, or 3427 nucleotides of a contiguous stretch of nucleotides of equivalent length comprised within nucleotides 194-3620 of SEQ ID NO: 2, or a corresponding stretch of nucleotides in a different serotype of adenovirus. In one embodiment, the helper plasmid does not comprise a contiguous sequence of at least 50, at least 60, or 69 nucleotides of a contiguous stretch of nucleotides of equivalent length comprised within nucleotides 4032-4100 of SEQ ID NO: 2, or a corresponding stretch of nucleotides in a different serotype of adenovirus.

In an embodiment, the helper plasmid does not comprise a contiguous sequence of at least 15000, at least 20000, at least 22000, or 22137 nucleotides of a contiguous stretch of nucleotides of equivalent length comprised within nucleotides 10619-32755 of SEQ ID NO: 2, or a corresponding stretch of nucleotides in a different serotype of adenovirus.

In an embodiment, the helper plasmid does not comprise a contiguous sequence of at least 15000, at least 20000, at least 21000, or 21711 nucleotides of a contiguous stretch of nucleotides of equivalent length comprised within nucleotides 11045-32755 of SEQ ID NO: 2, or a corresponding stretch of nucleotides in a different serotype of adenovirus.

Similarly, the inventors have identified regions of the AAV2 genome that do not need to be included in the helper plasmid and may accordingly be excluded in the interests of minimising the size of the helper plasmid. Accordingly, in one embodiment, the helper plasmid does not comprise a contiguous sequence of at least 200, at least 300, at least 350, or 363 nucleotides of a contiguous stretch of nucleotides of equivalent length comprised within nucleotides 4051-4413 of SEQ ID NO: 1, or a corresponding stretch of nucleotides in a different serotype of AAV. Similarly, in one embodiment, the helper plasmid does not comprise a contiguous sequence of at least 400, at least 500, at least 600, or 647 nucleotides of a contiguous stretch of nucleotides of equivalent length comprised within nucleotides 2301-2947 of SEQ ID NO: 1, or a corresponding stretch of nucleotides in a different serotype of AAV.

### Artificial rep binding sites

Rep proteins bind to a rep binding site (RBS), which is a nucleic acid sequence having a consensus sequence of GCTCGCTCGCTCGCTC (McCarty, D. M. et al. (1994), J. Virol., 68(8): 4988-4997)). Accordingly, a rep binding site is a nucleic acid sequence having homology to GCTCGCTCGCTCGCTC. Determining whether a nucleic acid sequence (test nucleic acid sequence) comprises a rep binding site, for example, determining whether a nucleic acid sequence comprises a sequence having homology to GCTCGCTCGCTCGCTC, can be carried out using an electrophoretic mobility shift assay (EMSA). The test nucleic acid sequence is applied to a first well of a gel suitable for use in electrophoresis, and a mixture of the test nucleic acid sequence and Rep68/Rep78 proteins is applied to a second well of the gel. If the test nucleic acid sequence comprises a rep binding site, this will cause a shift in the mobility of the nucleic acid sequence in the well comprising the Rep68/Rep78 proteins compared to the well that lacked the Rep68/Rep78 proteins.

Binding of Rep proteins to aberrant RBSs might result in Rep-mediated non-homologous recombination between different sequences comprising RBSs, resulting in undesired fusion sequences. For example, Rep proteins could bind the RBS in AAV ITRs and any RBS present on a bacterial plasmid backbone resulting in the fusion of plasmid backbone sequences to an ITR. This could result in enhanced replication and packaging of such undesired fusion sequences.

Accordingly, it is preferred that the only RBSs present in the plasmids of the invention correspond to RBSs present in wild type AAV (allowing the Rep proteins to carry out their normal function). However, when a plasmid is constructed it is possible that an RBS will be present, either in the plasmid backbone or elsewhere in the plasmid in non-AAV-derived sequences, and such sequences are considered to be *"artificial'* or *"aberrant"* RBSs, and should be avoided/removed.

A common source of artificial RBSs in plasmids is from the plasmid backbone. The plasmid backbone is the bacterial sequence needed to amplify the plasmids in bacterial host cells. The plasmid backbone may be the region of the plasmid that is not derived from adenovirus or AAV or is not situated between two AAV-derived ITRs. Optionally, the vector plasmid backbone encompasses any nucleotides except the cap gene, a promoter (region) operably linked to the cap gene, ITRs and the expression cassette. Optionally, the helper plasmid backbone encompasses any nucleotides except the at least one helper gene and associated adenovirus-derived regulatory elements, the at least one rep gene and associated AAV-derived regulatory elements, and one or more promoters operably linked to the at least one rep gene. In one embodiment, the helper plasmid and/or the vector plasmid comprises a backbone, and the plasmid backbone does not comprise an artificial RBS. Optionally, the helper plasmid and the vector plasmid each comprise a backbone, and neither plasmid backbone comprises an artificial RBS.

Accordingly, in one embodiment, the helper plasmid and/or the vector plasmid does not comprise an artificial RBS. Preferably, the helper plasmid and the vector plasmid do not comprise an artificial RBS. Optionally, there are no RBSs in the vector plasmid and/or the helper plasmid, except within any ITR(s), p5 promoter(s) and p19 promoter(s).

### AAV Cap gene

The vector plasmid may comprise an AAV cap gene. The AAV cap gene encodes a functional AAV Cap protein. The AAV cap gene may encode a functional set of AAV Cap proteins. AAV generally comprise three AAV Cap proteins, VP1, VP2 and VP3. These three proteins form a capsid into which the AAV genome is inserted, and allow the transfer of the AAV genome into a host cell. All of VP1, VP2 and VP3 are encoded in native AAV by a single gene, the AAV cap gene. The amino acid sequence of VP1 comprises the sequence of VP2. The portion of VP1 which does not form part of VP2 is referred to as VPlunique or VP1U. The amino acid sequence of VP2 comprises the sequence of VP3. The portion of VP2 which does not form part of VP3 is referred to as VP2unique or VP2U.

A *"functional"* set of AAV Cap proteins is one which allows for encapsidation of AAV. As discussed above, it is within the abilities of the skilled person to determine whether a given AAV Cap protein is or a set of AAV Cap proteins are functional. The skilled person merely needs to determine whether the encoded AAV Cap protein(s) support AAV production using an AAV production assay as described above. In this case, the test two-plasmid system will comprise a vector plasmid that comprises the AAV cap gene which encodes the AAV Cap protein(s) whose *"functionality"* is to be determined, and otherwise the test two-plasmid system used will be identical to the reference two-plasmid system. The AAV Cap protein(s) will be considered to be *"functional"* if it/they support(s) rAAV production at a level at least 25%, at least 40%, at least 50%, at least 70%, at least 80%, at least 90% or at least 95% of the level supported by the wild type AAV cap gene product, *i.e.* if the yield of rAAV produced is at least 25%, at least 40%, at least 50%, at least 70%, at least 80%, at least 90% or at least 95% of the yield of rAAV produced using the reference two-plasmid system. Preferably, the AAV Cap protein(s) will be considered to be *"functional"* if it/they support(s) rAAV production at a level at least 70%, at least 80%, at least 90% or at least 95% of the level supported by the wild type AAV cap gene product.

Optionally, VP2 and/or VP3 proteins are *"functional"* if an AAV comprising the VP2 and/or the VP3 proteins is able to transduce Huh7 cells at a level at least 25%, at least 40%, at least 50%, at least 70%, at least 80%, at least 90% or at least 95% of that of an equivalent AAV comprising a wild type VP2 and/or VP3 protein. The ability of an AAV particle to transduce Huh7 cells can be tested by adding a reporter protein such as green fluorescent protein (GFP) to the AAV particle, mixing the AAV particle with Huh7 cells, and measuring the fluorescence produced.

Optionally, the vector plasmid comprises an AAV cap gene that encodes a VP1, a VP2 and/or a VP3 protein. Optionally, the VP1, VP2 and VP3 proteins are expressed from more than one AAV cap gene. Optionally, the vector plasmid comprises an AAV cap gene that encodes a VP1, a VP2 and a VP3 protein. Optionally the vector plasmid comprises an AAV cap gene encoding a functional VP1, *i.e.* a VP1 protein capable of assembling with other AAV Cap proteins to encapsidate a viral genome.

Different serotypes of AAV have AAV Cap proteins having different amino acid sequences. An AAV cap gene encoding any (set of) AAV Cap protein(s) is suitable for use in connection with the present invention. The AAV Cap protein can be a native AAV Cap protein expressed in AAV of a certain serotype. Alternatively, the AAV Cap protein can be a non-natural, for example an engineered, AAV Cap protein, which is designed to comprise a sequence different to that of a native AAV Cap protein. Genes encoding non-natural Cap proteins are particularly advantageous, as in the context of gene therapy applications it is possible that fewer potential patients have levels of antibodies that prevent transduction by AAV comprising non-natural Cap proteins, relative to native capsids.

Optionally, the AAV cap gene encodes an AAV Cap protein from a serotype selected from the group consisting of serotypes 1, 2, 3A, 3B, 4, 5, 6, 7, 8, 9, 10, 11, 12, or 13. Optionally, the AAV cap gene encodes an AAV Cap protein selected from the group consisting of LK03, rh74, rh10 and Mut C (WO 2016/181123; WO 2013/029030; WO 2017/096164).

### AAV Cap gene promoter

Optionally, the vector plasmid comprises an AAV cap gene promoter. The AAV cap gene promoter may be operably linked to an AAV cap gene. Alternatively, the vector plasmid may not comprise an AAV cap gene, but may comprise a cloning site operably linked *(i.e.* in close juxtaposition: 5'-[cap gene promoter]-[cloning site]-3') to the cap gene promoter. The cloning site may be a multiple cloning site (MCS, or polylinker; see for example Figure 4C-E). The user may wish to have the option to add a specific cap gene for a specific application. For example, if the vector plasmid is to be used to produce AAV for use in gene therapy, the user may wish the vector plasmid to lack a cap gene, but comprise a cloning site to allow a specific cap gene to be cloned in for a specific application. The vector plasmid could be used in connection with any transgene (in an expression cassette), and the user may find that for certain transgenes, encapsidation of such cassettes into capsids having properties such as liver tropism is advantageous, whereas for other transgenes capsids having different tropisms are advantageous. By designing a vector plasmid that comprises a cap gene promoter linked to a cloning site, the user can readily *'plug in'* an appropriate cap gene for a specific application (such as use of a specific transgene).

The native AAV cap gene *(i.e.* the cap gene of a wild type AAV) is operably linked to a p40 promoter, a p5 promoter and a p19 promoter. Optionally, the at least one AAV cap gene promoter comprises an AAV p40 promoter, a p5 promoter, and/or a p19 promoter. Optionally, the at least one AAV cap gene promoter comprises an AAV p40 promoter, a p5 promoter, and a p19 promoter. However, any suitable promoter that is able to drive AAV cap gene expression can be used.

Whether or not a given AAV cap gene promoter is able to drive AAV cap gene expression may be determined using an AAV production assay as described above. Specifically, if the AAV cap gene promoter can drive AAV cap gene expression it will facilitate AAV production. In this case, the test two-plasmid system will comprise a vector plasmid that comprises the AAV cap gene and an AAV cap gene promoter whose ability to facilitate AAV production is to be tested, and otherwise the test two-plasmid system used will be identical to the reference two-plasmid system. In one embodiment, the AAV cap gene promoter will be considered to facilitate AAV production if it supports rAAV production at a level at least 25%, at least 40%, at least 50%, at least 60%, at least 70%, at least 80%, at least 90% or at least 95% of the level supported by a wild type p40 cap gene promoter, *i.e.* if the yield of rAAV vectors produced is at least 25%, at least 40%, at least 50%, at least 60%, at least 70%, at least 80%, at least 90% or at least 95% of the yield of rAAV produced using the reference two-plasmid system. Preferably, the AAV cap gene promoter will be considered to facilitate AAV production if it supports rAAV production at a level at least 70%, at least 80%, at least 90% or at least 95% of the yield of rAAV produced using the reference two-plasmid system.

The p40 promoter is understood to drive expression of the cap gene. The native p40 promoter is contained within the native rep gene. The AAV2 p40 promoter has a sequence of nucleotides 1710-1827 of SEQ ID NO: 1. However, p40 promoters in other serotypes of AAV may comprise slightly different sequences. In some embodiments, the p40 promoter has a sequence at least 95%, at least 98%, or 99% identical to nucleotides 1710-1827 of SEQ ID NO: 1, or a corresponding sequence from another serotype of AAV. In some embodiments, the p40 promoter has a sequence at least 98% identical to nucleotides 1710-1827 of SEQ ID NO: 1.

The present inventors have found that an at least one AAV cap gene promoter that comprises a p40 promoter, a p5 promoter and a p19 promoter is advantageous, as the p5 promoter and the p19 promoter play a role in regulating the p40 promoter, and the presence of the p40 promoter, the p5 promoter and the p19 promoter leads to timely regulated expression of the AAV cap gene resulting in high yield/high quality rAAV. Accordingly, in some embodiments, the at least one AAV cap gene promoter is comprised in a 'promoter region' comprising a p40 promoter, a p5 promoter and a p19 promoter. Optionally, the promoter region comprises a sequence at least 95%, at least 98%, at least 99% or 100% identity to the full length or a fragment of at least 800, at least 900, at least 1000 or at least 1100 nucleotides in length of the following stretches of native AAV2 sequence (SEQ ID NO: 1) positioned in immediate juxtaposition from 5' to 3': 200-354; 600-1049; 1701-2202, or to corresponding juxtaposed stretches of nucleotides from a different serotype of AAV.

The native p5 promoter is upstream of the native AAV rep gene. The AAV2 p5 promoter has a sequence of nucleotides 204-292 of SEQ ID NO: 1. However, p5 promoters in other serotypes of AAV may comprise slightly different sequences. In some embodiments, the p5 promoter has a sequence at least 95%, at least 98%, or at least 99% identical to nucleotides 204-292 of SEQ ID NO: 1, or a corresponding sequence from another serotype of AAV. In some embodiments, the p5 promoter has a sequence at least 98% identical to nucleotides 204-292 of SEQ ID NO: 1.

The p19 promoter is contained within the native AAV rep gene. The AAV2 p19 promoter has a sequence of nucleotides 730-890 of SEQ ID NO: 1. However, p19 promoters in other serotypes of AAV may comprise slightly different sequences. In some embodiments, the p19 promoter has a sequence at least 95%, at least 98%, or at least 99% identical to nucleotides 730-890 of SEQ ID NO: 1, or a corresponding sequence from another serotype of AAV. In some embodiments, the p19 promoter has a sequence at least 98% identical to nucleotides 730-890 of SEQ ID NO: 1.

### An expression cassette flanked on at least one side by an ITR

The two-plasmid system of the invention may be used to produce AAV vector for use in gene therapy. A *"gene therapy"* involves administering AAV/viral particles of the invention that is capable of expressing a transgene (such as a Factor IX-encoding nucleotide sequence) in the host to which it is administered. In such cases, the vector plasmid will comprise an expression cassette.

Optionally, the vector plasmid comprises at least one ITR. Thus, the vector plasmid comprises at least one ITR, but, more typically, two ITRs (generally with one either end of the expression cassette, i.e. one at the 5' end and one at the 3' end). There may be intervening sequences between the expression cassette and one or more of the ITRs. The expression cassette may be incorporated into a viral particle located between two regular ITRs or located on either side of an ITR engineered with two D regions. Optionally, the vector plasmid comprises ITR sequences which are derived from AAV1, AAV2, AAV4 and/or AAV6. Preferably the ITR sequences are AAV2 ITR sequences.

Optionally, the vector plasmid comprises an expression cassette. As described herein, an expression cassette refers to a sequence of nucleic acids comprising a transgene and a promoter operably linked to the transgene. Optionally, the cassette further comprises additional transcription regulatory elements, such as enhancers, introns, untranslated regions, transcriptional terminators etc.

Optionally, the expression cassette comprises a transcription regulatory element comprising the promoter element and/or enhancer element from HLP2, HLP1, LP1, HCR-hAAT, ApoE-hAAT, and/or LSP. These transcription regulatory elements are described in more detail in the following references: HLP2: WO16/075473; HLP1: McIntosh J. et al., Blood 2013 Apr 25, 121(17):3335-44; LP1: Nathwani et al., Blood. 2006 April 1, 107(7): 2653-2661; HCR-hAAT: Miao et al., Mol Ther. 2000;1: 522-532; ApoE-hAAT: Okuyama et al., Human Gene Therapy, 7, 637-645 (1996); and LSP: Wang et al., Proc Natl Acad Sci U S A. 1999 March 30, 96(7): 3906-3910. Each of these transcription regulatory elements comprises a promoter, an enhancer, and optionally other nucleotides. If the polynucleotide is intended for expression in the liver, the promoter may be a liver-specific promoter. Optionally, the promoter is a human liver-specific promoter.

The transgene may be any suitable gene. If the vector plasmid is for use in gene therapy, the transgene may be any gene that comprises or encodes a protein or nucleotide sequence that can be used to treat a disease. For example, the transgene may encode an enzyme, a metabolic protein, a signalling protein, an antibody, an antibody fragment, an antibody-like protein, an antigen, or a non-translated RNA such as an miRNA, siRNA, snRNA, or antisense RNA.

### Dispensable translation initiation codons

Optionally, the vector plasmid does not comprise any dispensable translation initiation codons. Optionally, the vector plasmid may comprise at least two genes that must be capable of being transcribed and translated (the transgene (in an expression cassette) and the AAV cap gene). Optionally, the transgene encodes a functional RNA which does not encode a translational protein or polypeptide. The transgene, if protein-encoding, and the AAV cap gene will comprise start (ATG or GTG) codons to promote initiation of translation of the gene. However, the vector plasmid may comprise additional instances of ATG or GTG (either in-frame or out of frame with the reading frame of these genes), and it is possible that translation may initiate at one of these positions. Since there is no need for translation to be initiated at the site of these additional instances of ATG or GTG, the ATG or GTG codons may be considered to be *"dispensable translation initiation codons".* It is preferred that dispensable translation initiation codons are removed or rendered non-functional, in particular where they occur in the promoter region. The promoter region is a region of the vector plasmid that comprises one or more promoters operably linked to the AAV cap gene. In some embodiments, the AAV cap gene is operably linked to one or more of the p5, p19 and p40 promoters, and in such embodiments the promoter region comprises the p5, p19 and p40 promoters.

Optionally, the plasmid comprises a promoter region comprising one or more promoters, and the promoter region does not comprise ATG or GTG codons. Optionally, the promoter region comprises p5, p19 and/or p40 promoters, and wherein ATG or GTG codons at one or more positions corresponding to positions (a) 321-323 (Rep78/68 ATG start codon), (b) 766-768 (ATG codon), (c) 955-957 (ATG codon), (d) 993-995 (Rep52/40 ATG start codon) and (e) 1014-1016 (GTG codon) of SEQ ID NO: 1 are absent or mutated.

Optionally, in the promoter region:
(a) nucleotides corresponding to nucleotides 321-323 of SEQ ID NO: 1 are absent;
(b) nucleotides corresponding to nucleotides 766-768 of SEQ ID NO: 1 are not ATG and are optionally ATT;
(c) nucleotides corresponding to nucleotides 955-957 of SEQ ID NO: 1 are absent;
(d) nucleotides corresponding to nucleotides 993-995 of SEQ ID NO: 1 are absent; and/or
(e) nucleotides corresponding to nucleotides 1014-1016 of SEQ ID NO: 1 are absent.

Optionally, in the promoter region :
(a) nucleotides corresponding to nucleotides 321-323 of SEQ ID NO: 1 are absent;
(b) nucleotides corresponding to nucleotides 766-768 of SEQ ID NO: 1 are not ATG and are optionally ATT;
(c) nucleotides corresponding to nucleotides 955-957 of SEQ ID NO: 1 are absent;
(d) nucleotides corresponding to nucleotides 993-995 of SEQ ID NO: 1 are absent; and
(e) nucleotides corresponding to nucleotides 1014-1016 of SEQ ID NO: 1 are absent.

### Size of the vector plasmid

As discussed above in connection with the helper plasmid, it is advantageous that plasmids used in the production of rAAV are as small as possible. Smaller plasmids are more cost-effective to produce and are easier to transfect into cells.

The size of the vector plasmid will in part be defined by the size of the promoters, transcription regulatory elements, transgenes and AAV cap genes that are included, and as discussed above, the nature of these features will be partly or fully defined by the application for which the rAAV is to be used.

However, the size of the vector plasmid can be reduced by ensuring that the size of the backbone that is used is small, and/or by ensuring that the vector plasmid does not comprise a spacer (a contiguous regions of non-coding nucleic acid sequences of greater than 200 nucleotides in length).

Optionally, the vector plasmid comprises a backbone of fewer than 4000 nucleotides, fewer than 3500 nucleotides, fewer than 3000 nucleotides, or fewer than 2500 nucleotides in length. Optionally, the vector plasmid does not comprise any spacers.

### Host cell

Disclosed is a host cell comprising the two-plasmid system of the invention, the helper plasmid of the invention or the vector plasmid.

The host cell is preferably a host cell that can be used to produce rAAV. The host cell may therefore be a host cell suitable for the production of rAAV. In addition, the host cell may be for the production of rAAV.

In general a host cell that is suitable for the production of rAAV is a host cell that is derived from a eukaryotic cell line, preferably a vertebrate cell line, preferably a mammalian cell line, preferably a human cell line. Optionally, the host cell is a cell selected from the group consisting of a HEK293T cell, a HEK293 cell, a HEK293EBNA cell, a CAP cell, a CAP-T cell, an AGE1.CR cell, a PerC6 cell, a C139 cell, an EB66 cell, a BHK cell, a COS cell, a Vero cell, a Hela cell, and an A549 cell. Preferably, the host cell is selected from the group consisting of a HEK293T cell, a HEK293 cell, a HEK293EBNA cell, a CAP cell, a CAP-T cell, an AGE1.CR cell, a PerC6 cell, a C139 cell, and an EB66 cell. Even more preferably, the host cell is selected from the group consisting of a HEK293T cell, a HEK293 cell, and a HEK293EBNA cell. For example, the host cell may be a HEK293T cell. Optionally, the host cell is a cell that expresses a functional adenoviral E1A/B protein. For example, the host cell may comprise a chromosome comprising a gene encoding a functional adenoviral E1A/B protein. Functional adenoviral E1A/B proteins are discussed in the section entitled *"At least one helper virus gene".*

It is within the abilities of the skilled person to determine whether a host cell is suitable for the production of rAAV. The skilled person merely needs to determine whether the host cell supports AAV production using an AAV production assay as described above. In this case, the test two-plasmid system and the reference two-plasmid system that are used will be identical. However, the test two-plasmid system will be transfected into the host cell whose suitability for the production of recombinant AAV is to be tested, and the reference two-plasmid system will be transfected into HEK293T cells. The host cell will be considered to be suitable for the production of recombinant AAV if it supports AAV production at a level at least 30%, at least 40%, at least 50%, at least 70%, at least 80%, at least 90% or at least 95% of the level supported by HEK293T cells, *i.e.* if the yield of recombinant AAV produced is at least 30%, at least 40%, at least 50%, at least 70%, at least 80%, at least 90% or at least 95% of the yield of recombinant AAV produced in HEK293T cells.

### A low level of replication competent AAV (rcAAV)

The two-plasmid system of the invention, the helper plasmid of the invention or the vector plasmid may be used for producing a rAAV preparation having a low level of replication competent AAV (rcAAV).

The helper plasmid or the vector plasmid may also be used for reducing or minimising the level of rcAAV produced during rAAV production.

The present invention also provides a method for producing a rAAV preparation comprising:
(a) obtaining the two-plasmid system of the invention, the helper plasmid of the invention or the vector plasmid;
(b) transfecting a host cell with the two-plasmid system of the invention, the helper plasmid of the invention or the vector plasmid; and
(c) culturing the host cell under conditions suitable for rAAV production
wherein the rAAV preparation comprises a low level of replication competent AAV (rcAAV).

Also provided is a method for reducing or minimising the level of replication competent AAV (rcAAV) produced during recombinant AAV (rAAV) production comprising:
(a) obtaining the two-plasmid system of the invention, the helper plasmid of the invention or the vector plasmid;
(b) transfecting a host cell with the two-plasmid system of the invention, the helper plasmid of the invention or the vector plasmid; and
(c) culturing the host cell under conditions suitable for rAAV production.

The method may further comprise a step of harvesting the rAAV to provide an rAAV preparation, optionally having a low level of rcAAV.

"RAAV" or *"recombinant AAV"* refers to AAV particles, *i.e.* particles comprising an AAV genome (such as a vector genome) and an AAV capsid.

For the purposes of the present invention, the terms *"rcAAV"* or *"replication competent AAV"* is intended to refer to rAAV particles that comprise a genome which comprises a rep gene or a genome which comprises a cap gene. Whilst, for the purposes of the present invention, the term *"rcAAV"* is intended to refer to rAAV particles that comprise a genome comprising a rep gene or a cap gene, it is understood that the rAAV particles that comprise a genome comprising a rep gene but not a cap gene (herein so-called *"cap-deficient rcAAV"*) or a genome comprising a cap gene but not a rep gene (herein so-called *"rep-deficient rcAAV"*) are not replication competent in isolation. Rather, in order to replicate (in the presence of the necessary helper virus functions, but without any requirement for AAV functions provided *in trans)* an AAV particle must comprise a rep gene and a cap gene (so-called *"pseudo-wild type rcAAV"*). However, an AAV particle that comprises a rep gene but does not comprise a cap gene can replicate when co-infected in a host cell with an AAV particle that comprises a cap gene, and an AAV particle that comprises a cap gene but does not comprise a rep gene can replicate when co-infected in a host cell with an AAV particle that comprises a rep gene. Thus, the term *"rcAAV"* encompasses *"cap-deficient rcAAV",* but also *"pseudo-wild type rcAAV"* and *"rep-deficient rcAAV"*). Pseudo-wild type rcAAV may (in the presence of helper virus functions) be able to replicate in a host cell without co-infection by another AAV particle, and produce progeny virus. Pseudo-wild type rcAAV may comprise a rep gene and a cap gene flanked by ITR sequences.

All species of rcAAV are undesirable in an rAAV preparation. Cap-deficient rcAAV may replicate when co-infected with cap-containing AAV particles. Rep-deficient rcAAV may replicate when co-infected with rep-containing AAV particles, and pseudo-wild type rcAAV may replicate without AAV co-infection. If the rcAAV replicates, it can cause significant side effects. Accordingly, reducing or minimising the level of rcAAV produced is advantageous. As used herein, the term *"reducing the level of rcAAV"* refers to decreasing the number of rcAAV that are produced. As used herein the term *"minimising the level of rcAAV"* refers to reducing the level of rcAAV to the lowest level possible given the constraints of the method.

Using the methods of the invention, the rAAV genome that is packaged should not comprise either a rep gene or a cap gene as, in general, neither the rep gene nor the cap gene is linked (on the same plasmid, in close proximity) to an ITR. However, single recombination events could result in the generation of cap-deficient or rep-deficient rcAAV. To generate pseudo-wild-type rcAAV, two recombination events must occur, and this is highly unlikely.

Whether or not an rAAV particle comprises a genome comprising a rep gene, or a preparation of rAAV particles comprises a particle comprising a genome comprising a rep gene, may be determined using qPCR. Optionally, the amount of rcAAV is measured by determining the copy number of rep produced. Optionally, the amount of rcAAV is measured by determining the copy number of rep per cell *(i.e.* host cell) using qPCR.

A primer (or two primers) specific for the rep gene should be used in the qPCR. Optionally, the (or both) primer(s) is/are specific for (reverse and complementary to or identical to depending on whether the primer is a forward primer or a reverse primer) a region of at least 12, at least 14, at least 16, or at least 18 of nucleotides 321-2252 of SEQ ID NO: 1, which encode the four Rep proteins. Optionally, the primer is specific for rep 40. Optionally, the primer is specific for rep 52. Optionally, the primer is specific for rep 68.

To determine the copy number of rep per cell using qPCR a second pair of primers should be used in the qPCR, which is specific for an endogenous gene (such as an endogenous housekeeping gene) in the host cell such as human albumin in HEK293 cells. Optionally, each primer or pair of primers is specific for (complementary to) a region of at least 8, at least 10, at least 12 or at least 15 nucleotides of the genomic sequence of human albumin. The copy number per cell may then be determined as the ratio of the copy number of rep to the copy number of the endogenous gene.

Alternatively, the above method to detect/measure rcAAV (pseudo-wild type and/or cap-deficient rcAAV) may be performed by cap gene-specific qPCR which can be used to detect rep-deficient rcAAV.

The methods or uses may provide an rAAV preparation. In such embodiments, the rAAV preparation preferably comprises a low level of rcAAV. A low level of rcAAV is generally a level of rcAAV that is lower than 1 rcAAV in 10⁷ rAAV, or is lower than that in an rAAV preparation that is produced using a method equivalent to that of a method of the invention, except that the vector plasmid comprises both at least one rep gene and at least one cap gene. The level of rAAV can be determined by using qPCR to determine the number of vector genomes, as described below in the section entitled *"high yield'.*

Optionally, the level of rcAAV is less than 1 rcAAV in 10⁷ recombinant AAV, less than 1 rcAAV in 10⁹ recombinant AAV, or less than 1 rcAAV in 10¹⁰ recombinant AAV. Optionally, the level of rcAAV is less than the level of rcAAV produced using an equivalent method except that the vector plasmid comprises both the at least one rep gene and at least one cap gene. Optionally, the level of rcAAV is measured using the qPCR assay above. Optionally, the level of rcAAV is measured using the qPCR assay after two or three generations of culturing. Optionally, the rcAAV is pseudo-wild type rcAAV. Optionally, the rcAAV is cap-deficient rcAAV. Optionally, the rcAAV is rep-deficient rcAAV. Optionally, the rcAAV comprises pseudo-wild type rcAAV, cap-deficient rcAAV and rep-deficient rcAAV.

The phrase *"method equivalent to that of a method of the invention"* is intended to refer to a method that is identical, except that the vector plasmid comprises both the at least one rep gene and at least one cap gene.

### A desired ratio of full to total particles

Also provided is a use of the two-plasmid system, the helper plasmid or the vector plasmid for producing an rAAV preparation having a desired ratio of full to total particles.

Also provided is a use of the two-plasmid system, the helper plasmid or the vector plasmid for controlling or maximising the ratio of full to total particles produced during rAAV production.

Also provided is a method for producing an rAAV preparation comprising:
(a) obtaining the two-plasmid system, the helper plasmid or the vector plasmid;
(b) transfecting a host cell with the two-plasmid system, the helper plasmid or the vector plasmid; and
(c) culturing the host cell under conditions suitable for rAAV production
wherein the rAAV preparation comprises a desired ratio of full to total particles.

Also provided is a method for controlling or maximising the ratio of full to total particles produced during rAAV production comprising:
(a) obtaining the two-plasmid system, the helper plasmid or the vector plasmid;
(b) transfecting a host cell with the two-plasmid system, the helper plasmid or the vector plasmid; and
(c) culturing the host cell under conditions suitable for rAAV production.

The method may further comprise a step of harvesting the rAAV to provide an rAAV preparation, optionally having a desired ratio of full to total particles.

The present inventors have determined that altering the ratio of helper plasmid to vector plasmid can be used to alter the ratio of full to total particles that are produced. Specifically, the inventors have determined that increasing the level of the vector plasmid relative to the helper plasmid decreases the ratio of full to total particles.

*"Full"* particles are rAAV particles comprising both a capsid and the intended vector genome, or at least a partial such genome as determined using the qPCR method described below. *"Empty"* particles (i.e. particles which are not full) comprise capsids but do not comprise a genome, or comprise only a partial genome (thereby not forming a complete rAAV particle) which is not detected using the qPCR method. However, the rAAV preparations may comprise both full particles and empty particles. Generally a low or minimised proportion of empty particles is desired. For example, if the rAAV are to be used in gene therapy, any empty particles will not comprise the entire expression cassette of interest and so will not be effective in therapy. On the other hand, there are circumstances where the presence of empty particles could be desirable. In some instances, and in some patient groups, it may be the case the empty particles behave as *"decoys"* to reduce the immune response in a patient to the administered rAAV particles (WO2013/078400). Furthermore, as will be discussed in more detail below, whilst increasing the level of the vector plasmid relative to the helper plasmid decreases the ratio of full to total particles, it also increases the yield of rAAV produced (and thereby in some cases the total number of full particles that are produced). Thus, even if the user of the method believes that a high full to total particle ratio is desirable, the user may use a lower proportion of helper plasmid if this leads to a greater yield. Alternatively, if the user, for example, implements a process change during development of a given product, e.g. switches from bioreactor A to bioreactor B resulting in different full to total particle ratios when keeping the plasmid ratio constant, the plasmid ratio can be altered to compensate for or offset the effect of the process change to ensure consistent full to total particle ratio throughout development, as this ratio is a critical quality parameter of a rAAV batch that needs to be controlled and kept comparable from batch to batch.

Accordingly, an advantage of the methods and uses is that they allow the user the flexibility to determine a desirable ratio of full to total particles and modify the ratio of helper plasmid to vector plasmid in order to achieve that desired ratio of full to total particles.

The ratio of full to total particles may be expressed herein as the percentage of the total number of particles (capsids) that notionally comprise a vector genome or at least a partial such genome (assuming one (partial) genome per capsid) as determined using the following qPCR assay. qPCR is carried out using a pair of primers that are that are able to amplify at least a region of the promoter of the expression cassette. Optionally, at least one of the primers is specific for (reverse and complementary to or identical to depending whether the primer is a forward primer or a reverse primer) a region of at least 12, at least 14, at least 16, or at least 18 nucleotides of the promoter of the expression cassette. Optionally, one primer is specific for the start of the promoter (the first at least 12 nucleotides of the promoter) and the other primer is specific for a region of the expression cassette that is 150 base pairs from the binding site of the first primer.

The ratio of full to total particles (as measured as a percentage of the total number of particles that are full particles) may be determined using qPCR to determine the number of vector genomes (as discussed in the previous paragraph), and using a capsid-specific ELISA to measure the total number of particles. For example, the capsid-specific ELISA may comprise exposing the rAAV preparation to an antibody that binds to the capsid protein. If, for example, the vector plasmid comprises a cap gene that encodes a capsid from an AAV2 serotype, the antibody may be an antibody that binds to the AAV2 capsid. For example, the user may coat a plate with an antibody that is specific for the capsid.

The user may then pass the rAAV preparation over the surface of the plate. The particles will bind to the antibody and be immobilised on the plate. The plate may then be washed to remove contaminants. The amount of particle present can then be detected by addition of a detection antibody that can bind to the capsid and is conjugated to a detection agent such as streptavidin peroxidase. The amount of particle present will be proportional to the colour change obtained when the streptavidin peroxidase is exposed to the chromogenic substrate TMB (tetramethylbenzidine).

Optionally, the desired ratio of full to total particles (expressed as a percentage of the total number of particles that notionally comprise a vector genome) is at least 2%, at least 3%, at least 4%, at least 5%, at least 7%, at least 10% or at least 15% (as measured on bulk product prior to harvesting, purifying and/or concentrating the rAAV preparation). In many cases, increasing the full to total particle ratio is advantageous, and the present inventors have determined that aiming for a full to total particle ratio of at least 2%, at least 3%, at least 4%, at least 5%, at least 7%, at least 10% or at least 15%. achieves a good balance between maintaining a high full to total particle ratio, whilst also achieving a good yield. Optionally, the desired ratio of full to total particles is a ratio of full to total particles that is at least 20% or at least 30% of the ratio of full to total particles achieved using an equivalent method with a ratio of helper plasmid to vector plasmid of 1.8:1.

Optionally, the ratio of helper plasmid to vector plasmid that is used, selected or adjusted to is between 3:1 to 1:10, between 1.5:1 and 1:9, between 1.4:1 and 1:8, between 1.3:1 and 1:7; between 1.2:1 and 1:6; between 1.1:1 and 1:5; between 1:1 and 1:4; or between 1:1.5 and 1:3. Optionally, the ratio of helper plasmid to vector plasmid comprises a molar excess of vector plasmid.

### High yield

Also provided is a use of the two-plasmid system, the helper plasmid or the vector plasmid for producing an rAAV preparation at a high or desired yield.

Also provided is a use of the two-plasmid system, the helper plasmid or the vector plasmid for increasing, optimising or maximising the yield of rAAV produced during rAAV production.

Also provided is a method for producing an rAAV preparation comprising:
(a) obtaining the two-plasmid system, the helper plasmid or the vector plasmid;
(b) transfecting a host cell with the two-plasmid system, the helper plasmid or the vector plasmid; and
(c) culturing the host cell under conditions suitable for rAAV production
wherein the rAAV preparation is at a high or desired yield.

Also provided is a method for increasing, optimising or maximising the yield of rAAV produced during rAAV production comprising:
(a) obtaining the two-plasmid system, the helper plasmid or the vector plasmid;
(b) transfecting a host cell with the two-plasmid system, the helper plasmid or the vector plasmid; and
(c) culturing the host cell under conditions suitable for rAAV production.

The method may further comprise a step of harvesting the rAAV vector to provide a rAAV preparation, optionally at a high or desired yield. The term *"yield"* refers to the amount of AAV particles that are prepared in the methods or uses of the invention. The *"yield"* may be expressed as the number of vector genomes (vg) per ml of medium, as measured before (as measured on bulk product prior to harvesting, purifying and/or concentrating the rAAV preparation). The number of vector genomes can be measured as discussed under the heading *"a desired ratio of full to total particles", i.e.* the yield of rAAV (such as rAAV particles) may be determined by using qPCR to quantify the number of nucleic acid sequences comprising a cassette comprising a promoter sequence (vg).

The present inventors have shown that modifying the ratio of helper plasmid to vector plasmid may be used to increase the yield (i.e. quantity of vgs or vg/ml produced) of a method that produces rAAV. Having the highest yield possible is clearly advantageous, as it reduces the amount of resources required to produce the rAAV. However, under some conditions increasing the yield to the highest possible level may result in a decrease in the ratio of full to total particles. Thus, if the user is producing the rAAV for an application where maximising the ratio of full to total particles is important, then he may opt to use a desired yield that is not the highest possible yield by using a helper plasmid to vector plasmid ratio that optimises in favour of the ratio of full to total (i.e. "%age full") particles.

As used herein the term *"maximising"* the yield refers to aiming for the highest possible yield within the limitations of the methods of the invention. As used herein the term *"optimising"* refers to increasing the yield, but aiming for a desired yield which may not be the maximum possible yield if, for example, the user is keen to ensure a high ratio of full to total particles (i.e. minimising the proportion of empty particles).

As used herein the term *"high yield"* generally refers to a yield that is greater than 2 × 10¹⁰vg/ml, or at least 2-fold greater than the yield achieved using an equivalent method with a ratio of helper plasmid to vector plasmid of 1.8:1. An *"equivalent method"* is a method that is identical, except that the ratio of helper plasmid to vector plasmid is 1.8:1.

Optionally, the high or desired yield is a yield greater than 2 × 10¹⁰ vg/ml, greater than 4 × 10¹⁰ vg/ml, greater than 6 × 10¹⁰ vg/ml, greater than 8 × 10¹⁰ vg/ml, greater than 1 × 10¹¹ vg/ml, greater than 2 × 10¹¹ vg/ml, or greater than 4 × 10¹¹ vg/ml.

Optionally, the high or desired yield is a yield that is at least 2-fold, at least 4-fold, at least 5-fold, or at least 6-fold higher than the yield achieved using an equivalent method with a ratio of helper plasmid to vector plasmid of 1.8:1.

Optionally, the ratio of helper plasmid to vector plasmid that is used, selected or adjusted to is between 3:1 to 1:10, between 1.5:1 and 1:9, between 1.4:1 and 1:8, between 1.3:1 and 1:7; between 1.2:1 and 1:6; between 1.1:1 and 1:5; between 1:1 and 1:4; or between 1:1.5 and 1:3. Optionally, the ratio of helper plasmid to vector plasmid comprises a molar excess of vector plasmid compared to helper plasmid.

### Balance between high yield and high full to total particle ratio

As discussed above, the ratio of full to total particles and the yield may be affected by altering the ratio of helper plasmid to vector plasmid used in the methods. Thus, the user may adjust the ratio of helper plasmid to vector plasmid to a suitable ratio for obtaining the desired full to total particle ratio. Optionally, the methods or uses may comprise a step of selecting a ratio of helper plasmid to vector plasmid. Altering the ratio of helper plasmid to vector plasmid, or selecting a ratio of helper plasmid to vector plasmid, may be achieved by simply mixing the helper plasmid and the vector plasmid in different relative proportions.

The user may need to carry out a test method to determine the ratio of helper plasmid to vector plasmid that is required to obtain the desired ratio of full to total particles, but this is not burdensome. For example, the skilled person may perform a small scale experiment to determine the required ratio of helper plasmid to vector plasmid, and then this ratio can be used in a larger scale production process. Optionally, the step of selecting a ratio of helper plasmid to vector plasmid may comprise performing small scale test runs of the method of the invention using different ratios of helper plasmid to vector plasmid.

Optionally, the ratio of helper plasmid to vector plasmid is selected or adjusted to a ratio that achieves a balanced yield versus full to total particle ratio. Optionally, the ratio of helper plasmid to vector plasmid is selected or adjusted to a ratio that achieves a maximum yield of rAAV (*i.e.* vgs) with the minimum yield of particles achievable at such maximum yield of rAAV, *i.e.* the user maximises the yield, and then determines the helper plasmid to vector plasmid ratio that achieves that yield while providing the highest ratio of full to total particles.

Optionally, the ratio of helper plasmid to vector plasmid that is used, selected or adjusted to is between 3:1 to 1:10, between 1.5:1 and 1:9, between 1.4:1 and 1:8, between 1.3:1 and 1:7; between 1.2:1 and 1:6; between 1.1:1 and 1:5; between 1:1 and 1:4; or between 1:1.5 and 1:3. Optionally, the ratio of helper plasmid to vector plasmid comprises a molar excess of vector plasmid.

### Transfecting, culturing and harvesting

The methods of the invention may comprise steps of obtaining the two-plasmid system of the invention, the helper plasmid of the invention or the vector plasmid, transfecting a host cell with the two-plasmid system, the helper plasmid or the vector plasmid, and culturing the host cell under conditions suitable for recombinant AAV production.

Transfecting a host cell with the two-plasmid system, the helper plasmid or the vector plasmid, may comprise exposing the host cell to the two-plasmid system, the helper plasmid or the vector plasmid in conditions suitable for transfection. For example, the user of the method may add a transfection agent (addition of a transfection agent would be considered to be a condition suitable for transfection). Alternatively, calcium phosphate transfection, electroporation or cationic liposomes could be used. Optionally, the step of transfecting the host cell takes place when the host cell has grown to confluence.

Culturing the host cell under conditions suitable for rAAV production refers to culturing the host cell under conditions at which it can grow and AAV can replicate. For example, the host cell may be cultured at a temperate between 32°C and 40°C, between 34°C and 38°C, between 35°C and 38°C or around 37°C. Optionally, the host cell may be cultured in the presence of a complete cell culture medium such as Dulbecco's Modified Eagle's Medium (DMEM). A completed cell culture medium is a medium that provides all the essential nutrients required for growth of the host cell. Optionally, the complete cell culture medium is supplemented with serum, such as fetal bovine serum or bovine serum albumin.

Optionally, the host cell is a host cell such as those defined above under the heading *"host cell".* Optionally, the host cell is selected from the group consisting of a HEK293T cell, a HEK293 cell, a HEK293EBNA cell, a CAP cell, a CAP-T cell, an AGE1.CR cell, a PerC6 cell, a C139 cell, and an EB66 cell. Optionally, the host cell is a cell that expresses a functional E1A/B protein.

The method may further comprise a step of purifying the rAAV. In general, a step of purifying the rAAV will involve increasing the concentration of the rAAV compared to other components of the preparation. Optionally, the step of purifying the rAAV results in a concentrated rAAV preparation. Optionally, the step of purifying the rAAV results in an isolated rAAV.

Any suitable purification method may be used. Optionally, the step of purifying the rAAV is carried out using a technique selected from the group consisting of gradient density centrifugation (such as CsCl or Iodixanol gradient density centrifugation), filtration, ion exchange chromatography, size exclusion chromatography, affinity chromatography and hydrophobic interaction chromatography.

Optionally, the method comprises further concentrating the rAAV using ultracentrifugation, tangential flow filtration, or gel filtration.

Optionally, the method comprises formulating the rAAV with a pharmaceutically acceptable excipient. The pharmaceutically acceptable excipients may comprise carriers, diluents and/or other medicinal agents, pharmaceutical agents or adjuvants, etc. Optionally, the pharmaceutically acceptable excipients comprise saline solution. Optionally, the pharmaceutically acceptable excipients comprise human serum albumin.

### Recombinant AAV (rAA V) preparations

A recombinant AAV (rAAV) preparation may be obtainable by a method of the invention. Recombinant AAV preparation may be obtained by a method of the invention.

The recombinant AAV preparations obtainable or obtained by the methods of the invention are advantageous, as they generally comprise a low level of rcAAV, and/or have a desired ratio of full to total particles. The sections entitled *"a desired ratio of full to total particles", "balance between high yield and high full to total particle ratio"* and *"a low level of replication competent AAV (rcAAV)"* provide further details of what is meant by the terms low level of rcAAV and a desired ratio of full to total particles.

**Sequence listing table**

| Sequence identity number | Sequence |
|---|---|
| 1 | Genome of AAV2 (AF043 3 03.1) |
| 2 | Genome of AdV5 (AC_000008.1) |
| 3 | Genome of AdV2 (AC_000007.1) |
| 4 | Helper gene region |
| 5 | Alternative AdV5 genome (AY339865.1) |

### EXAMPLES

### Example 1 - construction of helper and vector plasmids of ('trans-split') two-plasmid system

### Helper plasmid

To make the helper plasmid, nucleotides 200-4497 of wild type AAV2 (Genbank accession number AF043303; SEQ ID NO: 1) containing the rep and cap genes were cloned into the pUC19 (Yanisch-Perron et al (1985), Gene, 33:103-119). Next, AAV2 nucleotides 4461-4497 were deleted to minimise sequence homology with the vector plasmid (the construction of which is described below). To prevent Rep 78 expression while maintaining Rep 68 expression, the intron within the cloned rep gene was deleted. In order to provide for expression of Rep 52 following deletion of the intron, AAV2 nucleotides corresponding to Rep 52 including the p19 promoter were cloned immediately 3' of the intron-less Rep 68 gene. The majority of cap gene sequences were then deleted.

The two p40 promoters (one in each of the Rep 68- and Rep 52-encoding rep gene duplications) were rendered non-functional by ablation of the TATA boxes (mutation of the T corresponding to AAV2 position 1823 and AAG corresponding to AAV2 positions 1826-1828 to C and CTC respectively).

The resulting 'rep cassette' was then cloned into a plasmid comprising an 8342-nucleotide stretch comprising functional VA RNA I and II, E2A and E4 genes from adenovirus (i.e. helper virus) serotype 5 (SEQ ID NO: 4). The plasmid backbone, containing kanamycin resistance gene and bacterial origin of replication, was about 2.2kb in length, resulting in a helper plasmid of 14021 nucleotides.

Figure 2 is a schematic of the helper plasmid showing the main features. For the 'rep cassette' the portions of AAV2 sequence, by reference to the nucleotide positions of SEQ ID NO: 1, are indicated.

### Vector plasmid

To make the vector plasmid, nucleotides 200-4497 of wild type AAV2 (Genbank accession number AF043303; SEQ ID NO: 1) containing the rep and cap genes were cloned into the pUC19. Two portions of rep gene sequence, between the p5 and p19 and between the p19 and p40 promoters respectively, were then deleted to prevent Rep protein expression whilst maintaining the downstream cap gene under the regulation of the three native promoters. As for the helper plasmid described above, AAV2 nucleotides 4461-4497 were deleted to minimise sequence homology with the helper plasmid.

To minimise or prevent translation of undesired products from potential initiation codons within the remaining promoter region, four ATG codons and one GTG codon were removed: ATGs at positions corresponding to AAV2 nucleotides 321-323, 955-957 and 993-995, and a GTG corresponding to AAV2 nucleotides 1014-1016, were deleted, whilst ATG at AAV2 nucleotides 766-768 was mutated to ATT.

The AAV2 cap gene encoding the VPs 1, 2 and 3 immediately 3' of the above promoter region was replaced with the corresponding sequence of an engineered cap gene, which cap gene is 3 nucleotides (equating to one additional encoded amino acid) longer than the AAV2 cap gene. The resulting 'promoter-cap' cassette was cloned into a plasmid backbone containing kanamycin resistance gene and bacterial origin of replication. Into this backbone was inserted an expression cassette, containing the transgene sequence linked to promoter and polyA transcription regulatory elements, flanked by AAV2 sequence comprising the native AAV2 ITRs (AAV2 nucleotides 1-145 and 4535-4679).

In the case of vector plasmid comprising the 2672-nucleotide (ITR-to-ITR) Factor IX expression cassette, as used in Example 2, the plasmid backbone was approximately 2.3kb in length, resulting in a 8525-nucleotide vector plasmid. In all subsequent work, including production of the rAAV batches of Example 3, the plasmid backbone was reduced in length to approximately 2kb. The α-galactosidase A (GLA) expression cassette (Example 3) was 2297 nucleotides in length.

Figure 3 is a schematic of the vector plasmid showing the main features. For the 'promoter-cap' cassette, the portions of AAV2 sequence, by reference to the nucleotide positions of SEQ ID NO: 1, are indicated.

### Example 2 - two-plasmid system: comparison of helper:vector plasmid ratios

### Cell cultivation

HEK293T cells were maintained in adherent culture under standard conditions at 37°C, 95% relative humidity, and 5% v/v CO₂ in Dulbecco's Modified Eagle's Medium (DMEM) supplemented with 10% Fetal Bovine Serum (FBS) and 1% GlutaMax^{™} (L-alanine-L-glutamine dipeptide). Cellular confluence during passaging ranged from 40-95%.

### Transfection of HEK293T cells and preparation of cell lysates

HEK293T cells were transfected with helper plasmid and vector plasmid (the latter comprising the engineered cap gene and Factor IX expression cassette; Example 1) using different molar plasmid ratios (from helper:vector 3:1 to 1:6) while maintaining the total plasmid DNA amount. 1.5 × 10⁵ viable cells per cm² culture area were seeded in 6-cm dishes in a volume of 3 ml DMEM, 10% FBS, 1% GlutaMax^{™} the day before transfection resulting in 60-70% confluency on the day of transfection. PEI-DNA complexes were prepared in DMEM without supplements using the linear polyethylenimine transfection reagent PEIpro^{™} (Polyplus) according to the manufacturer's manual. An amount of 6 µg total plasmid DNA and a PEI-to-DNA ratio of 2:1 was maintained independent of the applied plasmid combination ratios. Cells were cultured until day 3 post-transfection, harvested in the medium and lysed by three freeze-thaw cycles (-80°C and 37°C). Cell debris was removed by centrifugation at 10,000 × g for 5 min.

### Quantification of rAA V vector genomes

The AAV vector genome assay is based on a quantitative polymerase chain reaction (qPCR) specific for the promoter sequence of the rAAV expression cassette. In principle, the qPCR primers can be designed to bind any part of the recombinant AAV genome which is not common to wild type AAV genomes, but is it recommended against using primer template sequences very close to the ITRs as doing so can lead to an exaggerated vector genome titre measurement.

Cell lysate test samples were subjected to a nuclease treatment procedure in order to remove non-packed vector genomes prior to performing the qPCR. To that aim the samples were pre-diluted 1:250 in nuclease-free water containing 0.125% Pluronic F-68. 25 µl of the pre-dilution were used for the digest with 2 units of Turbo DNase (ThermoFisher Scientific, Waltham, USA) and 1x Turbo DNase reaction buffer, resulting in a total reaction volume of 29 µl. Incubation was performed for 1 h at 37°C. Afterwards, 1 volume of 0.4 M NaOH was added and the samples were incubated for 45 min at 65°C. 1035 µl nuclease-free water supplemented with 0.1% Pluronic F-68 were added together with 30 µl 0.4 M HCl. To control for the quality of the Turbo DNase digest, a trending control containing unpurified cell lysate with a known AAV vector genome titre and spike-in controls using plasmid DNA carrying the promoter sequence were measured in parallel.

Per sample, 12.5 µl QuantiFast SYBR Green PCR Master Mix (Qiagen, Venlo, Netherlands) were mixed with 0.75 µl of qPCR primer working stock solution (containing 10µM of each primer) and filled up to a volume of 20 µl with nuclease-free water. 5 µl of Turbo DNase treated cell lysate or purified virus test sample were added to the mix (total reaction volume 25 µl, final primer concentration in the reaction 300 nM each) and qPCR was performed in a CFX 96 Touch Real Time PCR cycler (Bio-Rad Laboratories Inc., Hercules, USA) with following program steps: 95°C 5 min; 39 cycles (95°C 10 s, 60°C 30 s, plate read); 95°C 10 sec; 60-95°C (+0.5°C/step), 10 sec; plate read. To control for the quality of the qPCR, a trending control with known AAV vector genome titre was measured in parallel. To check for contaminations, a no template control (NTC, 5 µl H₂O) was also included. Standard row, test samples and controls were measured in triplicates for each dilution. Purified virus test samples and trending control were generally measured in 3 different dilutions in EB buffer (10 mM Tris-Cl, pH 8.5). Turbo DNase treated cell lysate test samples were directly used in the qPCR without any further dilution. Data were analysed using the CFX ManagerTM Software 3.1 (Bio-Rad Laboratories Inc.).

Melting curve analysis confirmed the presence of only one amplicon. Amplification results in nascent double stranded DNA amplicons detected with the fluorescent intercalator SYBR Green to monitor the PCR reaction in real time. Known quantities of the promoter genetic material, in the form of a linearised plasmid, were serially diluted to create a standard curve and sample vector genome titre was interpolated from the standard curve.

### Quantification of rAAV particles (capsids)

The AAV2 Titration ELISA method is a measure of total AAV particles (capsids) and is based on a commercially available kit (Progen^{™}, Heidelberg, Germany; catalogue number PRATV). This sandwich immunometric technique utilises monoclonal antibody A20 (Wobus et al (2000), J Virol, 74:9281-9293) for both capture and detection. The antibody is specific for a conformational epitope present on assembled capsids of serotypes AAV2, AAV3, and the engineered capsid used in these experiments.

The AAV2 Titration ELISA kit was used to quantify total AAV particles in cell lysates and purified virus preparations according to the manufacturer's instructions. In brief, 100 µl diluted AAV2 Kit Control, test samples, or trending control of engineered capsid with known total particle titre were added per well of a microtiter plate coated with monoclonal antibody A20 and incubated for 1 h at 37°C. Standard row, test samples and controls were measured in duplicates for each dilution. In a second step, 100 µl of pre-diluted biotin-conjugated monoclonal antibody A20 (1:20 in Assay Buffer [ASSB]) were added and incubated for 1 h at 37°C. Then, 100 µl of a pre-diluted streptavidin peroxidase conjugate (1:20 in ASSB) were added and incubated for 1 h at 37°C. 100 µl substrate solution (TMB [Tetramethylbenzidine]) were added and after incubation for 15 min, the reaction was stopped using 100 µl stop solution. The absorbance was measured photochemically at 450 nm using the SpectraMax M3 microplate reader (Molecular Devices, San Jose, USA). Data was analysed with the SoftMax Pro 7.0 Software (Molecular Devices).

The test samples were diluted into the assay range and AAV total particle concentrations were determined by interpolation using the standard curve which was prepared using the provided AAV2 Kit Control. ASSB was used as blank.

### Vector genome to total particle ratio

The ratio of vector genomes to total AAV particles is expressed as a percentage. This is based on the vector genome titre (determined by qPCR, as described above) and the number of total AAV particles (determined by the capsid ELISA, as described above).

### Results

As apparent from Figure 5A and B, elevation of the proportion of vector plasmid led to both higher particle and vector genome yields. However, the relative increase in particle (capsid) yields was more pronounced and the plateau in vector genome yields was achieved earlier. These observations were responsible for a gradual decrease in the vector genome to total particles ratio (Figure 5C). A plasmid ratio of helper:vector 1:3 resulted in an almost maximal increase in vector genome titre of approximately 3.5-fold compared to the previously applied 1.8:1 ratio, whereas the particle titer was increased approximately 8-fold.

For comparison, two plasmids in a conventional "non-split" configuration (i.e. wherein one plasmid contained the same AdV helper and vector genome (Factor IX expression cassette) sequences, and the other plasmid contained the same cap gene sequence in addition to an AAV2 rep cassette containing all four rep genes such that Rep and Cap functions are not split between the two plasmids) were transfected in an established molar plasmid ratio of 1.6:1, revealing considerably lower particle and vector genome yields.

Subsequent confirmatory sequencing of the helper plasmid revealed a mutation in the codon within the Rep 68-encoding sequence corresponding to positions 429-431 of AAV2 (SEQ ID NO: 1), resulting in a leucine to phenylalanine substitution at amino acid position 37 of the Rep 68 protein. Correction of the helper plasmid sequence to restore the leucine-encoding wild type AAV2 codon resulted in an increased vg/ml yield and an approximately two-fold increase in vector genomes to total particles ratio (i.e. % full particles), when directly compared against the 'mutated' helper plasmid. The corrected helper plasmid sequence was used in all subsequent work, including production of the rAAV batches analysed in Example 3.

### Example 3 - determination of replication competent AAV (rcAAV) frequency in rAAV produced by trans-split two-plasmid system

### rcAA V testing

rAAV purified bulk drug substance, manufactured in large-scale using the iCellis^{®} bioreactor with cells transfected with the two-plasmid system and purified using a number of downstream processes to remove product impurities, were subjected to a limit test for the presence of rcAAV. The rAAV batches were produced using vector plasmid comprising the engineered cap gene and (i) α-galactosidase A expression cassette (as mentioned in Example 1) or (ii) a Factor IX expression cassette identical to that mentioned in Example 1 except for a different partially codon-optimised Factor IX coding sequence. The two plasmid system used to produce these rAAV batches used the shortened vector plasmid backbone and corrected helper plasmid sequence as mentioned in Examples 1 and 2, respectively. In the limit test, HEK293 cells are transduced by the rAAV at its most concentrated form (drug substance post-purification and pre-formulation) in the presence or absence of wild-type adenovirus. Three successive rounds of virus amplification are conducted as described below.

Cells are seeded into T75/T175 flasks. At 80% +/- 10 confluency and >90% viability, cells are transduced with and without helper wild-type Adenovirus serotype 5 ("Ad5wt") at a multiplicity of infection (MOI) of 7.8×10³ and the rAAV product to be tested. Following 2 days incubation, the cells are harvested by detaching mechanically. Three sets of cells are collected and centrifuged from round one: 1) retained for back up (stored in case repeat analysis required); 2) for DNA purification and analysis; 3) for a second round of transduction. Cells for purification and following rounds of transduction are lysed by freeze/thaw (3x) in dry ice/ethanol to water bath 37°C, and Ad5wt inactivated by incubation at 56°C degrees for 30 mins and centrifugation at 1500g for 2 mins. The cells seeded for second transduction are transduced with lysate from first harvest, with and without Ad5wt, and the entire process is repeated to collect samples from harvest 2. The cells seeded for the third transduction are transduced with lysate from second harvest, with and without Ad5wt and the entire process repeated, to collect samples from harvest 3.

Genomic DNA is extracted from each of the three amplification steps using DNeasy^{®} Tissue kit (Qiagen) and quantified by UV at A260/A280. The presence of rcAAV is detected by real-time quantitative PCR (qPCR): DNA is isolated from the HEK293 cells and two sequences are amplified from the isolated DNA using 1) a primer specific for the rep-encoding sequence of AAV2 ("rep"), and 2) an endogenous housekeeping gene in HEK293 cells (human albumin; hAlb).

DNA at a concentration of 100ng is added to the 25µl qPCR reaction. The copy number of rep is calculated relative to the plasmid standard range (from 100 to 1×10⁸ copies per qPCR reaction) and relative copy numbers of the rep sequence per cell calculated as the ratio of the rep copies and the human albumin sequence copies, multiplied by two (hAlb, two copies/cell). Increased levels of the rep sequence per cell in consecutive amplification rounds indicates the presence of rcAAV.

The positive control is wild-type AAV2 with Adenovirus (Ad5wt); they are tested alone or in the presence of the rAAV product sample as inhibition controls. The control confirms the detection limit for the test (LOD) being between 10 rcAAV 1×10¹⁰ to 10 rcAAV per 1×10¹¹ vg of rAAV product sample for AAV2. The LOD is an assay and product-dependent parameter. The negative controls consist of non-infected cells (with or without adenovirus) and cells transduced with wt AAV2 (without adenovirus). Replication is established when the rep sequence copy number per cell is >10 in at least 1 of the 3 amplification rounds, and in subsequent rounds if more than one. Absence of rep is reported as 'no replication', meaning <10 rcAAV in 1×10¹⁰ *to* 1×10¹¹ vg of test sample. Detection of rep sequence is reported as 'replication', i.e. detection of rcAAV in 1×10¹⁰ *to* 1×10¹¹ vg of test sample.

### Results

The limit test was performed on several batches of each of the α-galactosidase A- and Factor IX-containing rAAV products. Replication competent positive controls of wild-type AAV2 coinfection with Ad5wt confirmed rcAAV detection by the assay. The rcAAV (AAV2) positive controls spiked into the rAAV product sample demonstrated no sample inhibition. The positive controls confirmed that the detection limit for the test (LOD) was 10 rcAAV per 1×10¹¹ vg of Factor IX-containing rAAV product sample, and 10 rcAAV per 1×10¹⁰ or 1×10¹¹ vg of α-galactosidase A-containing rAAV product sample. The negative controls of non-infected cells (with or without adenovirus) and cells transduced with wt AAV2 (without adenovirus) were negative for rcAAV.

Thus, the tested rAAV product batches produced using the two-plasmid system were shown to contain <1 rcAAV per 1×10¹⁰ vg of Factor IX-containing rAAV product, and <1 rcAAV per 1×10⁹ - 1×10¹⁰ vg of α-galactosidase A-containing rAAV product.

### Example 4

The two-plasmid system was used in connection with other transgenes and capsids, and relevant data are presented in Figures 6-9.

## Claims

1. A helper plasmid comprising at least one adeno-associated virus (AAV) rep gene encoding at least one functional AAV Rep protein and at least one helper virus gene, and which does not comprise a cap gene encoding a functional set of Cap proteins.

2. A two-plasmid system comprising the helper plasmid of claim 1 and a vector plasmid.

3. The two-plasmid system of claim 2, wherein:
(i) the vector plasmid comprises:
(a) an AAV cap gene encoding at least one functional AAV Cap protein; or
(b) at least one AAV cap gene promoter, a cloning site operably linked to the AAV cap gene promoter, and an expression cassette flanked on at least one side by an inverted terminal repeat (ITR);
wherein the vector plasmid does not comprise a rep gene encoding a functional Rep protein and the expression cassette comprises a transgene operably linked to at least one regulatory control element; and/or
(ii) the ratio of helper plasmid to vector plasmid is between 3:1 to 1:10, between 1.5:1 and 1:9, between 1.4:1 and 1:8, between 1.3:1 and 1:7; between 1.2:1 and 1:6; between 1.1:1 and 1:5; between 1:1 and 1:4; or between 1:1.5 and 1:3.

4. The two-plasmid system or helper plasmid of any one of the preceding claims, wherein the at least one AAV rep gene comprises a gene encoding a functional Rep 52 protein, at least one gene encoding a functional Rep 40 protein, and a gene encoding a functional Rep 68 protein.

5. The two-plasmid system or helper plasmid of any one of the preceding claims, wherein:
(i) the at least one AAV rep gene comprises a gene encoding a functional Rep 52 protein and the gene encoding a functional Rep 52 protein comprises a nucleic acid sequence having at least 95%, at least 98%, at least 99%, or 100% identity to the full length or a fragment of at least 800, at least 900, at least 1000, or at least 1100 nucleotides in length of nucleotides 993-2186 of SEQ ID NO: 1, or to a corresponding stretch of nucleotides in a different serotype of AAV; and/or
(ii)
(a) the at least one AAV rep gene comprises a gene encoding a functional Rep 40 protein and the gene encoding a functional Rep 40 protein comprises a nucleic acid sequence having at least 95%, at least 98%, at least 99%, or 100% identity to the full length or to a fragment of at least 600, at least 700, at least 800, or at least 900 nucleotides in length of a stretch of nucleotides corresponding to nucleotides 993-2252 minus nucleotides 1907-2227 of SEQ ID NO: 1, or to corresponding stretches of nucleotides in a different serotype of AAV; and/or
(b) the at least one AAV rep gene comprises a gene encoding a functional Rep 40 protein and the gene encoding a functional Rep 40 protein comprises a nucleic acid sequence having at least 95%, at least 98%, at least 99%, or 100% identity to the full length or to a fragment of at least 900, at least 1000, at least 1100, or at least 1200 nucleotides in length of nucleotides 993-2252 of SEQ ID NO: 1, or to a corresponding stretch of nucleotides in a different serotype of AAV; and/or
(iii) the at least one AAV rep gene comprises a gene encoding a functional Rep 68 protein and the gene encoding a functional Rep 68 protein comprises a nucleic acid sequence having at least 95%, at least 98%, at least 99%, or 100% identity to the full length or to a fragment of at least 1000, at least 1400, at least 1500, or at least 1600 nucleotides in length of a stretch of nucleotides corresponding to nucleotides 321-2252 minus nucleotides 1907-2227 of SEQ ID NO: 1, or to corresponding stretches of nucleotides in a different serotype of AAV.

6. The two-plasmid system or helper plasmid of any one of the preceding claims, wherein:
(i) the helper plasmid does not comprise a gene encoding a functional Rep 78 protein; and/or
(ii) the helper plasmid does not comprise a contiguous sequence of at least 1700, at least 1800, or all 1866 nucleotides corresponding to a contiguous stretch of nucleotides of equivalent length comprised within nucleotides 321-2186 of SEQ ID NO: 1 or within a corresponding stretch of nucleotides in a different serotype of AAV; and/or
(iii) the at least one AAV rep gene does not comprise a functional internal p40 promoter; and/or
(iv) the helper plasmid does not comprise a contiguous stretch of exclusively cap gene sequence of more than 60 nucleotides.

7. The two-plasmid system or helper plasmid of any one of the preceding claims, wherein:
(i) the helper plasmid comprises at least one helper virus gene and the at least one helper virus gene comprises a viral associated (VA) nucleic acid, an E2A gene and an E4 gene; and/or
(ii) the helper plasmid comprises at least one helper virus gene and the at least one helper virus gene comprises a VA nucleic acid, an E2A gene and an E4 gene and the E4 gene is not located between the VA nucleic acid and the E2A gene; and/or
(iii) the helper plasmid comprises at least one helper virus gene and the helper plasmid is less than 25000 bp, less than 20000 bp, less than 15000 bp, less than 14500 bp, between 10000 bp and 25000 bp, between 10000 bp and 20000 bp, between 12000 bp and 15000 bp, or 14021 bp in length; and/or
(iv) the helper plasmid does not comprise a contiguous sequence of at least 2000, at least 2500, at least 3000, or all 3427 nucleotides of a contiguous stretch of nucleotides of equivalent length comprised within nucleotides 194-3620 of SEQ ID NO: 2, or a corresponding stretch of nucleotides in a different serotype of adenovirus; and/or
(v) the helper plasmid does not comprise a contiguous sequence of at least 50, at least 60, or all 69 nucleotides of a contiguous stretch of nucleotides of equivalent length comprised within nucleotides 4032-4100 of SEQ ID NO: 2, or a corresponding stretch of nucleotides in a different serotype of adenovirus; and/or
(vi) the helper plasmid does not comprise a contiguous sequence of at least 200, at least 300, at least 350, or all 363 nucleotides of a contiguous stretch of nucleotides of equivalent length comprised within nucleotides 4051-4413 of SEQ ID NO: 1, or a corresponding stretch of nucleotides in a different serotype of AAV.

8. The two-plasmid system or helper plasmid of any one of the preceding claims, wherein:
(i) the helper plasmid does not comprise a contiguous sequence of at least 400, at least 500, at least 600, or all 647 nucleotides of a contiguous stretch of nucleotides of equivalent length comprised within nucleotides 2301-2947 of SEQ ID NO: 1, or a corresponding stretch of nucleotides in a different serotype of AAV; and/or
(ii) the helper plasmid comprises at least one helper virus gene and the at least one helper virus gene comprises a VA nucleic acid, an E2A gene and an E4 gene, and the VA nucleic acid, the E2A gene and the E4 gene are comprised within a contiguous portion of fewer than 15000, fewer than 12000, fewer than 10000, or fewer than 9000 nucleotides; and/or
(iii) the helper plasmid does not comprise a contiguous sequence of at least 15000, at least 20000, at least 22000, or all 22137 nucleotides of a contiguous stretch of nucleotides of equivalent length comprised within nucleotides 10619-32756 of SEQ ID NO: 2, or a corresponding stretch of nucleotides in a different serotype of adenovirus; and/or
(iv) the helper plasmid and/or the vector plasmid does not comprise an artificial Rep binding site.

9. The two-plasmid system or helper plasmid of any one of the preceding claims, wherein the helper plasmid comprises at least one helper virus gene and the at least one helper virus gene is comprised in a contiguous stretch of the plasmid having at least 95%, at least 98%, at least 99%, or 100% identity to the full length or to a fragment of at least 6000 nucleotides, at least 7000 nucleotides, or at least 8000 nucleotides in length of SEQ ID NO: 4.

10. The two-plasmid system of any one of claims 2-9, wherein:
(i) the vector plasmid comprises an at least one AAV cap gene promoter, which comprises an AAV p40 promoter, a p5 promoter, and a p19 promoter; and/or
(ii) the vector plasmid does not comprise any dispensable translation initiation codons; and/or
(iii) the vector plasmid comprises a promoter region comprising one or more promoters, and in the promoter region:
(a) nucleotides corresponding to nucleotides 321-323 of SEQ ID NO: 1 are absent;
(b) nucleotides corresponding to nucleotides 766-768 of SEQ ID NO: 1 are not ATG;
(c) nucleotides corresponding to nucleotides 955-957 of SEQ ID NO: 1 are absent;
(d) nucleotides corresponding to nucleotides 993-995 of SEQ ID NO: 1 are absent; and
(e) nucleotides corresponding to nucleotides 1014-1016 of SEQ ID NO: 1 are absent; and/or
(iv) the vector plasmid comprises a backbone less than 4000 nucleotides, less than 3500 nucleotides, less than 3000 nucleotides, or less than 2500 nucleotides in length.

11. The two plasmid system of claim 10(iii)(b), wherein nucleotides corresponding to nucleotides 766-768 of SEQ ID NO: 1 are ATT.

12. A method for producing a recombinant AAV preparation comprising:
(a) obtaining the two-plasmid system, the helper plasmid or the vector plasmid as defined in any one of claims 1-11;
(b) transfecting a host cell with the two-plasmid system, the helper plasmid or the vector plasmid as defined in any one of claims 1-11; and
(c) culturing the host cell under conditions suitable for recombinant AAV production.

13. The method of claim 12:
(i) further comprising a step of harvesting the recombinant AAV to provide a recombinant AAV preparation; and/or
(ii) wherein the recombinant AAV preparation comprises a low level of replication competent AAV (rcAAV); and/or
(iii) wherein the level of rcAAV is measured to be less than 1 rcAAV in 10⁷ recombinant AAV, less than 1 rcAAV in 10⁹ recombinant AAV, or less than 1 rcAAV in 10¹⁰ recombinant AAV; and/or
(iv) wherein the recombinant AAV preparation comprises a low level of rcAAV and the level of rcAAV is less than the level of rcAAV produced using an equivalent method except that the vector plasmid comprises both at least one AAV rep gene and at least one AAV cap gene; and/or
(v) comprising a step of selecting a ratio of helper plasmid to vector plasmid wherein the ratio of helper plasmid to vector plasmid is selected or adjusted to a ratio that allows the user to obtain the desired ratio of full to total particles or the high or desired yield of recombinant AAV vector; and/or
(vi) wherein the ratio of helper plasmid to vector plasmid is selected or adjusted to a ratio that achieves a maximum yield of recombinant AAV with the minimum yield of empty particles achievable at such maximum yield of recombinant AAV; and/or
(vii) wherein the desired ratio of full to total particles is a ratio of full to total particles that is at least 20% or at least 30% of the ratio of full to total particles achieved using an equivalent method with a ratio of helper plasmid to vector plasmid of 1.8:1; and/or
(viii) further comprising a step of purifying the recombinant AAV.

## Patentansprüche

1. Helferplasmid, das mindestens ein Adeno-assoziiertes Virus- (AAV) Rep-Gen, das für mindestens ein funktionelles AAV-Rep-Protein kodiert, und mindestens ein Helfervirusgen umfasst und das kein Cap-Gen enthält, das für einen funktionellen Satz von Cap-Proteinen kodiert.

2. Zwei-Plasmid-System, das das Helferplasmid nach Anspruch 1 und ein Vektorplasmid umfasst.

3. Zwei-Plasmid-System nach Anspruch 2, wobei:
(i) das Vektorplasmid Folgendes umfasst:
(a) ein AAV-Cap-Gen, das für mindestens ein funktionelles AAV-Cap-Protein kodiert; oder
(b) mindestens einen AAV-Cap-Gen-Promotor, eine Klonierungsstelle, die operativ mit dem AAV-Cap-Gen-Promotor verbunden ist, und eine Expressionskassette, die auf mindestens einer Seite von einer invertierten terminalen Wiederholung (Inverted Terminal Repeat, ITR) flankiert ist;
wobei das Vektorplasmid kein Rep-Gen umfasst, das für ein funktionelles Rep-Protein kodiert, und die Expressionskassette ein Transgen umfasst, das funktionell mit mindestens einem regulatorischen Kontrollelement verbunden ist; und/oder
(ii) das Verhältnis von Helferplasmid zu Vektorplasmid zwischen 3:1 bis 1:10, zwischen 1,5:1 und 1:9, zwischen 1,4:1 und 1:8, zwischen 1,3:1 und 1:7; zwischen 1,2:1 und 1:6; zwischen 1,1:1 und 1:5; 1:1 und 1:4; oder zwischen 1:1,5 und 1:3 liegt.

4. Zwei-Plasmid-System oder Helferplasmid nach einem der vorhergehenden Ansprüche, wobei das mindestens eine AAV-Rep-Gen Folgendes umfasst: ein Gen, das für ein funktionelles Rep-52-Protein kodiert, mindestens ein Gen, das für ein funktionelles Rep-40-Protein kodiert, und ein Gen, das für ein funktionelles Rep-68-Protein kodiert.

5. Zwei-Plasmid-System oder Helferplasmid nach einem der vorhergehenden Ansprüche, wobei:
(i) das mindestens eine AAV-Rep-Gen ein Gen umfasst, das für ein funktionelles Rep 52-Protein kodiert, und das Gen, das für ein funktionelles Rep-52-Protein kodiert, eine Nukleinsäuresequenz umfasst, die mindestens 95 %, mindestens 98 %, mindestens 99 % oder 100 % Identität mit der vollen Länge oder einem Fragment mit einer Länge von mindestens 800, mindestens 900, 1000 oder mindestens 1100 Nukleotiden der Nukleotide 993 bis 2186 von SEQ ID NO: 1 oder mit einem entsprechenden Nukleotidabschnitt in einem anderen AAV-Serotyp aufweist; und/oder
(ii) (a) das mindestens eine AAV-Rep-Gen ein Gen umfasst, das für ein funktionelles Rep-40-Protein kodiert, und das Gen, das für ein funktionelles Rep-40-Protein kodiert, eine Nukleinsäuresequenz umfasst, die mindestens 95 %, mindestens 98 %, mindestens 99 % oder mindestens 100 % Identität mit der vollen Länge oder mit einem Fragment mit einer Länge von mindestens 600, mindestens 700, mindestens 800 oder mindestens 900 Nukleotiden eines Nukleotidabschnitts, der den Nukleotiden 993 bis 2252 minus den Nukleotiden 1907 bis 2227 von SEQ ID NO: 1 entspricht oder mit entsprechenden Nukleotidabschnitten in einem anderen AAV-Serotyp aufweist; und/oder
(b) das mindestens eine AAV-Rep-Gen ein Gen umfasst, das für ein funktionelles Rep-40-Protein kodiert, und das Gen, das für ein funktionelles Rep-40-Protein kodiert, eine Nukleinsäuresequenz umfasst, die mindestens 95 %, mindestens 98 %, mindestens 99 % oder mindestens 100 % Identität mit der vollen Länge oder mit einem Fragment mit einer Länge von mindestens 900, mindestens 1000, mindestens1100 oder mindestens 1200 Nukleotiden der Nukleotide 993 bis 2252 von SEQ ID NO: 1 oder mit einem entsprechenden Nukleotidabschnitt in einem anderen AAV-Serotyp aufweist; und/oder
(iii) das mindestens eine AAV-Rep-Gen ein Gen umfasst, das für ein funktionelles Rep-68-Protein kodiert, und das Gen, das für ein funktionelles Rep-68-Protein kodiert, eine Nukleinsäuresequenz umfasst, die mindestens 95 %, mindestens 98 %, mindestens 99 % oder mindestens 100 % Identität mit der vollen Länge oder mit einem Fragment mit einer Länge von mindestens 1000, mindestens 1400, mindestens 1500 oder mindestens 1600 Nukleotiden eines Nukleotidabschnitts, der den Nukleotiden 321 bis 2252 minus den Nukleotiden 1907 bis 2227 von SEQ ID NO: 1 entspricht oder mit entsprechenden Nukleotidabschnitten in einem anderen AAV-Serotyp aufweist.

6. Zwei-Plasmid-System oder Helferplasmid nach einem der vorhergehenden Ansprüche, wobei:
(i) das Helferplasmid kein Gen umfasst, das für ein funktionelles Rep 78-Protein kodiert; und/oder
(ii) das Helferplasmid keine zusammenhängende Sequenz von mindestens 1700, mindestens 1800 oder allen 1866 Nukleotiden umfasst, die einem zusammenhängenden Nukleotidabschnitt äquivalenter Länge entspricht, der innerhalb der Nukleotide 321 bis 2186 von SEQ ID NO: 1 oder innerhalb eines entsprechenden Nukleotidabschnitts in einem anderen AAV-Serotyp enthalten ist; und/oder
(iii) das mindestens eine AAV-Rep-Gen keinen funktionellen internen p40-Promotor umfasst; und/oder
(iv) das Helferplasmid keinen zusammenhängenden Abschnitt von ausschließlich Cap-Gen-Sequenz von mehr als 60 Nukleotiden umfasst.

7. Zwei-Plasmid-System oder Helferplasmid nach einem der vorhergehenden Ansprüche, wobei:
(i) das Helferplasmid mindestens ein Helfervirusgen umfasst und das mindestens eine Helfervirusgen eine virale assoziierte (VA) Nukleinsäure, ein E2A-Gen und ein E4-Gen umfasst; und/oder
(ii) das Helferplasmid mindestens ein Helfervirusgen umfasst und das mindestens eine Helfervirusgen eine VA-Nukleinsäure, ein E2A-Gen und ein E4-Gen umfasst und das E4-Gen nicht zwischen der VA-Nukleinsäure und dem E2A-Gen liegt; und/oder
(iii) das Helferplasmid mindestens ein Helfervirusgen umfasst und das Helferplasmid weniger als 25000 bp, weniger als 20000 bp, weniger als 15000 bp, weniger als 145000 bp, weniger als 10000 bp, zwischen 10000 bp und 20000 bp, zwischen 12000 bp und 15000 bp oder 14021 bp lang ist; und/oder
(iv) das Helferplasmid keine zusammenhängende Sequenz von mindestens 2000, mindestens 2500, mindestens 3000 oder allen 3427 Nukleotiden eines zusammenhängenden Nukleotidabschnitts äquivalenter Länge, der innerhalb der Nukleotide 194 bis 3620 von SEQ ID NO: 2 enthalten ist, oder keinen entsprechenden Nukleotidabschnitt in einem anderen Serotyp des Adenovirus umfasst; und/oder
(v) das Helferplasmid keine zusammenhängende Sequenz von mindestens 50, mindestens 60 oder allen 69 Nukleotiden eines zusammenhängenden Nukleotidabschnitts äquivalenter Länge, der innerhalb der Nukleotide 4032 bis 4100 von SEQ ID NO: 2 enthalten ist, oder keinen entsprechenden Nukleotidabschnitt in einem anderen Serotyp des Adenovirus umfasst; und/oder
(vi) das Helferplasmid keine zusammenhängende Sequenz von mindestens 200, mindestens 300, mindestens 350 oder allen 363 Nukleotiden eines zusammenhängenden Nukleotidabschnitts äquivalenter Länge, der innerhalb der Nukleotide 4051 bis 4413 von SEQ ID NO: 1 enthalten ist, oder keinen entsprechenden Nukleotidabschnitt in einem anderen AAV-Serotyp aufweist.

8. Zwei-Plasmid-System oder Helferplasmid nach einem der vorhergehenden Ansprüche, wobei:
(i) das Helferplasmid keine zusammenhängende Sequenz von mindestens 400, mindestens 500, mindestens 600 oder allen 647 Nukleotiden eines zusammenhängenden Nukleotidabschnitts äquivalenter Länge, der innerhalb der Nukleotide 2301 bis 2947 von SEQ ID NO: 1 enthalten ist, oder keinen entsprechenden Nukleotidabschnitt in einem anderen Serotyp des AAV-Serotyps umfasst; und/oder
(ii) das Helferplasmid mindestens ein Helfervirusgen umfasst und das mindestens eine Helfervirusgen eine VA-Nukleinsäure, ein E2A-Gen und ein E4-Gen umfasst, und die VA-Nukleinsäure, das E2A-Gen und das E4-Gen in einem zusammenhängenden Abschnitt von weniger als 15000, weniger als 12000, weniger als 10000 oder weniger als 9000 Nukleotiden enthalten sind; und/oder
(iii) das Helferplasmid keine zusammenhängende Sequenz von mindestens 15000, mindestens 20000, mindestens 22000 oder allen 22137 Nukleotiden eines zusammenhängenden Nukleotidabschnitts äquivalenter Länge, der innerhalb der Nukleotide 10619 bis 32756 von SEQ ID NO: 2 enthalten ist, oder keinen entsprechenden Nukleotidabschnitt in einem anderen Serotyp des Adenovirus umfasst; und/oder
(iv) das Helferplasmid und/oder das Vektorplasmid keine künstliche Rep-Bindungsstelle umfasst.

9. Zwei-Plasmid-System oder Helferplasmid nach einem der vorhergehenden Ansprüche, wobei das Helferplasmid mindestens ein Helfervirusgen umfasst und das mindestens eine Hilfsvirusgen in einem zusammenhängenden Abschnitt des Plasmids enthalten ist, der mindestens 95 %, mindestens 98%, mindestens 99 % oder 100 % Identität mit der vollen Länge oder mit einem Fragment mit einer Länge von mindestens 6000 Nukleotiden, mindestens 7000 Nukleotiden oder 8000 Nukleotiden von SEQ ID NO: 4 aufweist.

10. Zwei-Plasmid-System nach einem der Ansprüche 2 bis 9, wobei:
(i) das Vektorplasmid mindestens einen AAV-Cap-Gen-Promotor enthält, der einen AAV p40-Promotor, einen p5-Promotor und einen p19-Promotor umfasst; und/oder
(ii) das Vektorplasmid keine entbehrlichen Translations-Initiationscodons enthält; und/oder
(iii) das Vektorplasmid eine Promotorregion umfasst, die einen oder mehrere Promotoren umfasst, und in der Promotorregion:
(a) Nukleotide, die den Nukleotiden 321 bis 323 von SEQ ID NO: 1 entsprechen, fehlen;
(b) Nukleotide, die den Nukleotiden 766 bis 768 der SEQ ID NO: 1 entsprechen, nicht ATG sind;
(c) Nukleotide, die den Nukleotiden 955 bis 957 von SEQ ID NO: 1 entsprechen, fehlen;
(d) Nukleotide, die den Nukleotiden 993 bis 995 von SEQ ID NO: 1 entsprechen, fehlen; und
(e) Nukleotide, die den Nukleotiden 1014 bis 1016 von SEQ ID NO: 1 entsprechen, fehlen; und/oder
(iv) das Vektorplasmid ein Rückgrat mit einer Länge von weniger als 4000 Nukleotiden, weniger als 3000 Nukleotiden oder weniger als 2500 Nukleotiden umfasst.

11. Zwei-Plasmid-System nach Anspruch 10(iii)(b), wobei Nukleotide, die den Nukleotiden 766 bis 768 von SEQ ID NO: 1 entsprechen, ATT sind.

12. Verfahren zur Herstellung eines rekombinanten AAV-Präparats, das Folgendes umfasst:
(a) Erhalten des Zwei-Plasmid-Systems, des Helferplasmids oder des Vektorplasmids, wie in einem der Ansprüche 1-11 definiert;
(b) Transfizieren einer Wirtszelle mit dem Zwei-Plasmid-System, dem Helferplasmid oder dem Vektorplasmid, wie in einem der Ansprüche 1-11 definiert; und
(c) Züchten der Wirtszelle unter Bedingungen, die für die rekombinante AAV-Produktion geeignet sind.

13. Verfahren nach Anspruch 12:
(i) das ferner einen Schritt des Erntens des rekombinanten AAV umfasst, um ein rekombinantes AAV-Präparat bereitzustellen; und/oder
(ii) wobei das rekombinante AAV-Präparat einen geringen Gehalt an replikationskompetentem AAV (rcAAV) umfasst; und/oder
(iii) wobei der Gehalt an rcAAV mit weniger als 1 rcAAV in 10⁷ rekombinanten AAV, weniger als 1 rcAAV in 10⁹ rekombinanten AAV oder weniger als 1 rcAAV in 10¹⁰ rekombinanten AAV gemessen wird; und/oder
(iv) wobei das rekombinante AAV-Präparat einen geringen Gehalt an rcAAV umfasst und der Gehalt an rcAAV geringer ist als der Gehalt an rcAAV, der unter Verwendung eines äquivalenten Verfahrens hergestellt wird, außer dass das Vektorplasmid sowohl mindestens ein AAV-Rep-Gen als auch mindestens ein AAV-Cap-Gen umfasst; und/oder
(v) das einen Schritt der Auswahl eines Verhältnisses von Helferplasmid zu Vektorplasmid umfasst, wobei das Verhältnis von Helferplasmid zu Vektorplasmid so gewählt oder auf ein Verhältnis eingestellt wird, dass dem Benutzer ermöglicht wird, das gewünschte Verhältnis von vollen zu gesamten Partikeln oder die hohe oder gewünschte Ausbeute an rekombinantem AAV-Vektor zu erhalten; und/oder
(vi) wobei das Verhältnis von Helferplasmid zu Vektorplasmid so gewählt oder auf ein Verhältnis eingestellt wird, dass eine maximale Ausbeute an rekombinantem AAV mit der minimalen Ausbeute an leeren Partikeln erzielt wird, die bei einer solchen maximalen Ausbeute an rekombinantem AAV erreichbar ist; und/oder
(vii) wobei das gewünschte Verhältnis von vollen zu gesamten Partikeln ein Verhältnis von vollen zu gesamten Partikeln ist, das mindestens 20 % oder mindestens 30 % des Verhältnisses von vollen zu gesamten Partikeln beträgt, das unter Verwendung eines äquivalenten Verfahrens mit einem Verhältnis von Helferplasmid zu Vektorplasmid von 1,8:1 erreicht wird; und/oder
(viii) das ferner einen Schritt der Reinigung des rekombinanten AAV umfasst.

## Revendications

1. Plasmide auxiliaire comprenant au moins un gène rep de virus adéno-associé (AAV) codant pour au moins une protéine Rep fonctionnelle d'AAV et au moins un gène de virus auxiliaire, et qui ne comprend pas de gène cap codant pour un ensemble fonctionnel de protéines Cap.

2. Système à deux plasmides comprenant le plasmide auxiliaire de la revendication 1 et un plasmide vecteur.

3. Système à deux plasmides selon la revendication 2, dans lequel :
(i) le plasmide vecteur comprend :
(a) un gène cap d'AAV codant pour au moins une protéine Cap fonctionnelle d'AAV ; ou
(b) au moins un promoteur de gène cap d'AAV, un site de clonage lié de manière opérationnelle au promoteur de gène cap d'AAV, et une cassette d'expression flanquée sur au moins un côté par une répétition terminale inversée (ITR) ;
dans lequel le plasmide vecteur ne comprend pas de gène rep codant pour une protéine Rep fonctionnelle et la cassette d'expression comprend un transgène lié de manière opérationnelle à au moins un élément de contrôle régulateur ; et/ou
(ii) le rapport du plasmide auxiliaire par rapport au plasmide vecteur est compris entre 3:1 et 1:10, entre 1,5:1 et 1:9, entre 1,4:1 et 1:8, entre 1,3:1 et 1:7, entre 1,2:1 et 1:6, entre 1,1:1 et 1:5 entre 1:1 et 1:4 ou entre 1:1,5 et 1:3.

4. Système à deux plasmides ou plasmide auxiliaire selon l'une quelconque des revendications précédentes, dans lequel l'au moins un gène rep d'AAV comprend un gène codant pour une protéine Rep 52 fonctionnelle, au moins un gène codant pour une protéine Rep 40 fonctionnelle, et un gène codant une protéine Rep 68 fonctionnelle.

5. Système à deux plasmides ou plasmide auxiliaire selon l'une quelconque des revendications précédentes, dans lequel :
(i) l'au moins un gène rep d'AAV comprend un gène codant pour une protéine Rep 52 fonctionnelle et le gène codant pour une protéine Rep 52 fonctionnelle comprend une séquence d'acide nucléique ayant au moins 95%, au moins 98 %, au moins 99 % ou 100 % d'identité avec la longueur totale ou un fragment d'au moins 800, d'au moins 900, d'au moins 1000 ou d'au moins 1100 nucléotides de longueur des nucléotides 993-2186 de SEQ ID NO : 1, ou avec une étendue correspondante de nucléotides dans un sérotype différent d'AAV ; et/ou
(ii) (a) l'au moins un gène rep d'AAV comprend un gène codant pour une protéine Rep 40 fonctionnelle et le gène codant pour une protéine Rep 40 fonctionnelle comprend une séquence d'acide nucléique ayant au moins 95%, au moins 98 %, au moins 99 % ou 100 % d'identité avec la longueur totale ou un fragment d'au moins 600, d'au moins 700, d'au moins 800 ou d'au moins 900 nucléotides de longueur d'une étendue de nucléotides correspondant aux nucléotides 993-2252 moins les nucléotides 1907-2227 de SEQ ID NO : 1, ou avec des étendues correspondantes de nucléotides dans un sérotype différent d'AAV ; et/ou
(b) l'au moins un gène rep d'AAV comprend un gène codant pour une protéine Rep 40 fonctionnelle et le gène codant pour une protéine Rep 40 fonctionnelle comprend une séquence d'acide nucléique ayant au moins 95%, au moins 98 %, au moins 99 % ou 100 % d'identité avec la longueur totale ou avec un fragment d'au moins 900, d'au moins 1000, d'au moins 1100 ou d'au moins 1200 nucléotides de longueur des nucléotides 993-2252 de SEQ ID NO : 1, ou avec une étendue correspondante de nucléotides dans un sérotype différent d'AAV ; et/ou
(iii) l'au moins un gène rep d'AAV comprend un gène codant pour une protéine Rep 68 fonctionnelle et le gène codant pour une protéine Rep 68 fonctionnelle comprend une séquence d'acide nucléique ayant au moins 95%, au moins 98 %, au moins 99 % ou 100 % d'identité avec la longueur totale ou avec un fragment d'au moins 1000, d'au moins 1400, d'au moins 1500 ou d'au moins 1600 nucléotides de longueur d'une étendue de nucléotides correspondant aux nucléotides 321-2252 moins les nucléotides 1907-2227 de SEQ ID NO : 1, ou avec des étendues correspondantes de nucléotides dans un sérotype différent d'AAV.

6. Système à deux plasmides ou plasmide auxiliaire selon l'une quelconque des revendications précédentes, dans lequel :
(i) le plasmide auxiliaire ne comprend pas de gène codant pour une protéine Rep 78 fonctionnelle ; et/ou
(ii) le plasmide auxiliaire ne comprend pas une séquence contiguë d'au moins 1700, d'au moins 1800 ou tous les 1866 nucléotides correspondant à une étendue contiguë de nucléotides de longueur équivalente comprise dans les nucléotides 321-2186 de SEQ ID NO : 1 ou dans une étendue correspondante de nucléotides dans un sérotype différent d'AAV ; et/ou
(iii) l'au moins un gène rep d'AAV ne comprend pas de promoteur p40 interne fonctionnel ; et/ou
(iv) le plasmide auxiliaire ne comprend pas une étendue contiguë de séquence de gène exclusivement cap de plus de 60 nucléotides.

7. Système à deux plasmides ou plasmide auxiliaire selon l'une quelconque des revendications précédentes, dans lequel :
(i) le plasmide auxiliaire comprend au moins un gène de virus auxiliaire et l'au moins un gène de virus auxiliaire comprend un acide nucléique associé au virus (VA), un gène E2A et un gène E4 ; et/ou
(ii) le plasmide auxiliaire comprend au moins un gène de virus auxiliaire et l'au moins un gène de virus auxiliaire comprend un acide nucléique VA, un gène E2A et un gène E4 et le gène E4 n'est pas situé entre l'acide nucléique VA et le gène E2A ; et/ou
(iii) le plasmide auxiliaire comprend au moins un gène de virus auxiliaire et le plasmide auxiliaire a une longueur d'au moins 25000 pb, d'au moins 20000 pb, d'au moins 15000 pb, d'au moins 14500 pb, comprise entre 10000 pb et 25000 pb, comprise entre 10000 pb et 20000 pb, comprise entre 12000 pb et 15000 pb ou 14021 pb; et/ou
(iv) le plasmide auxiliaire ne comprend pas une séquence contiguë d'au moins 2000, d'au moins 2500, d'au moins 3000 ou tous les 3427 nucléotides d'une étendue contiguë de nucléotides de longueur équivalente comprise dans les nucléotides 194-3620 de SEQ ID NO : 2, ou une étendue correspondante de nucléotides dans un sérotype différent d'adénovirus ; et/ou
(v) le plasmide auxiliaire ne comprend pas une séquence contiguë d'au moins 50, d'au moins 60 ou tous les 69 nucléotides d'une étendue contiguë de nucléotides de longueur équivalente comprise dans les nucléotides 4032-4100 de SEQ ID NO : 2, ou une étendue correspondante de nucléotides dans un sérotype différent d'adénovirus ; et/ou
(vi) le plasmide auxiliaire ne comprend pas une séquence contiguë d'au moins 200, d'au moins 300, d'au moins 350 ou tous les 363 nucléotides d'une étendue contiguë de nucléotides de longueur équivalente comprise dans les nucléotides 4051-4413 de SEQ ID NO : 1, ou une étendue correspondante de nucléotides dans un sérotype différent d'AAV.

8. Système à deux plasmides ou plasmide auxiliaire selon l'une quelconque des revendications précédentes, dans lequel :
(i) le plasmide auxiliaire ne comprend pas une séquence contiguë d'au moins 400, d'au moins 500, d'au moins 600 ou tous les 647 nucléotides d'une étendue contiguë de nucléotides de longueur équivalente comprise dans les nucléotides 2301-2947 de SEQ ID NO : 1, ou une séquence correspondante de nucléotides dans un sérotype différent d'AAV ; et/ou
(ii) le plasmide auxiliaire comprend au moins un gène de virus auxiliaire et l'au moins un gène de virus auxiliaire comprend un acide nucléique VA, un gène E2A et un gène E4, et l'acide nucléique VA, le gène E2A et le gène E4 gène sont compris dans une partie contiguë de moins de 15000, de moins de 12000, de moins de 10000 ou de moins de 9000 nucléotides ; et/ou
(iii) le plasmide auxiliaire ne comprend pas une séquence contiguë d'au moins 15000, d'au moins 20000, d'au moins 22000 ou tous les 22137 nucléotides d'une étendue contiguë de nucléotides de longueur équivalente comprise dans les nucléotides 10619-32756 de SEQ ID NO : 2, ou une étendue correspondante de nucléotides dans un sérotype différent d'adénovirus ; et/ou
(iv) le plasmide auxiliaire et/ou le plasmide vecteur ne comprend pas de site artificiel de liaison Rep.

9. Système à deux plasmides ou plasmide auxiliaire selon l'une quelconque des revendications précédentes, dans lequel le plasmide auxiliaire comprend au moins un gène de virus auxiliaire et l'au moins un gène de virus auxiliaire est compris dans une étendue contiguë du plasmide ayant au moins 95%, au moins 98%, au moins 99% ou 100% d'identité avec la longueur totale ou un fragment d'au moins 6000 nucléotides, d'au moins 7000 nucléotides ou d'au moins 8000 nucléotides de longueur de SEQ ID NO : 4.

10. Système à deux plasmides selon l'une quelconque des revendications 2 à 9, dans lequel :
(i) le plasmide vecteur comprend au moins un promoteur de gène cap d'AAV, qui comprend un promoteur p40 d'AAV, un promoteur p5 et un promoteur p19 ; et/ou
(ii) le plasmide vecteur ne comprend aucun codon d'initiation de traduction dispensable ; et/ou
(iii) le plasmide vecteur comprend une région promotrice comprenant un ou plusieurs promoteurs, et dans la région promotrice :
(a) les nucléotides correspondant aux nucléotides 321-323 de SEQ ID NO : 1 sont absents ;
(b) les nucléotides correspondant aux nucléotides 766-768 de SEQ ID NO : 1 ne sont pas ATG ;
(c) les nucléotides correspondant aux nucléotides 955-957 de SEQ ID NO : 1 sont absents ;
(d) les nucléotides correspondant aux nucléotides 993-995 de SEQ ID NO : 1 sont absents ; et
(e) les nucléotides correspondant aux nucléotides 1014-1016 de SEQ ID NO : 1 sont absents ; et/ou
(iv) le plasmide vecteur comprend un squelette d'une longueur inférieure à 4000 nucléotides, inférieure à 3500 nucléotides, inférieure à 3000 nucléotides ou inférieure à 2500 nucléotides.

11. Système à deux plasmides selon la revendication 10(iii)(b), dans lequel les nucléotides correspondant aux nucléotides 766-768 de SEQ ID NO : 1 sont ATT.

12. Procédé de production d'une préparation d'AAV recombinant comprenant :
(a) l'obtention du système à deux plasmides, du plasmide auxiliaire ou du plasmide vecteur tels que définis dans l'une quelconque des revendications 1 à 11 ;
(b) la transfection d'une cellule hôte avec le système à deux plasmides, le plasmide auxiliaire ou le plasmide vecteur tels que définis dans l'une quelconque des revendications 1 à 11 ; et
(c) la culture de la cellule hôte dans des conditions appropriées pour la production d'AAV recombinant.

13. Procédé selon la revendication 12 :
(i) comprenant en outre une étape de récolte de l'AAV recombinant pour fournir une préparation d'AAV recombinant ; et/ou
(ii) dans lequel la préparation d'AAV recombinant comprend un faible niveau d'AAV compétent pour la réplication (rcAAV) ; et/ou
(iii) dans lequel le niveau de rcAAV est mesuré comme étant inférieur à 1 rcAAV dans 10⁷ AAV recombinants, inférieur à 1 rcAAV dans 10⁹ AAV recombinants, ou inférieur à 1 rcAAV dans 10¹⁰ AAV recombinant ; et/ou
(iv) dans lequel la préparation d'AAV recombinant comprend un faible niveau de rcAAV et le niveau de rcAAV est inférieur au niveau de rcAAV produit en utilisant un procédé équivalent sauf que le plasmide vecteur comprend à la fois au moins un gène rep d'AAV et au moins un gène cap d'AAV ; et/ou
(v) comprenant une étape de sélection d'un rapport de plasmide auxiliaire par rapport au plasmide vecteur dans lequel le rapport de plasmide auxiliaire par rapport au plasmide vecteur est sélectionné ou ajusté à un rapport qui permet à l'utilisateur d'obtenir le rapport souhaité de particules entières par rapport aux particules totales ou le rendement élevé ou souhaité du vecteur AAV recombinant ; et/ou
(vi) dans lequel le rapport du plasmide auxiliaire par rapport au plasmide vecteur est sélectionné ou ajusté à un rapport qui atteint un rendement maximum d'AAV recombinant avec le rendement minimum de particules vides pouvant être obtenu à ce rendement maximum d'AAV recombinant ; et/ou
(vii) dans lequel le rapport souhaité de particules pleines par rapport aux particules totales est un rapport de particules pleines par rapports aux particules totales qui est d'au moins 20% ou d'au moins 30 % du rapport de particules pleines par rapport aux particules totales obtenu en utilisant un procédé équivalent avec un rapport du plasmide auxiliaire par rapport au plasmide vecteur de 1,8 : 1 ; et/ou
(viii) comprenant en outre une étape de purification de l'AAV recombinant.
